# EUROPEAN PATENT APPLICATION

(11) **EP 2 333 074 A1**
(43) Date of publication of application: **15.06.2011**
(21) Application number: 09179047.7
(22) Date of filing: 14.12.2009
(51) Int. Cl.: C12N 15/62, C07K 14/50, C12N 9/48, A61K 38/00

(54) **Substances and methods for the treatment of lysosmal storage diseases**

(71) Applicant: Steinfeld, Robert, 37077 Göttingen (DE)
(72) Inventor: Steinfeld, Robert, 37077 Göttingen (DE)
(74) Representative: Vossius & Partner

(57) **Abstract**

The present invention relates to a chimeric molecule comprising (i) a targeting moiety that binds to heparin or heparan sulfate proteoglycans, (ii) a lysosomal peptide or protein, (iii) wherein the targeting moiety is a neurotrophic growth factor and/or, wherein the targeting moiety comprises one of the following consensus sequences BBXB, BXBB, BBXXB, BXXBB, BBXXXB or BXXXBB and wherein B represents an arginine, lysine or histidine amino acid and X represents any amino acid, (iii) with the proviso that the targeting moiety is at least thirteen amino acids long.

SEQUENCE LISTING

<110>  Steinfeld, Robert

<120>  SUBSTANCES AND METHODS FOR THE TREATMENT OF LYSOSOMAL STORAGE
       DISEASES

<130>  R2546 EP BLN

<160>  72

<170>  PatentIn version 3.3

<210>  1
<211>  16
<212>  PRT
<213>  Homo sapiens

<400>  1

Lys Ile Arg Ser Glu Phe Lys Lys Lys Tyr Gly Lys Ser Leu Tyr Tyr
1               5                   10                  15


<210>  2
<211>  16
<212>  PRT
<213>  Homo sapiens

<400>  2

Gln Arg Phe Arg His Arg Asn Arg Lys Gly Tyr Arg Ser Gln Arg Gly
1               5                   10                  15


<210>  3
<211>  16
<212>  PRT
<213>  Homo sapiens

<400>  3

Lys Phe Ile Ile Pro Ser Pro Lys Arg Pro Val Lys Leu Leu Ser Gly
1               5                   10                  15


<210>  4
<211>  16
<212>  PRT
<213>  Homo sapiens

<400>  4

Gly His Phe Lys Asp Pro Lys Arg Leu Tyr Cys Lys Asn Gly Gly Phe
1               5                   10                  15


<210>  5
<211>  16
<212>  PRT
<213>  Homo sapiens

<400>  5

Asp Gly Gly Ser Pro Ile Arg His Tyr Leu Ile Lys Tyr Lys Ala Lys
1               5                   10                  15

<210>  6
<211>  16
<212>  PRT
<213>  Homo sapiens

<400>  6

Gly Leu Ser Glu Lys Thr Leu Arg Lys Trp Leu Lys Met Phe Lys Lys
1               5                   10                  15


<210>  7
<211>  16
<212>  PRT
<213>  Homo sapiens

<400>  7

Lys Leu Asn Cys Arg Leu Tyr Arg Lys Ala Asn Lys Ser Ser Lys Leu
1               5                   10                  15


<210>  8
<211>  16
<212>  PRT
<213>  Homo sapiens

<400>  8

Ser His Leu Arg Lys Leu Arg Lys Arg Leu Leu Arg Asp Ala Asp Asp
1               5                   10                  15


<210>  9
<211>  16
<212>  PRT
<213>  Homo sapiens

<400>  9

Gly His Arg Pro Leu Asp Lys Lys Arg Glu Glu Ala Pro Ser Leu Arg
1               5                   10                  15


<210>  10
<211>  15
<212>  PRT
<213>  Homo sapiens

<400>  10

Ser Arg Gly Lys Gly Arg Arg Gly Gln Arg Gly Lys Asn Arg Gly
1               5                   10                  15


<210>  11
<211>  16
<212>  PRT
<213>  Homo sapiens

<400>  11

Pro Asp Ala Pro Arg Ile Lys Lys Ile Val Gln Lys Lys Leu Ala Gly
1               5                   10                  15


<210>  12
<211>  16

<212>  PRT
<213>  Homo sapiens

<400>  12

Asp Lys Lys Gly Phe Tyr Lys Lys Lys Gln Cys Arg Pro Ser Lys Gly
1               5                   10                  15


<210>  13
<211>  16
<212>  PRT
<213>  Homo sapiens

<400>  13

Arg Gln Ile Lys Ile Trp Phe Gln Asn Arg Arg Met Lys Trp Lys Lys
1               5                   10                  15


<210>  14
<211>  13
<212>  PRT
<213>  Homo sapiens

<400>  14

Lys Arg Val Ser Arg Asn Lys Ser Glu Lys Lys Arg Arg
1               5                   10


<210>  15
<211>  12
<212>  DNA
<213>  Homo sapiens

<400>  15
agatcccccg gg                                                          12


<210>  16
<211>  12
<212>  DNA
<213>  Homo sapiens

<400>  16
ggatcccccg gg                                                          12


<210>  17
<211>  51
<212>  DNA
<213>  Homo sapiens

<400>  17
cgccagataa agatttggtt ccagaatcgg cgcatgaagt ggaagaagta a               51


<210>  18
<211>  16
<212>  PRT
<213>  Homo sapiens

<400>  18

Arg Gln Ile Lys Ile Trp Phe Gln Asn Arg Arg Met Lys Trp Lys Lys
1               5                   10                  15

<210> 19
<211> 42
<212> DNA
<213> Homo sapiens

<400> 19
aaaagagtat ctagaaacaa atctgaaaag aaacgtagat aa 42


<210> 20
<211> 13
<212> PRT
<213> Homo sapiens

<400> 20

Lys Arg Val Ser Arg Asn Lys Ser Glu Lys Lys Arg Arg
1               5                   10


<210> 21
<211> 30
<212> DNA
<213> Homo sapiens

<400> 21
agatccgtcg acatcgaagg tagaggcatt 30


<210> 22
<211> 30
<212> DNA
<213> Homo sapiens

<400> 22
ggatccgtcg acatcgaagg tagaggcatt 30


<210> 23
<211> 441
<212> DNA
<213> Homo sapiens

<400> 23
cccgccttgc ccgaggatgg cggcagcggc gccttcccgc ccggccactt caaggacccc 60

aagcggctgt actgcaaaaa cgggggcttc ttcctgcgca tccaccccga cggccgagtt 120

gacggggtcc gggagaagag cgaccctcac atcaagctac aacttcaagc agaagagaga 180

ggagttgtgt ctatcaaagg agtgtctgct aaccgttacc tggctatgaa ggaagatgga 240

agattactgg cttctaaatc tgttacggat gagtgtttct tttttgaacg attggaatct 300

aataactaca atacttaccg gtcaaggaaa tacaccagtt ggtatgtggc actgaaacga 360

actgggcagt ataaacttgg ctccaaaaca ggacctgggc agaaagctat actttttctt 420

ccaatgtctg ctaagagctg a 441


<210> 24
<211> 145
<212> PRT
<213> Homo sapiens

<400> 24

Pro Ala Leu Pro Glu Asp Gly Gly Ser Gly Ala Phe Pro Pro Gly His
1               5                   10                  15

Phe Lys Asp Pro Lys Arg Leu Tyr Cys Lys Asn Gly Gly Phe Phe Leu
            20                  25                  30

Arg Ile His Pro Asp Gly Arg Val Asp Gly Val Arg Glu Lys Ser Asp
        35                  40                  45

Pro His Ile Lys Leu Gln Leu Gln Ala Glu Glu Arg Gly Val Val Ser
    50                  55                  60

Ile Lys Gly Val Ser Ala Asn Arg Tyr Leu Ala Met Lys Glu Asp Gly
65                  70                  75                  80

Arg Leu Leu Ala Ser Lys Ser Val Thr Asp Glu Cys Phe Phe Phe Glu
                85                  90                  95

Arg Leu Glu Ser Asn Asn Tyr Asn Thr Tyr Arg Ser Arg Lys Tyr Thr
            100                 105                 110

Ser Trp Tyr Val Ala Leu Lys Arg Thr Gly Gln Tyr Lys Leu Gly Ser
        115                 120                 125

Lys Thr Gly Pro Gly Gln Lys Ala Ile Leu Phe Leu Pro Met Ser Ala
    130                 135                 140

Lys
145

<210>  25
<211>  441
<212>  DNA
<213>  Homo sapiens

<400>  25
cccgccttgc ccgaggatgg cggcagcggc gccttcccgc ccggccactt caaggacccc      60

aagcggctgt actgcaaaaa cgggggcttc ttcctgcgca tccaccccga cggccgagtt     120

gacggggtcc gggagaagag cgaccctcac atcaagctac aacttcaagc agaagagaga     180

ggagttgtgt ctatcaaagg agtgtctgct aaccgttacc tggctatgaa ggaagatgga     240

agattactgg cttctaaatc tgttacggat gagtgtttct tttttgcacg attggaatct     300

aataactaca atacttaccg gtcaaggaaa tacaccagtt ggtatgtggc actgaaacga     360

actgggcagt ataaacttgg ctccaaaaca ggacctgggc agaaagctat actttttctt     420

ccaatgtctg ctaagagctg a                                               441

<210>  26
<211>  146
<212>  PRT
<213>  Homo sapiens

<400>  26

Pro Ala Leu Pro Glu Asp Gly Gly Ser Gly Ala Phe Pro Pro Gly His
1               5                   10                  15

Phe Lys Asp Pro Lys Arg Leu Tyr Cys Lys Asn Gly Gly Phe Phe Leu
            20                  25                  30

Arg Ile His Pro Asp Gly Arg Val Asp Gly Val Arg Glu Lys Ser Asp
        35                  40                  45

Pro His Ile Lys Leu Gln Leu Gln Ala Glu Glu Arg Gly Val Val Ser
    50                  55                  60

Ile Lys Gly Val Ser Ala Asn Arg Tyr Leu Ala Met Lys Glu Asp Gly
65                  70                  75                  80

Arg Leu Leu Ala Ser Lys Ser Val Thr Asp Glu Cys Phe Phe Phe Ala
                85                  90                  95

Arg Leu Glu Ser Asn Asn Tyr Asn Thr Tyr Arg Ser Arg Lys Tyr Thr
            100                 105                 110

Ser Trp Tyr Val Ala Leu Lys Arg Thr Gly Gln Tyr Lys Leu Gly Ser
        115                 120                 125

Lys Thr Gly Pro Gly Gln Lys Ala Ile Leu Phe Leu Pro Met Ser Ala
    130                 135                 140

Lys Ser
145

<210>  27
<211>  441
<212>  DNA
<213>  Homo sapiens

<400>  27
cccgccttgc ccgaggatgg cggcagcggc gccttcccgc ccggccactt caaggacccc      60

aagcggctgt actgcaaaaa cgggggcttc ttcctgcgca tccaccccga cggccgagtt     120

gacgggacaa gggacaggag cgaccagcac attcagctgc agctcagtgc agaagagaga     180

ggagttgtgt ctatcaaagg agtgtctgct aaccgttacc tggctatgaa ggaagatgga     240

agattactgg cttctaaatc tgttacggat gagtgtttct tttttgaacg attggaatct     300

aataactaca atacttaccg gtcaaggaaa tacaccagtt ggtatgtggc actgaaacga     360

actgggcagt ataaacttgg ctccaaaaca ggacctgggc agaaagctat actttttctt     420

ccaatgtctg ctaagagctg a                                               441

<210>  28
<211>  146

<212>  PRT
<213>  Homo sapiens

<400>  28

Pro Ala Leu Pro Glu Asp Gly Gly Ser Gly Ala Phe Pro Pro Gly His
1               5                   10                  15

Phe Lys Asp Pro Lys Arg Leu Tyr Cys Lys Asn Gly Gly Phe Phe Leu
            20                  25                  30

Arg Ile His Pro Asp Gly Arg Val Asp Gly Thr Arg Asp Arg Ser Asp
        35                  40                  45

Gln His Ile Gln Leu Gln Leu Ser Ala Glu Glu Arg Gly Val Val Ser
    50                  55                  60

Ile Lys Gly Val Ser Ala Asn Arg Tyr Leu Ala Met Lys Glu Asp Gly
65                  70                  75                  80

Arg Leu Leu Ala Ser Lys Ser Val Thr Asp Glu Cys Phe Phe Phe Glu
                85                  90                  95

Arg Leu Glu Ser Asn Asn Tyr Asn Thr Tyr Arg Ser Arg Lys Tyr Thr
            100                 105                 110

Ser Trp Tyr Val Ala Leu Lys Arg Thr Gly Gln Tyr Lys Leu Gly Ser
        115                 120                 125

Lys Thr Gly Pro Gly Gln Lys Ala Ile Leu Phe Leu Pro Met Ser Ala
    130                 135                 140

Lys Ser
145

<210>  29
<211>  441
<212>  DNA
<213>  Homo sapiens

<400>  29
cccgccttgc ccgaggatgg cggcagcggc gccttcccgc ccggccactt caaggacccc      60

aagcggctgt actgcaaaaa cgggggcttc ttcctgcgca tccaccccga cggccgagtt     120

gacgggacaa gggacaggag cgaccagcac attcagctgc agctcagtgc agaagagaga     180

ggagttgtgt ctatcaaagg agtgtctgct aaccgttacc tggctatgaa ggaagatgga     240

agattactgg cttctaaatc tgttacggat gagtgtttct tttttgcacg attggaatct     300

aataactaca atacttaccg gtcaaggaaa tacaccagtt ggtatgtggc actgaaacga     360

actgggcagt ataaacttgg ctccaaaaca ggacctgggc agaaagctat actttttctt     420

ccaatgtctg ctaagagctg a                                               441

<210> 30
<211> 146
<212> PRT
<213> Homo sapiens

<400> 30

Pro Ala Leu Pro Glu Asp Gly Gly Ser Gly Ala Phe Pro Pro Gly His
1               5                   10                  15

Phe Lys Asp Pro Lys Arg Leu Tyr Cys Lys Asn Gly Gly Phe Phe Leu
            20                  25                  30

Arg Ile His Pro Asp Gly Arg Val Asp Gly Thr Arg Asp Arg Ser Asp
        35                  40                  45

Gln His Ile Gln Leu Gln Leu Ser Ala Glu Glu Arg Gly Val Val Ser
    50                  55                  60

Ile Lys Gly Val Ser Ala Asn Arg Tyr Leu Ala Met Lys Glu Asp Gly
65                  70                  75                  80

Arg Leu Leu Ala Ser Lys Ser Val Thr Asp Glu Cys Phe Phe Phe Ala
                85                  90                  95

Arg Leu Glu Ser Asn Asn Tyr Asn Thr Tyr Arg Ser Arg Lys Tyr Thr
            100                 105                 110

Ser Trp Tyr Val Ala Leu Lys Arg Thr Gly Gln Tyr Lys Leu Gly Ser
        115                 120                 125

Lys Thr Gly Pro Gly Gln Lys Ala Ile Leu Phe Leu Pro Met Ser Ala
    130                 135                 140

Lys Ser
145

<210> 31
<211> 1752
<212> DNA
<213> Homo sapiens

<400> 31
atgggactcc aagcctgcct cctagggctc tttgccctca tcctctctgg caaatgcagt 60

tacagcccgg agcccgacca gcggaggacg ctgcccccag gctgggtgtc cctgggccgt 120

gcggaccctg aggaagagct gagtctcacc tttgccctga gacagcagaa tgtggaaaga 180

ctctcggagc tggtgcaggc tgtgtcggat cccagctctc ctcaatacgg aaaatacctg 240

accctagaga atgtggctga tctggtgagg ccatccccac tgaccctcca cacggtgcaa 300

aaatggctct tggcagccgg agcccagaag tgccattctg tgatcacaca ggactttctg 360

acttgctggc tgagcatccg acaagcagag ctgctgctcc ctggggctga gtttcatcac 420

tatgtgggag gacctacgga aacccatgtt gtaaggtccc cacatcccta ccagcttcca 480

caggccttgg ccccccatgt ggactttgtg gggggactgc accgttttcc cccaacatca 540
tccctgaggc aacgtcctga gccgcaggtg acagggactg taggcctgca tctgggggta 600
accccctctg tgatccgtaa gcgatacaac ttgacctcac aagacgtggg ctctggcacc 660
agcaataaca gccaagcctg tgcccagttc ctggagcagt atttccatga ctcagacctg 720
gctcagttca tgcgcctctt cggtggcaac tttgcacatc aggcatcagt agcccgtgtg 780
gttggacaac agggccgggg ccgggccggg attgaggcca gtctagatgt gcagtacctg 840
atgagtgctg gtgccaacat ctccacctgg gtctacagta gccctggccg gcatgaggga 900
caggagccct tcctgcagtg gctcatgctg ctcagtaatg agtcagccct gccacatgtg 960
catactgtga gctatggaga tgatgaggac tccctcagca gcgcctacat ccagcgggtc 1020
aacactgagc tcatgaaggc tgccgctcgg ggtctcaccc tgctcttcgc ctcaggtgac 1080
agtggggccg ggtgttggtc tgtctctgga agacaccagt tccgccctac cttccctgcc 1140
tccagcccct atgtcaccac agtgggaggc acatccttcc aggaaccttt cctcatcaca 1200
aatgaaattg ttgactatat cagtggtggt ggcttcagca atgtgttccc acggccttca 1260
taccaggagg aagctgtaac gaagttcctg agctctagcc cccacctgcc accatccagt 1320
tacttcaatg ccagtggccg tgcctaccca gatgtggctg cactttctga tggctactgg 1380
gtggtcagca acagagtgcc cattccatgg gtgtccggaa cctcggcctc tactccagtg 1440
tttgggggga tcctatcctt gatcaatgag cacaggatcc ttagtggccg cccccctctt 1500
ggctttctca acccaaggct ctaccagcag catggggcag gactctttga tgtaacccgt 1560
ggctgccatg agtcctgtct ggatgaagag gtagagggcc agggtttctg ctctggtcct 1620
ggctgggatc ctgtaacagg ctggggaaca cccaacttcc cagctttgct gaagactcta 1680
ctcaacccca gatcccccgg gcgccagata aagatttggt tccagaatcg gcgcatgaag 1740
tggaagaagt aa 1752

<210> 32
<211> 583
<212> PRT
<213> Homo sapiens

<400> 32

Met Gly Leu Gln Ala Cys Leu Leu Gly Leu Phe Ala Leu Ile Leu Ser
1               5                   10                  15

Gly Lys Cys Ser Tyr Ser Pro Glu Pro Asp Gln Arg Arg Thr Leu Pro
            20                  25                  30

Pro Gly Trp Val Ser Leu Gly Arg Ala Asp Pro Glu Glu Glu Leu Ser
        35                  40                  45

Leu Thr Phe Ala Leu Arg Gln Gln Asn Val Glu Arg Leu Ser Glu Leu
    50                  55                  60

Val Gln Ala Val Ser Asp Pro Ser Ser Pro Gln Tyr Gly Lys Tyr Leu
65                  70                  75                  80

Thr Leu Glu Asn Val Ala Asp Leu Val Arg Pro Ser Pro Leu Thr Leu
                85                  90                  95

His Thr Val Gln Lys Trp Leu Leu Ala Ala Gly Ala Gln Lys Cys His
            100                 105                 110

Ser Val Ile Thr Gln Asp Phe Leu Thr Cys Trp Leu Ser Ile Arg Gln
        115                 120                 125

Ala Glu Leu Leu Leu Pro Gly Ala Glu Phe His His Tyr Val Gly Gly
    130                 135                 140

Pro Thr Glu Thr His Val Val Arg Ser Pro His Pro Tyr Gln Leu Pro
145                 150                 155                 160

Gln Ala Leu Ala Pro His Val Asp Phe Val Gly Gly Leu His Arg Phe
                165                 170                 175

Pro Pro Thr Ser Ser Leu Arg Gln Arg Pro Glu Pro Gln Val Thr Gly
            180                 185                 190

Thr Val Gly Leu His Leu Gly Val Thr Pro Ser Val Ile Arg Lys Arg
        195                 200                 205

Tyr Asn Leu Thr Ser Gln Asp Val Gly Ser Gly Thr Ser Asn Asn Ser
    210                 215                 220

Gln Ala Cys Ala Gln Phe Leu Glu Gln Tyr Phe His Asp Ser Asp Leu
225                 230                 235                 240

Ala Gln Phe Met Arg Leu Phe Gly Gly Asn Phe Ala His Gln Ala Ser
                245                 250                 255

Val Ala Arg Val Val Gly Gln Gln Gly Arg Gly Arg Ala Gly Ile Glu
            260                 265                 270

Ala Ser Leu Asp Val Gln Tyr Leu Met Ser Ala Gly Ala Asn Ile Ser
        275                 280                 285

Thr Trp Val Tyr Ser Ser Pro Gly Arg His Glu Gly Gln Glu Pro Phe
    290                 295                 300

Leu Gln Trp Leu Met Leu Leu Ser Asn Glu Ser Ala Leu Pro His Val
305                 310                 315                 320

His Thr Val Ser Tyr Gly Asp Asp Glu Asp Ser Leu Ser Ser Ala Tyr
                325                 330                 335

Ile Gln Arg Val Asn Thr Glu Leu Met Lys Ala Ala Ala Arg Gly Leu
            340                 345                 350

Thr Leu Leu Phe Ala Ser Gly Asp Ser Gly Ala Gly Cys Trp Ser Val
        355                 360                 365

Ser Gly Arg His Gln Phe Arg Pro Thr Phe Pro Ala Ser Ser Pro Tyr
    370                 375                 380

Val Thr Thr Val Gly Gly Thr Ser Phe Gln Glu Pro Phe Leu Ile Thr
385                 390                 395                 400

Asn Glu Ile Val Asp Tyr Ile Ser Gly Gly Gly Phe Ser Asn Val Phe
                405                 410                 415

Pro Arg Pro Ser Tyr Gln Glu Glu Ala Val Thr Lys Phe Leu Ser Ser
            420                 425                 430

Ser Pro His Leu Pro Pro Ser Ser Tyr Phe Asn Ala Ser Gly Arg Ala
        435                 440                 445

Tyr Pro Asp Val Ala Ala Leu Ser Asp Gly Tyr Trp Val Val Ser Asn
    450                 455                 460

Arg Val Pro Ile Pro Trp Val Ser Gly Thr Ser Ala Ser Thr Pro Val
465                 470                 475                 480

Phe Gly Gly Ile Leu Ser Leu Ile Asn Glu His Arg Ile Leu Ser Gly
                485                 490                 495

Arg Pro Pro Leu Gly Phe Leu Asn Pro Arg Leu Tyr Gln Gln His Gly
            500                 505                 510

Ala Gly Leu Phe Asp Val Thr Arg Gly Cys His Glu Ser Cys Leu Asp
        515                 520                 525

Glu Glu Val Glu Gly Gln Gly Phe Cys Ser Gly Pro Gly Trp Asp Pro
    530                 535                 540

Val Thr Gly Trp Gly Thr Pro Asn Phe Pro Ala Leu Leu Lys Thr Leu
545                 550                 555                 560

Leu Asn Pro Arg Ser Pro Gly Arg Gln Ile Lys Ile Trp Phe Gln Asn
                565                 570                 575

Arg Arg Met Lys Trp Lys Lys
            580

<210>  33
<211>  1743
<212>  DNA

<213>  Homo sapiens

<400>  33
atgggactcc aagcctgcct cctagggctc tttgccctca tcctctctgg caaatgcagt      60

tacagcccgg agcccgacca gcggaggacg ctgcccccag gctgggtgtc cctgggccgt     120

gcggaccctg aggaagagct gagtctcacc tttgccctga gacagcagaa tgtggaaaga     180

ctctcggagc tggtgcaggc tgtgtcggat cccagctctc ctcaatacgg aaaatacctg     240

accctagaga atgtggctga tctggtgagg ccatccccac tgaccctcca cacggtgcaa     300

aaatggctct tggcagccgg agcccagaag tgccattctg tgatcacaca ggactttctg     360

acttgctggc tgagcatccg acaagcagag ctgctgctcc ctggggctga gtttcatcac     420

tatgtgggag gacctacgga aacccatgtt gtaaggtccc cacatcccta ccagcttcca     480

caggccttgg ccccccatgt ggactttgtg gggggactgc accgttttcc cccaacatca     540

tccctgaggc aacgtcctga gccgcaggtg acagggactg taggcctgca tctgggggta     600

accccctctg tgatccgtaa gcgatacaac ttgacctcac aagacgtggg ctctggcacc     660

agcaataaca gccaagcctg tgcccagttc ctggagcagt atttccatga ctcagacctg     720

gctcagttca tgcgcctctt cggtggcaac tttgcacatc aggcatcagt agcccgtgtg     780

gttggacaac agggccgggg ccgggccggg attgaggcca gtctagatgt gcagtacctg     840

atgagtgctg gtgccaacat ctccacctgg gtctacagta gccctggccg gcatgaggga     900

caggagccct tcctgcagtg gctcatgctg ctcagtaatg agtcagccct gccacatgtg     960

catactgtga gctatggaga tgatgaggac tccctcagca gcgcctacat ccagcgggtc    1020

aacactgagc tcatgaaggc tgccgctcgg ggtctcaccc tgctcttcgc ctcaggtgac    1080

agtggggccg ggtgttggtc tgtctctgga agacaccagt tccgccctac cttccctgcc    1140

tccagcccct atgtcaccac agtgggaggc acatccttcc aggaaccttt cctcatcaca    1200

aatgaaattg ttgactatat cagtggtggt ggcttcagca atgtgttccc acggccttca    1260

taccaggagg aagctgtaac gaagttcctg agctctagcc cccacctgcc accatccagt    1320

tacttcaatg ccagtggccg tgcctaccca gatgtggctg cactttctga tggctactgg    1380

gtggtcagca acagagtgcc cattccatgg gtgtccggaa cctcggcctc tactccagtg    1440

tttgggggga tcctatcctt gatcaatgag cacaggatcc ttagtggccg cccccctctt    1500

ggctttctca acccaaggct ctaccagcag catggggcag gactctttga tgtaacccgt    1560

ggctgccatg agtcctgtct ggatgaagag gtagagggcc agggtttctg ctctggtcct    1620

ggctgggatc ctgtaacagg ctggggaaca cccaacttcc cagctttgct gaagactcta    1680

ctcaacccca gatcccccgg gaaaagagta tctagaaaca aatctgaaaa gaaacgtaga    1740

taa                                                                  1743

<210>  34
<211>  580
<212>  PRT
<213>  Homo sapiens

<400> 34

Met Gly Leu Gln Ala Cys Leu Leu Gly Leu Phe Ala Leu Ile Leu Ser
1               5                   10                  15

Gly Lys Cys Ser Tyr Ser Pro Glu Pro Asp Gln Arg Arg Thr Leu Pro
            20                  25                  30

Pro Gly Trp Val Ser Leu Gly Arg Ala Asp Pro Glu Glu Glu Leu Ser
        35                  40                  45

Leu Thr Phe Ala Leu Arg Gln Gln Asn Val Glu Arg Leu Ser Glu Leu
    50                  55                  60

Val Gln Ala Val Ser Asp Pro Ser Ser Pro Gln Tyr Gly Lys Tyr Leu
65                  70                  75                  80

Thr Leu Glu Asn Val Ala Asp Leu Val Arg Pro Ser Pro Leu Thr Leu
                85                  90                  95

His Thr Val Gln Lys Trp Leu Leu Ala Ala Gly Ala Gln Lys Cys His
            100                 105                 110

Ser Val Ile Thr Gln Asp Phe Leu Thr Cys Trp Leu Ser Ile Arg Gln
        115                 120                 125

Ala Glu Leu Leu Leu Pro Gly Ala Glu Phe His His Tyr Val Gly Gly
    130                 135                 140

Pro Thr Glu Thr His Val Val Arg Ser Pro His Pro Tyr Gln Leu Pro
145                 150                 155                 160

Gln Ala Leu Ala Pro His Val Asp Phe Val Gly Gly Leu His Arg Phe
                165                 170                 175

Pro Pro Thr Ser Ser Leu Arg Gln Arg Pro Glu Pro Gln Val Thr Gly
            180                 185                 190

Thr Val Gly Leu His Leu Gly Val Thr Pro Ser Val Ile Arg Lys Arg
        195                 200                 205

Tyr Asn Leu Thr Ser Gln Asp Val Gly Ser Gly Thr Ser Asn Asn Ser
    210                 215                 220

Gln Ala Cys Ala Gln Phe Leu Glu Gln Tyr Phe His Asp Ser Asp Leu
225                 230                 235                 240

Ala Gln Phe Met Arg Leu Phe Gly Gly Asn Phe Ala His Gln Ala Ser
                245                 250                 255

Val Ala Arg Val Val Gly Gln Gln Gly Arg Gly Arg Ala Gly Ile Glu

            260                 265                 270

Ala Ser Leu Asp Val Gln Tyr Leu Met Ser Ala Gly Ala Asn Ile Ser
        275                 280                 285

Thr Trp Val Tyr Ser Ser Pro Gly Arg His Glu Gly Gln Glu Pro Phe
    290                 295                 300

Leu Gln Trp Leu Met Leu Leu Ser Asn Glu Ser Ala Leu Pro His Val
305                 310                 315                 320

His Thr Val Ser Tyr Gly Asp Asp Glu Asp Ser Leu Ser Ser Ala Tyr
                325                 330                 335

Ile Gln Arg Val Asn Thr Glu Leu Met Lys Ala Ala Ala Arg Gly Leu
            340                 345                 350

Thr Leu Leu Phe Ala Ser Gly Asp Ser Gly Ala Gly Cys Trp Ser Val
        355                 360                 365

Ser Gly Arg His Gln Phe Arg Pro Thr Phe Pro Ala Ser Ser Pro Tyr
    370                 375                 380

Val Thr Thr Val Gly Gly Thr Ser Phe Gln Glu Pro Phe Leu Ile Thr
385                 390                 395                 400

Asn Glu Ile Val Asp Tyr Ile Ser Gly Gly Gly Phe Ser Asn Val Phe
                405                 410                 415

Pro Arg Pro Ser Tyr Gln Glu Glu Ala Val Thr Lys Phe Leu Ser Ser
            420                 425                 430

Ser Pro His Leu Pro Pro Ser Ser Tyr Phe Asn Ala Ser Gly Arg Ala
        435                 440                 445

Tyr Pro Asp Val Ala Ala Leu Ser Asp Gly Tyr Trp Val Val Ser Asn
    450                 455                 460

Arg Val Pro Ile Pro Trp Val Ser Gly Thr Ser Ala Ser Thr Pro Val
465                 470                 475                 480

Phe Gly Gly Ile Leu Ser Leu Ile Asn Glu His Arg Ile Leu Ser Gly
                485                 490                 495

Arg Pro Pro Leu Gly Phe Leu Asn Pro Arg Leu Tyr Gln Gln His Gly
            500                 505                 510

Ala Gly Leu Phe Asp Val Thr Arg Gly Cys His Glu Ser Cys Leu Asp
        515                 520                 525

Glu Glu Val Glu Gly Gln Gly Phe Cys Ser Gly Pro Gly Trp Asp Pro

530                 535                 540

Val Thr Gly Trp Gly Thr Pro Asn Phe Pro Ala Leu Leu Lys Thr Leu
545                 550                 555                 560

Leu Asn Pro Arg Ser Pro Gly Lys Arg Val Ser Arg Asn Lys Ser Glu
                565                 570                 575

Lys Lys Arg Arg
            580

<210>  35
<211>  2160
<212>  DNA
<213>  Homo sapiens

<400>  35
atgggactcc aagcctgcct cctagggctc tttgccctca tcctctctgg caaatgcagt      60

tacagcccgg agcccgacca gcggaggacg ctgcccccag gctgggtgtc cctgggccgt     120

gcggaccctg aggaagagct gagtctcacc tttgccctga gacagcagaa tgtggaaaga     180

ctctcggagc tggtgcaggc tgtgtcggat cccagctctc ctcaatacgg aaaatacctg     240

accctagaga atgtggctga tctggtgagg ccatccccac tgaccctcca cacggtgcaa     300

aaatggctct tggcagccgg agcccagaag tgccattctg tgatcacaca ggactttctg     360

acttgctggc tgagcatccg acaagcagag ctgctgctcc ctggggctga gtttcatcac     420

tatgtgggag gacctacgga aacccatgtt gtaaggtccc cacatcccta ccagcttcca     480

caggccttgg ccccccatgt ggactttgtg gggggactgc accgttttcc cccaacatca     540

tccctgaggc aacgtcctga gccgcaggtg acagggactg taggcctgca tctgggggta     600

accccctctg tgatccgtaa gcgatacaac ttgacctcac aagacgtggg ctctggcacc     660

agcaataaca gccaagcctg tgcccagttc ctggagcagt atttccatga ctcagacctg     720

gctcagttca tgcgcctctt cggtggcaac tttgcacatc aggcatcagt agcccgtgtg     780

gttggacaac agggccgggg ccgggccggg attgaggcca gtctagatgt gcagtacctg     840

atgagtgctg gtgccaacat ctccacctgg gtctacagta gccctggccg gcatgaggga     900

caggagccct tcctgcagtg gctcatgctg ctcagtaatg agtcagccct gccacatgtg     960

catactgtga gctatggaga tgatgaggac tccctcagca gcgcctacat ccagcgggtc    1020

aacactgagc tcatgaaggc tgccgctcgg ggtctcaccc tgctcttcgc ctcaggtgac    1080

agtggggccg ggtgttggtc tgtctctgga agacaccagt tccgccctac cttccctgcc    1140

tccagcccct atgtcaccac agtgggaggc acatccttcc aggaaccttt cctcatcaca    1200

aatgaaattg ttgactatat cagtggtggt ggcttcagca atgtgttccc acggccttca    1260

taccaggagg aagctgtaac gaagttcctg agctctagcc cccacctgcc accatccagt    1320

tacttcaatg ccagtggccg tgcctaccca gatgtggctg cactttctga tggctactgg    1380

gtggtcagca acagagtgcc cattccatgg gtgtccggaa cctcggcctc tactccagtg    1440

tttgggggga tcctatcctt gatcaatgag cacaggatcc ttagtggccg cccccctctt 1500

ggctttctca acccaaggct ctaccagcag catggggcag gactctttga tgtaacccgt 1560

ggctgccatg agtcctgtct ggatgaagag gtagagggcc agggtttctg ctctggtcct 1620

ggctgggatc ctgtaacagg ctggggaaca cccaacttcc cagctttgct gaagactcta 1680

ctcaacccca gatccgtcga catcgaaggt agaggcattc ccgccttgcc cgaggatggc 1740

ggcagcggcg ccttcccgcc cggccacttc aaggacccca agcggctgta ctgcaaaaac 1800

gggggcttct tcctgcgcat ccaccccgac ggccgagttg acggggtccg ggagaagagc 1860

gaccctcaca tcaagctaca acttcaagca gaagagagag gagttgtgtc tatcaaagga 1920

gtgtctgcta accgttacct ggctatgaag gaagatggaa gattactggc ttctaaatct 1980

gttacggatg agtgtttctt ttttgaacga ttggaatcta ataactacaa tacttaccgg 2040

tcaaggaaat acaccagttg gtatgtggca ctgaaacgaa ctgggcagta taaacttggc 2100

tccaaaacag gacctgggca gaaagctata ctttttcttc caatgtctgc taagagctga 2160

<210> 36
<211> 719
<212> PRT
<213> Homo sapiens

<400> 36

Met Gly Leu Gln Ala Cys Leu Leu Gly Leu Phe Ala Leu Ile Leu Ser
1               5                   10                  15

Gly Lys Cys Ser Tyr Ser Pro Glu Pro Asp Gln Arg Arg Thr Leu Pro
            20                  25                  30

Pro Gly Trp Val Ser Leu Gly Arg Ala Asp Pro Glu Glu Glu Leu Ser
        35                  40                  45

Leu Thr Phe Ala Leu Arg Gln Gln Asn Val Glu Arg Leu Ser Glu Leu
    50                  55                  60

Val Gln Ala Val Ser Asp Pro Ser Ser Pro Gln Tyr Gly Lys Tyr Leu
65                  70                  75                  80

Thr Leu Glu Asn Val Ala Asp Leu Val Arg Pro Ser Pro Leu Thr Leu
                85                  90                  95

His Thr Val Gln Lys Trp Leu Leu Ala Ala Gly Ala Gln Lys Cys His
            100                 105                 110

Ser Val Ile Thr Gln Asp Phe Leu Thr Cys Trp Leu Ser Ile Arg Gln
        115                 120                 125

Ala Glu Leu Leu Leu Pro Gly Ala Glu Phe His His Tyr Val Gly Gly
    130                 135                 140

Pro Thr Glu Thr His Val Val Arg Ser Pro His Pro Tyr Gln Leu Pro
145                 150                 155                 160

Gln Ala Leu Ala Pro His Val Asp Phe Val Gly Gly Leu His Arg Phe
                165                 170                 175

Pro Pro Thr Ser Ser Leu Arg Gln Arg Pro Glu Pro Gln Val Thr Gly
            180                 185                 190

Thr Val Gly Leu His Leu Gly Val Thr Pro Ser Val Ile Arg Lys Arg
        195                 200                 205

Tyr Asn Leu Thr Ser Gln Asp Val Gly Ser Gly Thr Ser Asn Asn Ser
    210                 215                 220

Gln Ala Cys Ala Gln Phe Leu Glu Gln Tyr Phe His Asp Ser Asp Leu
225                 230                 235                 240

Ala Gln Phe Met Arg Leu Phe Gly Gly Asn Phe Ala His Gln Ala Ser
                245                 250                 255

Val Ala Arg Val Val Gly Gln Gln Gly Arg Gly Arg Ala Gly Ile Glu
            260                 265                 270

Ala Ser Leu Asp Val Gln Tyr Leu Met Ser Ala Gly Ala Asn Ile Ser
        275                 280                 285

Thr Trp Val Tyr Ser Ser Pro Gly Arg His Glu Gly Gln Glu Pro Phe
    290                 295                 300

Leu Gln Trp Leu Met Leu Leu Ser Asn Glu Ser Ala Leu Pro His Val
305                 310                 315                 320

His Thr Val Ser Tyr Gly Asp Asp Glu Asp Ser Leu Ser Ser Ala Tyr
                325                 330                 335

Ile Gln Arg Val Asn Thr Glu Leu Met Lys Ala Ala Ala Arg Gly Leu
            340                 345                 350

Thr Leu Leu Phe Ala Ser Gly Asp Ser Gly Ala Gly Cys Trp Ser Val
        355                 360                 365

Ser Gly Arg His Gln Phe Arg Pro Thr Phe Pro Ala Ser Ser Pro Tyr
    370                 375                 380

Val Thr Thr Val Gly Gly Thr Ser Phe Gln Glu Pro Phe Leu Ile Thr
385                 390                 395                 400

Asn Glu Ile Val Asp Tyr Ile Ser Gly Gly Gly Phe Ser Asn Val Phe
                405                 410                 415

Pro Arg Pro Ser Tyr Gln Glu Glu Ala Val Thr Lys Phe Leu Ser Ser
            420                 425                 430

Ser Pro His Leu Pro Pro Ser Ser Tyr Phe Asn Ala Ser Gly Arg Ala
        435                 440                 445

Tyr Pro Asp Val Ala Ala Leu Ser Asp Gly Tyr Trp Val Val Ser Asn
    450                 455                 460

Arg Val Pro Ile Pro Trp Val Ser Gly Thr Ser Ala Ser Thr Pro Val
465                 470                 475                 480

Phe Gly Gly Ile Leu Ser Leu Ile Asn Glu His Arg Ile Leu Ser Gly
                485                 490                 495

Arg Pro Pro Leu Gly Phe Leu Asn Pro Arg Leu Tyr Gln Gln His Gly
            500                 505                 510

Ala Gly Leu Phe Asp Val Thr Arg Gly Cys His Glu Ser Cys Leu Asp
        515                 520                 525

Glu Glu Val Glu Gly Gln Gly Phe Cys Ser Gly Pro Gly Trp Asp Pro
    530                 535                 540

Val Thr Gly Trp Gly Thr Pro Asn Phe Pro Ala Leu Leu Lys Thr Leu
545                 550                 555                 560

Leu Asn Pro Arg Ser Val Asp Ile Glu Gly Arg Gly Ile Pro Ala Leu
                565                 570                 575

Pro Glu Asp Gly Gly Ser Gly Ala Phe Pro Pro Gly His Phe Lys Asp
            580                 585                 590

Pro Lys Arg Leu Tyr Cys Lys Asn Gly Gly Phe Phe Leu Arg Ile His
        595                 600                 605

Pro Asp Gly Arg Val Asp Gly Val Arg Glu Lys Ser Asp Pro His Ile
    610                 615                 620

Lys Leu Gln Leu Gln Ala Glu Glu Arg Gly Val Val Ser Ile Lys Gly
625                 630                 635                 640

Val Ser Ala Asn Arg Tyr Leu Ala Met Lys Glu Asp Gly Arg Leu Leu
                645                 650                 655

Ala Ser Lys Ser Val Thr Asp Glu Cys Phe Phe Phe Glu Arg Leu Glu
            660                 665                 670

Ser Asn Asn Tyr Asn Thr Tyr Arg Ser Arg Lys Tyr Thr Ser Trp Tyr
        675                 680                 685

Val Ala Leu Lys Arg Thr Gly Gln Tyr Lys Leu Gly Ser Lys Thr Gly
    690                 695                 700

Pro Gly Gln Lys Ala Ile Leu Phe Leu Pro Met Ser Ala Lys Ser
705                 710                 715

<210>  37
<211>  2160
<212>  DNA
<213>  Homo sapiens

<400>  37
atgggactcc aagcctgcct cctagggctc tttgccctca tcctctctgg caaatgcagt      60
tacagcccgg agcccgacca gcggaggacg ctgcccccag gctgggtgtc cctgggccgt     120
gcggaccctg aggaagagct gagtctcacc tttgccctga gacagcagaa tgtggaaaga     180
ctctcggagc tggtgcaggc tgtgtcggat cccagctctc ctcaatacgg aaaatacctg     240
accctagaga atgtggctga tctggtgagg ccatccccac tgaccctcca cacggtgcaa     300
aaatggctct tggcagccgg agcccagaag tgccattctg tgatcacaca ggactttctg     360
acttgctggc tgagcatccg acaagcagag ctgctgctcc ctggggctga gtttcatcac     420
tatgtgggag gacctacgga aacccatgtt gtaaggtccc cacatcccta ccagcttcca     480
caggccttgg ccccccatgt ggactttgtg gggggactgc accgttttcc cccaacatca     540
tccctgaggc aacgtcctga gccgcaggtg acagggactg taggcctgca tctgggggta     600
accccctctg tgatccgtaa gcgatacaac ttgacctcac aagacgtggg ctctggcacc     660
agcaataaca gccaagcctg tgcccagttc ctggagcagt atttccatga ctcagacctg     720
gctcagttca tgcgcctctt cggtggcaac tttgcacatc aggcatcagt agcccgtgtg     780
gttggacaac agggccgggg ccgggccggg attgaggcca gtctagatgt gcagtacctg     840
atgagtgctg gtgccaacat ctccacctgg gtctacagta gccctggccg gcatgaggga     900
caggagccct tcctgcagtg gctcatgctg ctcagtaatg agtcagccct gccacatgtg     960
catactgtga gctatggaga tgatgaggac tccctcagca gcgcctacat ccagcgggtc    1020
aacactgagc tcatgaaggc tgccgctcgg ggtctcaccc tgctcttcgc ctcaggtgac    1080
agtggggccg ggtgttggtc tgtctctgga agacaccagt tccgccctac cttccctgcc    1140
tccagcccct atgtcaccac agtgggaggc acatccttcc aggaaccttt cctcatcaca    1200
aatgaaattg ttgactatat cagtggtggt ggcttcagca atgtgttccc acggccttca    1260
taccaggagg aagctgtaac gaagttcctg agctctagcc cccacctgcc accatccagt    1320
tacttcaatg ccagtggccg tgcctaccca gatgtggctg cactttctga tggctactgg    1380
gtggtcagca acagagtgcc cattccatgg gtgtccggaa cctcggcctc tactccagtg    1440
tttgggggga tcctatcctt gatcaatgag cacaggatcc ttagtggccg cccccctctt    1500
ggctttctca acccaaggct ctaccagcag catggggcag gactctttga tgtaacccgt    1560
ggctgccatg agtcctgtct ggatgaagag gtagagggcc agggtttctg ctctggtcct    1620

ggctgggatc ctgtaacagg ctggggaaca cccaacttcc cagctttgct gaagactcta 1680

ctcaacccca gatccgtcga catcgaaggt agaggcattc ccgccttgcc cgaggatggc 1740

ggcagcggcg ccttcccgcc cggccacttc aaggacccca agcggctgta ctgcaaaaac 1800

gggggcttct tcctgcgcat ccaccccgac ggccgagttg acggggtccg ggagaagagc 1860

gaccctcaca tcaagctaca acttcaagca gaagagagag gagttgtgtc tatcaaagga 1920

gtgtctgcta accgttacct ggctatgaag gaagatggaa gattactggc ttctaaatct 1980

gttacggatg agtgtttctt ttttgcacga ttggaatcta ataactacaa tacttaccgg 2040

tcaaggaaat acaccagttg gtatgtggca ctgaaacgaa ctgggcagta taaacttggc 2100

tccaaaacag gacctgggca gaaagctata ctttttcttc caatgtctgc taagagctga 2160

<210> 38
<211> 719
<212> PRT
<213> Homo sapiens

<400> 38

Met Gly Leu Gln Ala Cys Leu Leu Gly Leu Phe Ala Leu Ile Leu Ser
1               5                   10                  15

Gly Lys Cys Ser Tyr Ser Pro Glu Pro Asp Gln Arg Arg Thr Leu Pro
            20                  25                  30

Pro Gly Trp Val Ser Leu Gly Arg Ala Asp Pro Glu Glu Glu Leu Ser
        35                  40                  45

Leu Thr Phe Ala Leu Arg Gln Gln Asn Val Glu Arg Leu Ser Glu Leu
    50                  55                  60

Val Gln Ala Val Ser Asp Pro Ser Ser Pro Gln Tyr Gly Lys Tyr Leu
65                  70                  75                  80

Thr Leu Glu Asn Val Ala Asp Leu Val Arg Pro Ser Pro Leu Thr Leu
                85                  90                  95

His Thr Val Gln Lys Trp Leu Leu Ala Ala Gly Ala Gln Lys Cys His
            100                 105                 110

Ser Val Ile Thr Gln Asp Phe Leu Thr Cys Trp Leu Ser Ile Arg Gln
        115                 120                 125

Ala Glu Leu Leu Leu Pro Gly Ala Glu Phe His His Tyr Val Gly Gly
    130                 135                 140

Pro Thr Glu Thr His Val Val Arg Ser Pro His Pro Tyr Gln Leu Pro
145                 150                 155                 160

Gln Ala Leu Ala Pro His Val Asp Phe Val Gly Gly Leu His Arg Phe

                165                 170                 175

Pro Pro Thr Ser Ser Leu Arg Gln Arg Pro Glu Pro Gln Val Thr Gly
            180                 185                 190

Thr Val Gly Leu His Leu Gly Val Thr Pro Ser Val Ile Arg Lys Arg
        195                 200                 205

Tyr Asn Leu Thr Ser Gln Asp Val Gly Ser Gly Thr Ser Asn Asn Ser
    210                 215                 220

Gln Ala Cys Ala Gln Phe Leu Glu Gln Tyr Phe His Asp Ser Asp Leu
225                 230                 235                 240

Ala Gln Phe Met Arg Leu Phe Gly Gly Asn Phe Ala His Gln Ala Ser
                245                 250                 255

Val Ala Arg Val Val Gly Gln Gln Gly Arg Gly Arg Ala Gly Ile Glu
            260                 265                 270

Ala Ser Leu Asp Val Gln Tyr Leu Met Ser Ala Gly Ala Asn Ile Ser
        275                 280                 285

Thr Trp Val Tyr Ser Ser Pro Gly Arg His Glu Gly Gln Glu Pro Phe
    290                 295                 300

Leu Gln Trp Leu Met Leu Leu Ser Asn Glu Ser Ala Leu Pro His Val
305                 310                 315                 320

His Thr Val Ser Tyr Gly Asp Asp Glu Asp Ser Leu Ser Ser Ala Tyr
                325                 330                 335

Ile Gln Arg Val Asn Thr Glu Leu Met Lys Ala Ala Ala Arg Gly Leu
            340                 345                 350

Thr Leu Leu Phe Ala Ser Gly Asp Ser Gly Ala Gly Cys Trp Ser Val
        355                 360                 365

Ser Gly Arg His Gln Phe Arg Pro Thr Phe Pro Ala Ser Ser Pro Tyr
    370                 375                 380

Val Thr Thr Val Gly Gly Thr Ser Phe Gln Glu Pro Phe Leu Ile Thr
385                 390                 395                 400

Asn Glu Ile Val Asp Tyr Ile Ser Gly Gly Gly Phe Ser Asn Val Phe
                405                 410                 415

Pro Arg Pro Ser Tyr Gln Glu Glu Ala Val Thr Lys Phe Leu Ser Ser
            420                 425                 430

Ser Pro His Leu Pro Pro Ser Ser Tyr Phe Asn Ala Ser Gly Arg Ala

435                 440                 445

Tyr Pro Asp Val Ala Ala Leu Ser Asp Gly Tyr Trp Val Val Ser Asn
    450                 455                 460

Arg Val Pro Ile Pro Trp Val Ser Gly Thr Ser Ala Ser Thr Pro Val
465                 470                 475                 480

Phe Gly Gly Ile Leu Ser Leu Ile Asn Glu His Arg Ile Leu Ser Gly
                485                 490                 495

Arg Pro Pro Leu Gly Phe Leu Asn Pro Arg Leu Tyr Gln Gln His Gly
            500                 505                 510

Ala Gly Leu Phe Asp Val Thr Arg Gly Cys His Glu Ser Cys Leu Asp
        515                 520                 525

Glu Glu Val Glu Gly Gln Gly Phe Cys Ser Gly Pro Gly Trp Asp Pro
    530                 535                 540

Val Thr Gly Trp Gly Thr Pro Asn Phe Pro Ala Leu Leu Lys Thr Leu
545                 550                 555                 560

Leu Asn Pro Arg Ser Val Asp Ile Glu Gly Arg Gly Ile Pro Ala Leu
                565                 570                 575

Pro Glu Asp Gly Gly Ser Gly Ala Phe Pro Pro Gly His Phe Lys Asp
            580                 585                 590

Pro Lys Arg Leu Tyr Cys Lys Asn Gly Gly Phe Phe Leu Arg Ile His
        595                 600                 605

Pro Asp Gly Arg Val Asp Gly Val Arg Glu Lys Ser Asp Pro His Ile
    610                 615                 620

Lys Leu Gln Leu Gln Ala Glu Glu Arg Gly Val Val Ser Ile Lys Gly
625                 630                 635                 640

Val Ser Ala Asn Arg Tyr Leu Ala Met Lys Glu Asp Gly Arg Leu Leu
                645                 650                 655

Ala Ser Lys Ser Val Thr Asp Glu Cys Phe Phe Phe Ala Arg Leu Glu
            660                 665                 670

Ser Asn Asn Tyr Asn Thr Tyr Arg Ser Arg Lys Tyr Thr Ser Trp Tyr
        675                 680                 685

Val Ala Leu Lys Arg Thr Gly Gln Tyr Lys Leu Gly Ser Lys Thr Gly
    690                 695                 700

Pro Gly Gln Lys Ala Ile Leu Phe Leu Pro Met Ser Ala Lys Ser

705                    710                    715

<210>  39
<211>  2160
<212>  DNA
<213>  Homo sapiens

<400>  39
atgggactcc aagcctgcct cctagggctc tttgccctca tcctctctgg caaatgcagt      60

tacagcccgg agcccgacca gcggaggacg ctgcccccag gctgggtgtc cctgggccgt     120

gcggaccctg aggaagagct gagtctcacc tttgccctga gacagcagaa tgtggaaaga     180

ctctcggagc tggtgcaggc tgtgtcggat cccagctctc ctcaatacgg aaaatacctg     240

accctagaga atgtggctga tctggtgagg ccatccccac tgaccctcca cacggtgcaa     300

aaatggctct tggcagccgg agcccagaag tgccattctg tgatcacaca ggactttctg     360

acttgctggc tgagcatccg acaagcagag ctgctgctcc ctggggctga gtttcatcac     420

tatgtgggag gacctacgga aacccatgtt gtaaggtccc cacatcccta ccagcttcca     480

caggccttgg ccccccatgt ggactttgtg gggggactgc accgttttcc cccaacatca     540

tccctgaggc aacgtcctga gccgcaggtg acagggactg taggcctgca tctgggggta     600

accccctctg tgatccgtaa gcgatacaac ttgacctcac aagacgtggg ctctggcacc     660

agcaataaca gccaagcctg tgcccagttc ctggagcagt atttccatga ctcagacctg     720

gctcagttca tgcgcctctt cggtggcaac tttgcacatc aggcatcagt agcccgtgtg     780

gttggacaac agggccgggg ccgggccggg attgaggcca gtctagatgt gcagtacctg     840

atgagtgctg gtgccaacat ctccacctgg gtctacagta gccctggccg gcatgaggga     900

caggagccct tcctgcagtg gctcatgctg ctcagtaatg agtcagccct gccacatgtg     960

catactgtga gctatggaga tgatgaggac tccctcagca gcgcctacat ccagcgggtc    1020

aacactgagc tcatgaaggc tgccgctcgg ggtctcaccc tgctcttcgc ctcaggtgac    1080

agtggggccg ggtgttggtc tgtctctgga agacaccagt tccgccctac cttccctgcc    1140

tccagcccct atgtcaccac agtgggaggc acatccttcc aggaaccttt cctcatcaca    1200

aatgaaattg ttgactatat cagtggtggt ggcttcagca atgtgttccc acggccttca    1260

taccaggagg aagctgtaac gaagttcctg agctctagcc cccacctgcc accatccagt    1320

tacttcaatg ccagtggccg tgcctaccca gatgtggctg cactttctga tggctactgg    1380

gtggtcagca acagagtgcc cattccatgg gtgtccggaa cctcggcctc tactccagtg    1440

tttgggggga tcctatcctt gatcaatgag cacaggatcc ttagtggccg cccccctctt    1500

ggctttctca acccaaggct ctaccagcag catggggcag gactctttga tgtaacccgt    1560

ggctgccatg agtcctgtct ggatgaagag gtagagggcc agggtttctg ctctggtcct    1620

ggctgggatc ctgtaacagg ctggggaaca cccaacttcc cagctttgct gaagactcta    1680

ctcaacccca gatccgtcga catcgaaggt agaggcattc ccgccttgcc cgaggatggc    1740

ggcagcggcg ccttcccgcc cggccacttc aaggacccca agcggctgta ctgcaaaaac    1800

gggggcttct tcctgcgcat ccaccccgac ggccgagttg acgggacaag ggacaggagc    1860

gaccagcaca ttcagctgca gctcagtgca gaagagagag gagttgtgtc tatcaaagga    1920

gtgtctgcta accgttacct ggctatgaag gaagatggaa gattactggc ttctaaatct    1980

gttacggatg agtgtttctt ttttgaacga ttggaatcta ataactacaa tacttaccgg    2040

tcaaggaaat acaccagttg gtatgtggca ctgaaacgaa ctgggcagta taaacttggc    2100

tccaaaacag gacctgggca gaaagctata ctttttcttc caatgtctgc taagagctga    2160

<210>  40
<211>  719
<212>  PRT
<213>  Homo sapiens

<400>  40

Met Gly Leu Gln Ala Cys Leu Leu Gly Leu Phe Ala Leu Ile Leu Ser
1               5                   10                  15

Gly Lys Cys Ser Tyr Ser Pro Glu Pro Asp Gln Arg Arg Thr Leu Pro
            20                  25                  30

Pro Gly Trp Val Ser Leu Gly Arg Ala Asp Pro Glu Glu Glu Leu Ser
        35                  40                  45

Leu Thr Phe Ala Leu Arg Gln Gln Asn Val Glu Arg Leu Ser Glu Leu
    50                  55                  60

Val Gln Ala Val Ser Asp Pro Ser Ser Pro Gln Tyr Gly Lys Tyr Leu
65                  70                  75                  80

Thr Leu Glu Asn Val Ala Asp Leu Val Arg Pro Ser Pro Leu Thr Leu
                85                  90                  95

His Thr Val Gln Lys Trp Leu Leu Ala Ala Gly Ala Gln Lys Cys His
            100                 105                 110

Ser Val Ile Thr Gln Asp Phe Leu Thr Cys Trp Leu Ser Ile Arg Gln
        115                 120                 125

Ala Glu Leu Leu Leu Pro Gly Ala Glu Phe His His Tyr Val Gly Gly
    130                 135                 140

Pro Thr Glu Thr His Val Val Arg Ser Pro His Pro Tyr Gln Leu Pro
145                 150                 155                 160

Gln Ala Leu Ala Pro His Val Asp Phe Val Gly Gly Leu His Arg Phe
                165                 170                 175

Pro Pro Thr Ser Ser Leu Arg Gln Arg Pro Glu Pro Gln Val Thr Gly
            180                 185                 190

Thr Val Gly Leu His Leu Gly Val Thr Pro Ser Val Ile Arg Lys Arg
        195                 200                 205

Tyr Asn Leu Thr Ser Gln Asp Val Gly Ser Gly Thr Ser Asn Asn Ser
    210                 215                 220

Gln Ala Cys Ala Gln Phe Leu Glu Gln Tyr Phe His Asp Ser Asp Leu
225                 230                 235                 240

Ala Gln Phe Met Arg Leu Phe Gly Gly Asn Phe Ala His Gln Ala Ser
                245                 250                 255

Val Ala Arg Val Val Gly Gln Gln Gly Arg Gly Arg Ala Gly Ile Glu
            260                 265                 270

Ala Ser Leu Asp Val Gln Tyr Leu Met Ser Ala Gly Ala Asn Ile Ser
        275                 280                 285

Thr Trp Val Tyr Ser Ser Pro Gly Arg His Glu Gly Gln Glu Pro Phe
    290                 295                 300

Leu Gln Trp Leu Met Leu Leu Ser Asn Glu Ser Ala Leu Pro His Val
305                 310                 315                 320

His Thr Val Ser Tyr Gly Asp Asp Glu Asp Ser Leu Ser Ser Ala Tyr
                325                 330                 335

Ile Gln Arg Val Asn Thr Glu Leu Met Lys Ala Ala Ala Arg Gly Leu
            340                 345                 350

Thr Leu Leu Phe Ala Ser Gly Asp Ser Gly Ala Gly Cys Trp Ser Val
        355                 360                 365

Ser Gly Arg His Gln Phe Arg Pro Thr Phe Pro Ala Ser Ser Pro Tyr
    370                 375                 380

Val Thr Thr Val Gly Gly Thr Ser Phe Gln Glu Pro Phe Leu Ile Thr
385                 390                 395                 400

Asn Glu Ile Val Asp Tyr Ile Ser Gly Gly Gly Phe Ser Asn Val Phe
                405                 410                 415

Pro Arg Pro Ser Tyr Gln Glu Glu Ala Val Thr Lys Phe Leu Ser Ser
            420                 425                 430

Ser Pro His Leu Pro Pro Ser Ser Tyr Phe Asn Ala Ser Gly Arg Ala
        435                 440                 445

Tyr Pro Asp Val Ala Ala Leu Ser Asp Gly Tyr Trp Val Val Ser Asn
    450                 455                 460

Arg Val Pro Ile Pro Trp Val Ser Gly Thr Ser Ala Ser Thr Pro Val
465                 470                 475                 480

Phe Gly Gly Ile Leu Ser Leu Ile Asn Glu His Arg Ile Leu Ser Gly
                485                 490                 495

Arg Pro Pro Leu Gly Phe Leu Asn Pro Arg Leu Tyr Gln Gln His Gly
            500                 505                 510

Ala Gly Leu Phe Asp Val Thr Arg Gly Cys His Glu Ser Cys Leu Asp
        515                 520                 525

Glu Glu Val Glu Gly Gln Gly Phe Cys Ser Gly Pro Gly Trp Asp Pro
    530                 535                 540

Val Thr Gly Trp Gly Thr Pro Asn Phe Pro Ala Leu Leu Lys Thr Leu
545                 550                 555                 560

Leu Asn Pro Arg Ser Val Asp Ile Glu Gly Arg Gly Ile Pro Ala Leu
                565                 570                 575

Pro Glu Asp Gly Gly Ser Gly Ala Phe Pro Pro Gly His Phe Lys Asp
            580                 585                 590

Pro Lys Arg Leu Tyr Cys Lys Asn Gly Gly Phe Phe Leu Arg Ile His
        595                 600                 605

Pro Asp Gly Arg Val Asp Gly Thr Arg Asp Arg Ser Asp Gln His Ile
    610                 615                 620

Gln Leu Gln Leu Ser Ala Glu Glu Arg Gly Val Val Ser Ile Lys Gly
625                 630                 635                 640

Val Ser Ala Asn Arg Tyr Leu Ala Met Lys Glu Asp Gly Arg Leu Leu
                645                 650                 655

Ala Ser Lys Ser Val Thr Asp Glu Cys Phe Phe Phe Glu Arg Leu Glu
            660                 665                 670

Ser Asn Asn Tyr Asn Thr Tyr Arg Ser Arg Lys Tyr Thr Ser Trp Tyr
        675                 680                 685

Val Ala Leu Lys Arg Thr Gly Gln Tyr Lys Leu Gly Ser Lys Thr Gly
    690                 695                 700

Pro Gly Gln Lys Ala Ile Leu Phe Leu Pro Met Ser Ala Lys Ser
705                 710                 715

<210>  41
<211>  2160
<212>  DNA

<213>  Homo sapiens

<400>  41
atgggactcc aagcctgcct cctagggctc tttgccctca tcctctctgg caaatgcagt      60
tacagcccgg agcccgacca gcggaggacg ctgcccccag gctgggtgtc cctgggccgt     120
gcggaccctg aggaagagct gagtctcacc tttgccctga gacagcagaa tgtggaaaga     180
ctctcggagc tggtgcaggc tgtgtcggat cccagctctc ctcaatacgg aaaatacctg     240
accctagaga atgtggctga tctggtgagg ccatccccac tgaccctcca cacggtgcaa     300
aaatggctct tggcagccgg agcccagaag tgccattctg tgatcacaca ggactttctg     360
acttgctggc tgagcatccg acaagcagag ctgctgctcc ctggggctga gtttcatcac     420
tatgtgggag gacctacgga aacccatgtt gtaaggtccc cacatcccta ccagcttcca     480
caggccttgg ccccccatgt ggactttgtg gggggactgc accgttttcc cccaacatca     540
tccctgaggc aacgtcctga gccgcaggtg acagggactg taggcctgca tctgggggta     600
accccctctg tgatccgtaa gcgatacaac ttgacctcac aagacgtggg ctctggcacc     660
agcaataaca gccaagcctg tgcccagttc ctggagcagt atttccatga ctcagacctg     720
gctcagttca tgcgcctctt cggtggcaac tttgcacatc aggcatcagt agcccgtgtg     780
gttggacaac agggccgggg ccgggccggg attgaggcca gtctagatgt gcagtacctg     840
atgagtgctg gtgccaacat ctccacctgg gtctacagta gccctggccg gcatgaggga     900
caggagccct tcctgcagtg gctcatgctg ctcagtaatg agtcagccct gccacatgtg     960
catactgtga gctatggaga tgatgaggac tccctcagca gcgcctacat ccagcgggtc    1020
aacactgagc tcatgaaggc tgccgctcgg ggtctcaccc tgctcttcgc ctcaggtgac    1080
agtggggccg ggtgttggtc tgtctctgga agacaccagt tccgccctac cttccctgcc    1140
tccagcccct atgtcaccac agtgggaggc acatccttcc aggaaccttt cctcatcaca    1200
aatgaaattg ttgactatat cagtggtggt ggcttcagca atgtgttccc acggccttca    1260
taccaggagg aagctgtaac gaagttcctg agctctagcc cccacctgcc accatccagt    1320
tacttcaatg ccagtggccg tgcctaccca gatgtggctg cactttctga tggctactgg    1380
gtggtcagca acagagtgcc cattccatgg gtgtccggaa cctcggcctc tactccagtg    1440
tttgggggga tcctatcctt gatcaatgag cacaggatcc ttagtggccg cccccctctt    1500
ggctttctca acccaaggct ctaccagcag catggggcag gactctttga tgtaacccgt    1560
ggctgccatg agtcctgtct ggatgaagag gtagagggcc agggtttctg ctctggtcct    1620
ggctgggatc ctgtaacagg ctggggaaca cccaacttcc cagctttgct gaagactcta    1680
ctcaacccca gatccgtcga catcgaaggt agaggcattc ccgccttgcc cgaggatggc    1740
ggcagcggcg ccttcccgcc cggccacttc aaggacccca agcggctgta ctgcaaaaac    1800
gggggcttct tcctgcgcat ccaccccgac ggccgagttg acgggacaag ggacaggagc    1860
gaccagcaca ttcagctgca gctcagtgca gaagagagag gagttgtgtc tatcaaagga    1920
gtgtctgcta accgttacct ggctatgaag gaagatggaa gattactggc ttctaaatct    1980

gttacggatg agtgtttctt ttttgcacga ttggaatcta ataactacaa tacttaccgg 2040

tcaaggaaat acaccagttg gtatgtggca ctgaaacgaa ctgggcagta taaacttggc 2100

tccaaaacag gacctgggca gaaagctata ctttttcttc caatgtctgc taagagctga 2160

<210> 42
<211> 719
<212> PRT
<213> Homo sapiens

<400> 42

<210> 43
<211> 276
<212> DNA
<213> Homo sapiens

<400> 43
atgcagccct ccagccttct gccgctcgcc ctctgcctgc tggctgcacc cgccggatct 60

tccaagccac aagcactggc cacaccaaac aaggaggagc acgggaaaag aaagaagaaa 120

ggcaaggggc tagggaagaa gagggaccca tgtcttcgga aatacaagga cttctgcatc 180

catggagaat gcaaatatgt gaaggagctc cgggctccct cctgcatctg ccacccgggt 240

taccatggag agaggtgtca tgggctgagc ggatct 276

<210> 44
<211> 141
<212> DNA
<213> Homo sapiens

<400> 44
atgcagccct ccagccttct gccgctcgcc ctctgcctgc tggctgcacc cgccggatct 60

gggaaaagaa agaagaaagg caaggggcta gggaagaaga gggacccatc tcttcggaaa 120

tacaaggact tctccggatc t 141

<210> 45
<211> 1725
<212> DNA
<213> Homo sapiens

<400> 45
atgcagccct ccagccttct gccgctcgcc ctctgcctgc tggctgcacc cgccggatct 60

tccaagccac aagcactggc cacaccaaac aaggaggagc acgggaaaag aaagaagaaa 120

ggcaaggggc tagggaagaa gagggaccca tgtcttcgga aatacaagga cttctgcatc 180

catggagaat gcaaatatgt gaaggagctc cgggctccct cctgcatctg ccacccgggt 240

taccatggag agaggtgtca tgggctgagc ggatctcgtc cccggaacgc actgctgctc 300

ctcgcggatg acggaggctt tgagagtggc gcgtacaaca acagcgccat cgccaccccg 360

cacctggacg ccttggcccg ccgcagcctc ctctttcgca atgccttcac ctcggtcagc 420

agctgctctc ccagccgcgc cagcctcctc actggcctgc cccagcatca gaatgggatg 480

tacgggctgc accaggacgt gcaccacttc aactccttcg acaaggtgcg gagcctgccg 540

ctgctgctca gccaagctgg tgtgcgcaca ggcatcatcg ggaagaagca cgtggggccg 600

gagaccgtgt acccgtttga ctttgcgtac acggaggaga atggctccgt cctccaggtg 660

gggcggaaca tcactagaat taagctgctc gtccggaaat tcctgcagac tcaggatgac 720

cggcctttct tcctctacgt cgccttccac gacccccacc gctgtgggca ctcccagccc 780

cagtacggaa ccttctgtga gaagtttggc aacggagaga gcggcatggg tcgtatccca 840

gactggaccc cccaggccta cgacccactg gacgtgctgg tgccttactt cgtccccaac 900

accccggcag cccgagccga cctggccgct cagtacacca ccgtaggccg catggaccaa 960

ggagttggac tggtgctcca ggagctgcgt gacgccggtg tcctgaacga cacactggtg 1020

atcttcacgt ccgacaacgg gatccccttc cccagcggca ggaccaacct gtactggccg 1080

ggcactgctg aacccttact ggtgtcatcc ccggagcacc caaaacgctg gggccaagtc 1140

agcgaggcct acgtgagcct cctagacctc acgcccacca tcttggattg gttctcgatc 1200

ccgtacccca gctacgccat ctttggctcg aagaccatcc acctcactgg ccggtccctc 1260

ctgccggcgc tggaggccga gcccctctgg gccaccgtct ttggcagcca gagccaccac 1320

gaggtcacca tgtcctaccc catgcgctcc gtgcagcacc ggcacttccg cctcgtgcac 1380

aacctcaact tcaagatgcc ctttcccatc gaccaggact tctacgtctc acccaccttc 1440

caggacctcc tgaaccgcac tacagctggt cagcccacgg gctggtacaa ggacctccgt 1500

cattactact accgggcgcg ctgggagctc tacgaccgga gccgggaccc ccacgagacc 1560

cagaacctgg ccaccgaccc gcgctttgct cagcttctgg agatgcttcg ggaccagctg 1620

gccaagtggc agtgggagac ccacgacccc tgggtgtgcg cccccgacgg cgtcctggag 1680

gagaagctct ctccccagtg ccagcccctc cacaatgagc tgtaa 1725

<210> 46
<211> 574
<212> PRT
<213> Homo sapiens

<400> 46

Met Gln Pro Ser Ser Leu Leu Pro Leu Ala Leu Cys Leu Leu Ala Ala
1               5                   10                  15

Pro Ala Gly Ser Ser Lys Pro Gln Ala Leu Ala Thr Pro Asn Lys Glu
            20                  25                  30

Glu His Gly Lys Arg Lys Lys Lys Gly Lys Gly Leu Gly Lys Lys Arg
        35                  40                  45

Asp Pro Cys Leu Arg Lys Tyr Lys Asp Phe Cys Ile His Gly Glu Cys
    50                  55                  60

Lys Tyr Val Lys Glu Leu Arg Ala Pro Ser Cys Ile Cys His Pro Gly
65                  70                  75                  80

Tyr His Gly Glu Arg Cys His Gly Leu Ser Gly Ser Arg Pro Arg Asn
                85                  90                  95

Ala Leu Leu Leu Leu Ala Asp Asp Gly Gly Phe Glu Ser Gly Ala Tyr
            100                 105                 110

Asn Asn Ser Ala Ile Ala Thr Pro His Leu Asp Ala Leu Ala Arg Arg
        115                 120                 125

Ser Leu Leu Phe Arg Asn Ala Phe Thr Ser Val Ser Ser Cys Ser Pro
    130                 135                 140

Ser Arg Ala Ser Leu Leu Thr Gly Leu Pro Gln His Gln Asn Gly Met
145                 150                 155                 160

Tyr Gly Leu His Gln Asp Val His His Phe Asn Ser Phe Asp Lys Val
                165                 170                 175

Arg Ser Leu Pro Leu Leu Leu Ser Gln Ala Gly Val Arg Thr Gly Ile
            180                 185                 190

Ile Gly Lys Lys His Val Gly Pro Glu Thr Val Tyr Pro Phe Asp Phe
        195                 200                 205

Ala Tyr Thr Glu Glu Asn Gly Ser Val Leu Gln Val Gly Arg Asn Ile
    210                 215                 220

Thr Arg Ile Lys Leu Leu Val Arg Lys Phe Leu Gln Thr Gln Asp Asp
225                 230                 235                 240

Arg Pro Phe Phe Leu Tyr Val Ala Phe His Asp Pro His Arg Cys Gly
                245                 250                 255

His Ser Gln Pro Gln Tyr Gly Thr Phe Cys Glu Lys Phe Gly Asn Gly
            260                 265                 270

Glu Ser Gly Met Gly Arg Ile Pro Asp Trp Thr Pro Gln Ala Tyr Asp
        275                 280                 285

Pro Leu Asp Val Leu Val Pro Tyr Phe Val Pro Asn Thr Pro Ala Ala
    290                 295                 300

Arg Ala Asp Leu Ala Ala Gln Tyr Thr Thr Val Gly Arg Met Asp Gln
305                 310                 315                 320

Gly Val Gly Leu Val Leu Gln Glu Leu Arg Asp Ala Gly Val Leu Asn
                325                 330                 335

Asp Thr Leu Val Ile Phe Thr Ser Asp Asn Gly Ile Pro Phe Pro Ser
            340                 345                 350

Gly Arg Thr Asn Leu Tyr Trp Pro Gly Thr Ala Glu Pro Leu Leu Val
        355                 360                 365

Ser Ser Pro Glu His Pro Lys Arg Trp Gly Gln Val Ser Glu Ala Tyr
    370                 375                 380

Val Ser Leu Leu Asp Leu Thr Pro Thr Ile Leu Asp Trp Phe Ser Ile
385                 390                 395                 400

Pro Tyr Pro Ser Tyr Ala Ile Phe Gly Ser Lys Thr Ile His Leu Thr
                405                 410                 415

Gly Arg Ser Leu Leu Pro Ala Leu Glu Ala Glu Pro Leu Trp Ala Thr
            420                 425                 430

Val Phe Gly Ser Gln Ser His His Glu Val Thr Met Ser Tyr Pro Met
        435                 440                 445

Arg Ser Val Gln His Arg His Phe Arg Leu Val His Asn Leu Asn Phe
    450                 455                 460

Lys Met Pro Phe Pro Ile Asp Gln Asp Phe Tyr Val Ser Pro Thr Phe
465                 470                 475                 480

Gln Asp Leu Leu Asn Arg Thr Thr Ala Gly Gln Pro Thr Gly Trp Tyr
                485                 490                 495

Lys Asp Leu Arg His Tyr Tyr Tyr Arg Ala Arg Trp Glu Leu Tyr Asp
            500                 505                 510

Arg Ser Arg Asp Pro His Glu Thr Gln Asn Leu Ala Thr Asp Pro Arg
        515                 520                 525

Phe Ala Gln Leu Leu Glu Met Leu Arg Asp Gln Leu Ala Lys Trp Gln
    530                 535                 540

Trp Glu Thr His Asp Pro Trp Val Cys Ala Pro Asp Gly Val Leu Glu
545                 550                 555                 560

Glu Lys Leu Ser Pro Gln Cys Gln Pro Leu His Asn Glu Leu
                565                 570

<210>  47
<211>  1590
<212>  DNA
<213>  Homo sapiens

<400>  47
atgcagccct ccagccttct gccgctcgcc ctctgcctgc tggctgcacc cgccggatct      60

gggaaaagaa agaagaaagg caaggggcta gggaagaaga gggacccatc tcttcggaaa     120

tacaaggact tctccggatc tcgtccccgg aacgcactgc tgctcctcgc ggatgacgga     180

ggctttgaga gtggcgcgta caacaacagc gccatcgcca ccccgcacct ggacgccttg     240

gcccgccgca gcctcctctt tcgcaatgcc ttcacctcgg tcagcagctg ctctcccagc     300

cgcgccagcc tcctcactgg cctgccccag catcagaatg ggatgtacgg gctgcaccag     360

gacgtgcacc acttcaactc cttcgacaag gtgcggagcc tgccgctgct gctcagccaa     420

gctggtgtgc gcacaggcat catcgggaag aagcacgtgg ggccggagac cgtgtacccg     480

tttgactttg cgtacacgga ggagaatggc tccgtcctcc aggtggggcg gaacatcact     540

agaattaagc tgctcgtccg gaaattcctg cagactcagg atgaccggcc tttcttcctc     600

tacgtcgcct tccacgaccc ccaccgctgt gggcactccc agccccagta cggaaccttc     660

tgtgagaagt ttggcaacgg agagagcggc atgggtcgta tcccagactg gaccccccag      720

gcctacgacc cactggacgt gctggtgcct tacttcgtcc ccaacacccc ggcagcccga     780

gccgacctgg ccgctcagta caccaccgta ggccgcatgg accaaggagt tggactggtg     840

ctccaggagc tgcgtgacgc cggtgtcctg aacgacacac tggtgatctt cacgtccgac     900

aacgggatcc ccttccccag cggcaggacc aacctgtact ggccgggcac tgctgaaccc 960

ttactggtgt catccccgga gcacccaaaa cgctggggcc aagtcagcga ggcctacgtg 1020

agcctcctag acctcacgcc caccatcttg gattggttct cgatcccgta ccccagctac 1080

gccatctttg gctcgaagac catccacctc actggccggt ccctcctgcc ggcgctggag 1140

gccgagcccc tctgggccac cgtctttggc agccagagcc accacgaggt caccatgtcc 1200

taccccatgc gctccgtgca gcaccggcac ttccgcctcg tgcacaacct caacttcaag 1260

atgccctttc ccatcgacca ggacttctac gtctcaccca ccttccagga cctcctgaac 1320

cgcactacag ctggtcagcc cacgggctgg tacaaggacc tccgtcatta ctactaccgg 1380

gcgcgctggg agctctacga ccggagccgg gacccccacg agacccagaa cctggccacc 1440

gacccgcgct ttgctcagct tctggagatg cttcgggacc agctggccaa gtggcagtgg 1500

gagacccacg acccctgggt gtgcgccccc gacggcgtcc tggaggagaa gctctctccc 1560

cagtgccagc ccctccacaa tgagctgtaa 1590

<210> 48
<211> 529
<212> PRT
<213> Homo sapiens

<400> 48

Met Gln Pro Ser Ser Leu Leu Pro Leu Ala Leu Cys Leu Leu Ala Ala
1               5                   10                  15

Pro Ala Gly Ser Gly Lys Arg Lys Lys Lys Gly Lys Gly Leu Gly Lys
            20                  25                  30

Lys Arg Asp Pro Ser Leu Arg Lys Tyr Lys Asp Phe Ser Gly Ser Arg
        35                  40                  45

Pro Arg Asn Ala Leu Leu Leu Leu Ala Asp Asp Gly Gly Phe Glu Ser
    50                  55                  60

Gly Ala Tyr Asn Asn Ser Ala Ile Ala Thr Pro His Leu Asp Ala Leu
65                  70                  75                  80

Ala Arg Arg Ser Leu Leu Phe Arg Asn Ala Phe Thr Ser Val Ser Ser
                85                  90                  95

Cys Ser Pro Ser Arg Ala Ser Leu Leu Thr Gly Leu Pro Gln His Gln
            100                 105                 110

Asn Gly Met Tyr Gly Leu His Gln Asp Val His His Phe Asn Ser Phe
        115                 120                 125

Asp Lys Val Arg Ser Leu Pro Leu Leu Leu Ser Gln Ala Gly Val Arg
    130                 135                 140

Thr Gly Ile Ile Gly Lys Lys His Val Gly Pro Glu Thr Val Tyr Pro
145                 150                 155                 160

Phe Asp Phe Ala Tyr Thr Glu Glu Asn Gly Ser Val Leu Gln Val Gly
                165                 170                 175

Arg Asn Ile Thr Arg Ile Lys Leu Leu Val Arg Lys Phe Leu Gln Thr
            180                 185                 190

Gln Asp Asp Arg Pro Phe Phe Leu Tyr Val Ala Phe His Asp Pro His
        195                 200                 205

Arg Cys Gly His Ser Gln Pro Gln Tyr Gly Thr Phe Cys Glu Lys Phe
    210                 215                 220

Gly Asn Gly Glu Ser Gly Met Gly Arg Ile Pro Asp Trp Thr Pro Gln
225                 230                 235                 240

Ala Tyr Asp Pro Leu Asp Val Leu Val Pro Tyr Phe Val Pro Asn Thr
                245                 250                 255

Pro Ala Ala Arg Ala Asp Leu Ala Ala Gln Tyr Thr Thr Val Gly Arg
            260                 265                 270

Met Asp Gln Gly Val Gly Leu Val Leu Gln Glu Leu Arg Asp Ala Gly
        275                 280                 285

Val Leu Asn Asp Thr Leu Val Ile Phe Thr Ser Asp Asn Gly Ile Pro
    290                 295                 300

Phe Pro Ser Gly Arg Thr Asn Leu Tyr Trp Pro Gly Thr Ala Glu Pro
305                 310                 315                 320

Leu Leu Val Ser Ser Pro Glu His Pro Lys Arg Trp Gly Gln Val Ser
                325                 330                 335

Glu Ala Tyr Val Ser Leu Leu Asp Leu Thr Pro Thr Ile Leu Asp Trp
            340                 345                 350

Phe Ser Ile Pro Tyr Pro Ser Tyr Ala Ile Phe Gly Ser Lys Thr Ile
        355                 360                 365

His Leu Thr Gly Arg Ser Leu Leu Pro Ala Leu Glu Ala Glu Pro Leu
    370                 375                 380

Trp Ala Thr Val Phe Gly Ser Gln Ser His His Glu Val Thr Met Ser
385                 390                 395                 400

Tyr Pro Met Arg Ser Val Gln His Arg His Phe Arg Leu Val His Asn
                405                 410                 415

Leu Asn Phe Lys Met Pro Phe Pro Ile Asp Gln Asp Phe Tyr Val Ser
            420                 425                 430

Pro Thr Phe Gln Asp Leu Leu Asn Arg Thr Thr Ala Gly Gln Pro Thr
        435                 440                 445

Gly Trp Tyr Lys Asp Leu Arg His Tyr Tyr Tyr Arg Ala Arg Trp Glu
    450                 455                 460

Leu Tyr Asp Arg Ser Arg Asp Pro His Glu Thr Gln Asn Leu Ala Thr
465                 470                 475                 480

Asp Pro Arg Phe Ala Gln Leu Leu Glu Met Leu Arg Asp Gln Leu Ala
                485                 490                 495

Lys Trp Gln Trp Glu Thr His Asp Pro Trp Val Cys Ala Pro Asp Gly
            500                 505                 510

Val Leu Glu Glu Lys Leu Ser Pro Gln Cys Gln Pro Leu His Asn Glu
        515                 520                 525

Leu

<210>  49
<211>  1650
<212>  DNA
<213>  Homo sapiens

<400>  49
atgcagccct ccagccttct gccgctcgcc ctctgcctgc tggctgcacc cgccggatct      60

gggaaaagaa agaagaaagg caaggggcta gggaagaaga gggacccatc tcttcggaaa     120

tacaaggact tctccggatc tcgtccccgg aacgcactgc tgctcctcgc ggatgacgga     180

ggctttgaga gtggcgcgta caacaacagc gccatcgcca ccccgcacct ggacgccttg     240

gcccgccgca gcctcctctt tcgcaatgcc ttcacctcgg tcagcagctg ctctcccagc     300

cgcgccagcc tcctcactgg cctgccccag catcagaatg ggatgtacgg gctgcaccag     360

gacgtgcacc acttcaactc cttcgacaag gtgcggagcc tgccgctgct gctcagccaa     420

gctggtgtgc gcacaggcat catcgggaag aagcacgtgg ggccggagac cgtgtacccg     480

tttgactttg cgtacacgga ggagaatggc tccgtcctcc aggtggggcg gaacatcact     540

agaattaagc tgctcgtccg gaaattcctg cagactcagg atgaccggcc tttcttcctc     600

tacgtcgcct tccacgaccc ccaccgctgt gggcactccc agccccagta cggaaccttc     660

tgtgagaagt ttggcaacgg agagagcggc atgggtcgta tcccagactg gaccccccag      720

gcctacgacc cactggacgt gctggtgcct tacttcgtcc ccaacacccc ggcagcccga     780

gccgacctgg ccgctcagta caccaccgta ggccgcatgg accaaggagt tggactggtg     840

ctccaggagc tgcgtgacgc cggtgtcctg aacgacacac tggtgatctt cacgtccgac     900

aacgggatcc ccttccccag cggcaggacc aacctgtact ggccgggcac tgctgaaccc 960

ttactggtgt catccccgga gcacccaaaa cgctggggcc aagtcagcga ggcctacgtg 1020

agcctcctag acctcacgcc caccatcttg gattggttct cgatcccgta ccccagctac 1080

gccatctttg gctcgaagac catccacctc actggccggt ccctcctgcc ggcgctggag 1140

gccgagcccc tctgggccac cgtctttggc agccagagcc accacgaggt caccatgtcc 1200

taccccatgc gctccgtgca gcaccggcac ttccgcctcg tgcacaacct caacttcaag 1260

atgccctttc ccatcgacca ggacttctac gtctcaccca ccttccagga cctcctgaac 1320

cgcactacag ctggtcagcc cacgggctgg tacaaggacc tccgtcatta ctactaccgg 1380

gcgcgctggg agctctacga ccggagccgg gacccccacg agacccagaa cctggccacc 1440

gacccgcgct ttgctcagct tctggagatg cttcgggacc agctggccaa gtggcagtgg 1500

gagacccacg acccctgggt gtgcgccccc gacggcgtcc tggaggagaa gctctctccc 1560

cagtgccagc ccctccacaa tgagctgaga tcccccgggc gccagataaa gatttggttc 1620

cagaatcggc gcatgaagtg gaagaagtaa 1650

<210> 50
<211> 549
<212> PRT
<213> Homo sapiens

<400> 50

Met Gln Pro Ser Ser Leu Leu Pro Leu Ala Leu Cys Leu Leu Ala Ala
1               5                   10                  15

Pro Ala Gly Ser Gly Lys Arg Lys Lys Lys Gly Lys Gly Leu Gly Lys
            20                  25                  30

Lys Arg Asp Pro Ser Leu Arg Lys Tyr Lys Asp Phe Ser Gly Ser Arg
        35                  40                  45

Pro Arg Asn Ala Leu Leu Leu Leu Ala Asp Asp Gly Gly Phe Glu Ser
    50                  55                  60

Gly Ala Tyr Asn Asn Ser Ala Ile Ala Thr Pro His Leu Asp Ala Leu
65                  70                  75                  80

Ala Arg Arg Ser Leu Leu Phe Arg Asn Ala Phe Thr Ser Val Ser Ser
                85                  90                  95

Cys Ser Pro Ser Arg Ala Ser Leu Leu Thr Gly Leu Pro Gln His Gln
            100                 105                 110

Asn Gly Met Tyr Gly Leu His Gln Asp Val His His Phe Asn Ser Phe
        115                 120                 125

Asp Lys Val Arg Ser Leu Pro Leu Leu Leu Ser Gln Ala Gly Val Arg

130                 135                 140

Thr Gly Ile Ile Gly Lys Lys His Val Gly Pro Glu Thr Val Tyr Pro
145                 150                 155                 160

Phe Asp Phe Ala Tyr Thr Glu Glu Asn Gly Ser Val Leu Gln Val Gly
                165                 170                 175

Arg Asn Ile Thr Arg Ile Lys Leu Leu Val Arg Lys Phe Leu Gln Thr
            180                 185                 190

Gln Asp Asp Arg Pro Phe Phe Leu Tyr Val Ala Phe His Asp Pro His
        195                 200                 205

Arg Cys Gly His Ser Gln Pro Gln Tyr Gly Thr Phe Cys Glu Lys Phe
    210                 215                 220

Gly Asn Gly Glu Ser Gly Met Gly Arg Ile Pro Asp Trp Thr Pro Gln
225                 230                 235                 240

Ala Tyr Asp Pro Leu Asp Val Leu Val Pro Tyr Phe Val Pro Asn Thr
                245                 250                 255

Pro Ala Ala Arg Ala Asp Leu Ala Ala Gln Tyr Thr Thr Val Gly Arg
            260                 265                 270

Met Asp Gln Gly Val Gly Leu Val Leu Gln Glu Leu Arg Asp Ala Gly
        275                 280                 285

Val Leu Asn Asp Thr Leu Val Ile Phe Thr Ser Asp Asn Gly Ile Pro
    290                 295                 300

Phe Pro Ser Gly Arg Thr Asn Leu Tyr Trp Pro Gly Thr Ala Glu Pro
305                 310                 315                 320

Leu Leu Val Ser Ser Pro Glu His Pro Lys Arg Trp Gly Gln Val Ser
                325                 330                 335

Glu Ala Tyr Val Ser Leu Leu Asp Leu Thr Pro Thr Ile Leu Asp Trp
            340                 345                 350

Phe Ser Ile Pro Tyr Pro Ser Tyr Ala Ile Phe Gly Ser Lys Thr Ile
        355                 360                 365

His Leu Thr Gly Arg Ser Leu Leu Pro Ala Leu Glu Ala Glu Pro Leu
    370                 375                 380

Trp Ala Thr Val Phe Gly Ser Gln Ser His His Glu Val Thr Met Ser
385                 390                 395                 400

Tyr Pro Met Arg Ser Val Gln His Arg His Phe Arg Leu Val His Asn

405                 410                 415

Leu Asn Phe Lys Met Pro Phe Pro Ile Asp Gln Asp Phe Tyr Val Ser
            420                 425                 430

Pro Thr Phe Gln Asp Leu Leu Asn Arg Thr Thr Ala Gly Gln Pro Thr
        435                 440                 445

Gly Trp Tyr Lys Asp Leu Arg His Tyr Tyr Tyr Arg Ala Arg Trp Glu
    450                 455                 460

Leu Tyr Asp Arg Ser Arg Asp Pro His Glu Thr Gln Asn Leu Ala Thr
465                 470                 475                 480

Asp Pro Arg Phe Ala Gln Leu Leu Glu Met Leu Arg Asp Gln Leu Ala
                485                 490                 495

Lys Trp Gln Trp Glu Thr His Asp Pro Trp Val Cys Ala Pro Asp Gly
            500                 505                 510

Val Leu Glu Glu Lys Leu Ser Pro Gln Cys Gln Pro Leu His Asn Glu
        515                 520                 525

Leu Arg Ser Pro Gly Arg Gln Ile Lys Ile Trp Phe Gln Asn Arg Arg
    530                 535                 540

Met Lys Trp Lys Lys
545

<210>  51
<211>  1692
<212>  DNA
<213>  Homo sapiens

<400>  51
atgggactcc aagcctgcct cctagggctc tttgccctca tcctctctgg caaatgcagt      60

tacagcccgg agcccgacca gcggaggacg ctgcccccag gctgggtgtc cctgggccgt     120

gcggaccctg aggaagagct gagtctcacc tttgccctga gacagcagaa tgtggaaaga     180

ctctcggagc tggtgcaggc tgtgtcggat cccagctctc ctcaatacgg aaaatacctg     240

accctagaga atgtggctga tctggtgagg ccatccccac tgaccctcca cacggtgcaa     300

aaatggctct tggcagccgg agcccagaag tgccattctg tgatcacaca ggactttctg     360

acttgctggc tgagcatccg acaagcagag ctgctgctcc ctggggctga gtttcatcac     420

tatgtgggag gacctacgga aacccatgtt gtaaggtccc cacatcccta ccagcttcca     480

caggccttgg ccccccatgt ggactttgtg gggggactgc accgttttcc cccaacatca     540

tccctgaggc aacgtcctga gccgcaggtg acagggactg taggcctgca tctgggggta     600

accccctctg tgatccgtaa gcgatacaac ttgacctcac aagacgtggg ctctggcacc     660

agcaataaca gccaagcctg tgcccagttc ctggagcagt atttccatga ctcagacctg     720

gctcagttca tgcgcctctt cggtggcaac tttgcacatc aggcatcagt agcccgtgtg 780

gttggacaac agggccgggg ccgggccggg attgaggcca gtctagatgt gcagtacctg 840

atgagtgctg gtgccaacat ctccacctgg gtctacagta gccctggccg gcatgaggga 900

caggagccct tcctgcagtg gctcatgctg ctcagtaatg agtcagccct gccacatgtg 960

catactgtga gctatggaga tgatgaggac tccctcagca gcgcctacat ccagcgggtc 1020

aacactgagc tcatgaaggc tgccgctcgg ggtctcaccc tgctcttcgc ctcaggtgac 1080

agtggggccg ggtgttggtc tgtctctgga agacaccagt tccgccctac cttccctgcc 1140

tccagcccct atgtcaccac agtgggaggc acatccttcc aggaaccttt cctcatcaca 1200

aatgaaattg ttgactatat cagtggtggt ggcttcagca atgtgttccc acggccttca 1260

taccaggagg aagctgtaac gaagttcctg agctctagcc cccacctgcc accatccagt 1320

tacttcaatg ccagtggccg tgcctaccca gatgtggctg cactttctga tggctactgg 1380

gtggtcagca acagagtgcc cattccatgg gtgtccggaa cctcggcctc tactccagtg 1440

tttgggggga tcctatcctt gatcaatgag cacaggatcc ttagtggccg cccccctctt 1500

ggctttctca acccaaggct ctaccagcag catggggcag gactctttga tgtaacccgt 1560

ggctgccatg agtcctgtct ggatgaagag gtagagggcc agggtttctg ctctggtcct 1620

ggctgggatc ctgtaacagg ctggggaaca cccaacttcc cagctttgct gaagactcta 1680

ctcaacccct ga 1692

<210> 52
<211> 563
<212> PRT
<213> Homo sapiens

<400> 52

<210>  53
<211>  1239
<212>  DNA
<213>  Homo sapiens

<400>  53
atgcagccct ccagccttct gccgctcgcc ctctgcctgc tggctgcacc cgcctccgcg      60

ctcgtcagga tcccgctgca caagttcacg tccatccgcc ggaccatgtc ggaggttggg     120

ggctctgtgg aggacctgat tgccaaaggc cccgtctcaa agtactccca ggcggtgcca     180

gccgtgaccg aggggcccat tcccgaggtg ctcaagaact acatggacgc ccagtactac     240

ggggagattg gcatcgggac gcccccccag tgcttcacag tcgtcttcga cacgggctcc     300

tccaacctgt gggtcccctc catccactgc aaactgctgg acatcgcttg ctggatccac     360

cacaagtaca acagcgacaa gtccagcacc tacgtgaaga atggtacctc gtttgacatc 420
cactatggct cgggcagcct ctccgggtac ctgagccagg acactgtgtc ggtgccctgc 480
cagtcagcgt cgtcagcctc tgccctgggc ggtgtcaaag tggagaggca ggtctttggg 540
gaggccacca agcagccagg catcaccttc atcgcagcca agttcgatgg catcctgggc 600
atggcctacc cccgcatctc cgtcaacaac gtgctgcccg tcttcgacaa cctgatgcag 660
cagaagctgg tggaccagaa catcttctcc ttctacctga gcagggaccc agatgcgcag 720
cctgggggtg agctgatgct gggtggcaca gactccaagt attacaaggg ttctctgtcc 780
tacctgaatg tcacccgcaa ggcctactgg caggtccacc tggaccaggt ggaggtggcc 840
agcgggctga ccctgtgcaa ggagggctgt gaggccattg tggacacagg cacttccctc 900
atggtgggcc cggtggatga ggtgcgcgag ctgcagaagg ccatcggggc cgtgccgctg 960
attcagggcg agtacatgat cccctgtgag aaggtgtcca ccctgcccgc gatcacactg 1020
aagctgggag gcaaaggcta caagctgtcc ccagaggact acacgctcaa ggtgtcgcag 1080
gccgggaaga ccctctgcct gagcggcttc atgggcatgg acatcccgcc acccagcggg 1140
ccactctgga tcctgggcga cgtcttcatc ggccgctact acactgtgtt tgaccgtgac 1200
aacaacaggg tgggcttcgc cgaggctgcc cgcctctag 1239

<210> 54
<211> 412
<212> PRT
<213> Homo sapiens

<400> 54

Met Gln Pro Ser Ser Leu Leu Pro Leu Ala Leu Cys Leu Leu Ala Ala
1               5                   10                  15

Pro Ala Ser Ala Leu Val Arg Ile Pro Leu His Lys Phe Thr Ser Ile
            20                  25                  30

Arg Arg Thr Met Ser Glu Val Gly Gly Ser Val Glu Asp Leu Ile Ala
        35                  40                  45

Lys Gly Pro Val Ser Lys Tyr Ser Gln Ala Val Pro Ala Val Thr Glu
    50                  55                  60

Gly Pro Ile Pro Glu Val Leu Lys Asn Tyr Met Asp Ala Gln Tyr Tyr
65                  70                  75                  80

Gly Glu Ile Gly Ile Gly Thr Pro Pro Gln Cys Phe Thr Val Val Phe
                85                  90                  95

Asp Thr Gly Ser Ser Asn Leu Trp Val Pro Ser Ile His Cys Lys Leu
            100                 105                 110

Leu Asp Ile Ala Cys Trp Ile His His Lys Tyr Asn Ser Asp Lys Ser

115                 120                 125

Ser Thr Tyr Val Lys Asn Gly Thr Ser Phe Asp Ile His Tyr Gly Ser
    130                 135                 140

Gly Ser Leu Ser Gly Tyr Leu Ser Gln Asp Thr Val Ser Val Pro Cys
145                 150                 155                 160

Gln Ser Ala Ser Ser Ala Ser Ala Leu Gly Gly Val Lys Val Glu Arg
                165                 170                 175

Gln Val Phe Gly Glu Ala Thr Lys Gln Pro Gly Ile Thr Phe Ile Ala
            180                 185                 190

Ala Lys Phe Asp Gly Ile Leu Gly Met Ala Tyr Pro Arg Ile Ser Val
        195                 200                 205

Asn Asn Val Leu Pro Val Phe Asp Asn Leu Met Gln Gln Lys Leu Val
    210                 215                 220

Asp Gln Asn Ile Phe Ser Phe Tyr Leu Ser Arg Asp Pro Asp Ala Gln
225                 230                 235                 240

Pro Gly Gly Glu Leu Met Leu Gly Gly Thr Asp Ser Lys Tyr Tyr Lys
                245                 250                 255

Gly Ser Leu Ser Tyr Leu Asn Val Thr Arg Lys Ala Tyr Trp Gln Val
            260                 265                 270

His Leu Asp Gln Val Glu Val Ala Ser Gly Leu Thr Leu Cys Lys Glu
        275                 280                 285

Gly Cys Glu Ala Ile Val Asp Thr Gly Thr Ser Leu Met Val Gly Pro
    290                 295                 300

Val Asp Glu Val Arg Glu Leu Gln Lys Ala Ile Gly Ala Val Pro Leu
305                 310                 315                 320

Ile Gln Gly Glu Tyr Met Ile Pro Cys Glu Lys Val Ser Thr Leu Pro
                325                 330                 335

Ala Ile Thr Leu Lys Leu Gly Gly Lys Gly Tyr Lys Leu Ser Pro Glu
            340                 345                 350

Asp Tyr Thr Leu Lys Val Ser Gln Ala Gly Lys Thr Leu Cys Leu Ser
        355                 360                 365

Gly Phe Met Gly Met Asp Ile Pro Pro Pro Ser Gly Pro Leu Trp Ile
    370                 375                 380

Leu Gly Asp Val Phe Ile Gly Arg Tyr Tyr Thr Val Phe Asp Arg Asp

385                 390                 395                 400

Asn Asn Arg Val Gly Phe Ala Glu Ala Ala Arg Leu
                405                 410

<210>  55
<211>  921
<212>  DNA
<213>  Homo sapiens

<400>  55
atggcgtcgc ccggctgcct gtggctcttg gctgtggctc tcctgccatg gacctgcgct      60

tctcgggcgc tgcagcatct ggacccgccg gcgccgctgc cgttggtgat ctggcatggg     120

atgggagaca gctgttgcaa tcccttaagc atgggtgcta ttaaaaaaat ggtggagaag     180

aaaatacctg gaatttacgt cttatcttta gagattggga agaccctgat ggaggacgtg     240

gagaacagct tcttcttgaa tgtcaattcc caagtaacaa cagtgtgtca ggcacttgct     300

aaggatccta aattgcagca aggctacaat gctatgggat tctcccaggg aggccaattt     360

ctgagggcag tggctcagag atgcccttca cctcccatga tcaatctgat ctcggttggg     420

ggacaacatc aaggtgtttt tggactccct cgatgcccag gagagagctc tcacatctgt     480

gacttcatcc gaaaaacact gaatgctggg gcgtactcca aagttgttca ggaacgcctc     540

gtgcaagccg aatactggca tgaccccata aaggaggatg tgtatcgcaa ccacagcatc     600

ttcttggcag atataaatca ggagcggggt atcaatgagt cctacaagaa aaacctgatg     660

gccctgaaga agtttgtgat ggtgaaattc ctcaatgatt ccattgtgga ccctgtagat     720

tcggagtggt ttggatttta cagaagtggc caagccaagg aaaccattcc cttacaggag     780

acctccctgt acacacagga ccgcctgggg ctaaaggaaa tggacaatgc aggacagcta     840

gtgtttctgg ctacagaagg ggaccatctt cagttgtctg aagaatggtt ttatgcccac     900

atcataccat tccttggatg a                                               921

<210>  56
<211>  306
<212>  PRT
<213>  Homo sapiens

<400>  56

<210>  57
<211>  1509
<212>  DNA

<213>  Homo sapiens

<400>  57
atgagctgcc ccgtgcccgc ctgctgcgcg ctgctgctag tcctggggct ctgccgggcg      60

cgtccccgga acgcactgct gctcctcgcg gatgacggag gctttgagag tggcgcgtac     120

aacaacagcg ccatcgccac cccgcacctg gacgccttgg cccgccgcag cctcctcttt     180

cgcaatgcct tcacctcggt cagcagctgc tctcccagcc gcgccagcct cctcactggc     240

ctgccccagc atcagaatgg gatgtacggg ctgcaccagg acgtgcacca cttcaactcc     300

ttcgacaagg tgcggagcct gccgctgctg ctcagccaag ctggtgtgcg cacaggcatc     360

atcgggaaga agcacgtggg gccggagacc gtgtacccgt ttgactttgc gtacacggag     420

gagaatggct ccgtcctcca ggtggggcgg aacatcacta gaattaagct gctcgtccgg     480

aaattcctgc agactcagga tgaccggcct ttcttcctct acgtcgcctt ccacgacccc     540

caccgctgtg ggcactccca gccccagtac ggaaccttct gtgagaagtt tggcaacgga     600

gagagcggca tgggtcgtat cccagactgg accccccagg cctacgaccc actggacgtg     660

ctggtgcctt acttcgtccc caacaccccg gcagcccgag ccgacctggc cgctcagtac     720

accaccgtag gccgcatgga ccaaggagtt ggactggtgc tccaggagct gcgtgacgcc     780

ggtgtcctga acgacacact ggtgatcttc acgtccgaca acgggatccc cttccccagc     840

ggcaggacca acctgtactg gccgggcact gctgaaccct tactggtgtc atccccggag     900

cacccaaaac gctggggcca agtcagcgag gcctacgtga gcctcctaga cctcacgccc     960

accatcttgg attggttctc gatcccgtac cccagctacg ccatctttgg ctcgaagacc    1020

atccacctca ctggccggtc cctcctgccg gcgctggagg ccgagcccct ctgggccacc    1080

gtctttggca gccagagcca ccacgaggtc accatgtcct accccatgcg ctccgtgcag    1140

caccggcact tccgcctcgt gcacaacctc aacttcaaga tgccctttcc catcgaccag    1200

gacttctacg tctcacccac cttccaggac ctcctgaacc gcactacagc tggtcagccc    1260

acgggctggt acaaggacct ccgtcattac tactaccggg cgcgctggga gctctacgac    1320

cggagccggg acccccacga gacccagaac ctggccaccg acccgcgctt tgctcagctt    1380

ctggagatgc ttcgggacca gctggccaag tggcagtggg agacccacga cccctgggtg    1440

tgcgcccccg acggcgtcct ggaggagaag ctctctcccc agtgccagcc cctccacaat    1500

gagctgtga                                                            1509

<210>  58
<211>  502
<212>  PRT
<213>  Homo sapiens

<400>  58

<210>  59
<211>  1962
<212>  DNA
<213>  Homo sapiens

<400>  59
atgcgtcccc tgcgcccccg cgccgcgctg ctggcgctcc tggcctcgct cctggccgcg      60

cccccggtgg ccccggccga ggccccgcac ctggtgcatg tggacgcggc ccgcgcgctg     120

tggcccctgc ggcgcttctg gaggagcaca ggcttctgcc ccccgctgcc acacagccag     180

gctgaccagt acgtcctcag ctgggaccag cagctcaacc tcgcctatgt gggcgccgtc     240

cctcaccgcg gcatcaagca ggtccggacc cactggctgc tggagcttgt caccaccagg 300
gggtccactg gacggggcct gagctacaac ttcacccacc tggacgggta cctggacctt 360
ctcagggaga accagctcct cccagggttt gagctgatgg gcagcgcctc gggccacttc 420
actgactttg aggacaagca gcaggtgttt gagtggaagg acttggtctc cagcctggcc 480
aggagataca tcggtaggta cggactggcg catgtttcca agtggaactt cgagacgtgg 540
aatgagccag accaccacga ctttgacaac gtctccatga ccatgcaagg cttcctgaac 600
tactacgatg cctgctcgga gggtctgcgc gccgccagcc ccgccctgcg gctgggaggc 660
cccggcgact ccttccacac cccaccgcga tccccgctga gctggggcct cctgcgccac 720
tgccacgacg gtaccaactt cttcactggg gaggcgggcg tgcggctgga ctacatctcc 780
ctccacagga agggtgcgcg cagctccatc tccatcctgg agcaggagaa ggtcgtcgcg 840
cagcagatcc ggcagctctt ccccaagttc gcggacaccc ccatttacaa cgacgaggcg 900
gacccgctgg tgggctggtc cctgccacag ccgtggaggg cggacgtgac ctacgcggcc 960
atggtggtga aggtcatcgc gcagcatcag aacctgctac tggccaacac cacctccgcc 1020
ttcccctacg cgctcctgag caacgacaat gccttcctga gctaccaccc gcaccccttc 1080
gcgcagcgca cgctcaccgc gcgcttccag gtcaacaaca cccgcccgcc gcacgtgcag 1140
ctgttgcgca agccggtgct cacggccatg gggctgctgg cgctgctgga tgaggagcag 1200
ctctgggccg aagtgtcgca ggccgggacc gtcctggaca gcaaccacac ggtgggcgtc 1260
ctggccagcg cccaccgccc ccagggcccg gccgacgcct ggcgcgccgc ggtgctgatc 1320
tacgcgagcg acgacacccg cgcccacccc aaccgcagcg tcgcggtgac cctgcggctg 1380
cgcggggtgc cccccggccc gggcctggtc tacgtcacgc gctacctgga caacgggctc 1440
tgcagccccg acggcgagtg gcggcgcctg ggccggcccg tcttccccac ggcagagcag 1500
ttccggcgca tgcgcgcggc tgaggacccg gtggccgcgg cgccccgccc cttacccgcc 1560
ggcggccgcc tgaccctgcg ccccgcgctg cggctgccgt cgcttttgct ggtgcacgtg 1620
tgtgcgcgcc ccgagaagcc gcccgggcag gtcacgcggc tccgcgccct gcccctgacc 1680
caagggcagc tggttctggt ctggtcggat gaacacgtgg gctccaagtg cctgtggaca 1740
tacgagatcc agttctctca ggacggtaag gcgtacaccc cggtcagcag gaagccatcg 1800
accttcaacc tctttgtgtt cagcccagac acaggtgctg tctctggctc ctaccgagtt 1860
cgagccctgg actactgggc ccgaccaggc cccttctcgg accctgtgcc gtacctggag 1920
gtccctgtgc caagagggcc cccatccccg ggcaatccat ga 1962

<210> 60
<211> 653
<212> PRT
<213> Homo sapiens

<400> 60

<210>  61
<211>  1653
<212>  DNA
<213>  Homo sapiens

<400>  61
atgccgccac cccggaccgg ccgaggcctt ctctggctgg gtctggttct gagctccgtc      60

tgcgtcgccc tcggatccga aacgcaggcc aactcgacca cagatgctct gaacgttctt     120

ctcatcatcg tggatgacct gcgcccctcc ctgggctgtt atggggataa gctggtgagg     180

tccccaaata ttgaccaact ggcatcccac agcctcctct tccagaatgc ctttgcgcag     240

caagcagtgt gcgccccgag ccgcgtttct ttcctcactg gcaggagacc tgacaccacc     300

cgcctgtacg acttcaactc ctactggagg gtgcacgctg gaaacttctc caccatcccc     360

cagtacttca aggagaatgg ctatgtgacc atgtcggtgg gaaaagtctt tcaccctggg     420

atatcttcta accataccga tgattctccg tatagctggt cttttccacc ttatcatcct     480

tcctctgaga agtatgaaaa cactaagaca tgtcgagggc cagatggaga actccatgcc     540

aacctgcttt gccctgtgga tgtgctggat gttcccgagg gcaccttgcc tgacaaacag     600

agcactgagc aagccataca gttgttggaa aagatgaaaa cgtcagccag tcctttcttc     660

ctggccgttg ggtatcataa gccacacatc cccttcagat accccaagga atttcagaag     720

ttgtatccct tggagaacat caccctggcc cccgatcccg aggtccctga tggcctaccc     780

cctgtggcct acaacccctg gatggacatc aggcaacggg aagacgtcca agccttaaac     840

atcagtgtgc cgtatggtcc aattcctgtg gactttcagc ggaaaatccg ccagagctac     900

tttgcctctg tgtcatattt ggatacacag gtcggccgcc tcttgagtgc tttggacgat     960

cttcagctgg ccaacagcac catcattgca tttacctcgg atcatgggtg ggctctaggt    1020

gaacatggag aatgggccaa atacagcaat tttgatgttg ctacccatgt tcccctgata    1080

ttctatgttc ctggaaggac ggcttcactt ccggaggcag gcgagaagct tttcccttac 1140

ctcgaccctt ttgattccgc ctcacagttg atggagccag gcaggcaatc catggacctt 1200

gtggaacttg tgtctctttt tcccacgctg gctggacttg caggactgca ggttccacct 1260

cgctgccccg ttccttcatt tcacgttgag ctgtgcagag aaggcaagaa ccttctgaag 1320

cattttcgat tccgtgactt ggaagaggat ccgtacctcc ctggtaatcc ccgtgaactg 1380

attgcctata gccagtatcc ccggccttca gacatccctc agtggaattc tgacaagccg 1440

agtttaaaag atataaagat catgggctat tccatacgca ccatagacta taggtatact 1500

gtgtgggttg gcttcaatcc tgatgaattt ctagctaact tttctgacat ccatgcaggg 1560

gaactgtatt ttgtggattc tgacccattg caggatcaca atatgtataa tgattcccaa 1620

ggtggagatc ttttccagtt gttgatgcct tga 1653

<210> 62
<211> 550
<212> PRT
<213> Homo sapiens

<400> 62

Met Pro Pro Pro Arg Thr Gly Arg Gly Leu Leu Trp Leu Gly Leu Val
1               5                   10                  15

Leu Ser Ser Val Cys Val Ala Leu Gly Ser Glu Thr Gln Ala Asn Ser
            20                  25                  30

Thr Thr Asp Ala Leu Asn Val Leu Leu Ile Ile Val Asp Asp Leu Arg
        35                  40                  45

Pro Ser Leu Gly Cys Tyr Gly Asp Lys Leu Val Arg Ser Pro Asn Ile
    50                  55                  60

Asp Gln Leu Ala Ser His Ser Leu Leu Phe Gln Asn Ala Phe Ala Gln
65                  70                  75                  80

Gln Ala Val Cys Ala Pro Ser Arg Val Ser Phe Leu Thr Gly Arg Arg
                85                  90                  95

Pro Asp Thr Thr Arg Leu Tyr Asp Phe Asn Ser Tyr Trp Arg Val His
            100                 105                 110

Ala Gly Asn Phe Ser Thr Ile Pro Gln Tyr Phe Lys Glu Asn Gly Tyr
        115                 120                 125

Val Thr Met Ser Val Gly Lys Val Phe His Pro Gly Ile Ser Ser Asn
    130                 135                 140

His Thr Asp Asp Ser Pro Tyr Ser Trp Ser Phe Pro Pro Tyr His Pro
145                 150                 155                 160

Ser Ser Glu Lys Tyr Glu Asn Thr Lys Thr Cys Arg Gly Pro Asp Gly
                165                 170                 175

Glu Leu His Ala Asn Leu Leu Cys Pro Val Asp Val Leu Asp Val Pro
            180                 185                 190

Glu Gly Thr Leu Pro Asp Lys Gln Ser Thr Glu Gln Ala Ile Gln Leu
        195                 200                 205

Leu Glu Lys Met Lys Thr Ser Ala Ser Pro Phe Phe Leu Ala Val Gly
    210                 215                 220

Tyr His Lys Pro His Ile Pro Phe Arg Tyr Pro Lys Glu Phe Gln Lys
225                 230                 235                 240

Leu Tyr Pro Leu Glu Asn Ile Thr Leu Ala Pro Asp Pro Glu Val Pro
                245                 250                 255

Asp Gly Leu Pro Pro Val Ala Tyr Asn Pro Trp Met Asp Ile Arg Gln
            260                 265                 270

Arg Glu Asp Val Gln Ala Leu Asn Ile Ser Val Pro Tyr Gly Pro Ile
        275                 280                 285

Pro Val Asp Phe Gln Arg Lys Ile Arg Gln Ser Tyr Phe Ala Ser Val
    290                 295                 300

Ser Tyr Leu Asp Thr Gln Val Gly Arg Leu Leu Ser Ala Leu Asp Asp
305                 310                 315                 320

Leu Gln Leu Ala Asn Ser Thr Ile Ile Ala Phe Thr Ser Asp His Gly
                325                 330                 335

Trp Ala Leu Gly Glu His Gly Glu Trp Ala Lys Tyr Ser Asn Phe Asp
            340                 345                 350

Val Ala Thr His Val Pro Leu Ile Phe Tyr Val Pro Gly Arg Thr Ala
        355                 360                 365

Ser Leu Pro Glu Ala Gly Glu Lys Leu Phe Pro Tyr Leu Asp Pro Phe
    370                 375                 380

Asp Ser Ala Ser Gln Leu Met Glu Pro Gly Arg Gln Ser Met Asp Leu
385                 390                 395                 400

Val Glu Leu Val Ser Leu Phe Pro Thr Leu Ala Gly Leu Ala Gly Leu
                405                 410                 415

Gln Val Pro Pro Arg Cys Pro Val Pro Ser Phe His Val Glu Leu Cys
            420                 425                 430

Arg Glu Gly Lys Asn Leu Leu Lys His Phe Arg Phe Arg Asp Leu Glu
        435                 440                 445

Glu Asp Pro Tyr Leu Pro Gly Asn Pro Arg Glu Leu Ile Ala Tyr Ser
    450                 455                 460

Gln Tyr Pro Arg Pro Ser Asp Ile Pro Gln Trp Asn Ser Asp Lys Pro
465                 470                 475                 480

Ser Leu Lys Asp Ile Lys Ile Met Gly Tyr Ser Ile Arg Thr Ile Asp
                485                 490                 495

Tyr Arg Tyr Thr Val Trp Val Gly Phe Asn Pro Asp Glu Phe Leu Ala
            500                 505                 510

Asn Phe Ser Asp Ile His Ala Gly Glu Leu Tyr Phe Val Asp Ser Asp
        515                 520                 525

Pro Leu Gln Asp His Asn Met Tyr Asn Asp Ser Gln Gly Gly Asp Leu
    530                 535                 540

Phe Gln Leu Leu Met Pro
545                 550

<210>  63
<211>  1524
<212>  DNA
<213>  Homo sapiens

<400>  63
atgggggcac cgcggtccct cctcctggcc ctggctgctg gcctggccgt tgcccgtccg      60

cccaacatcg tgctgatctt tgccgacgac ctcggctatg gggacctggg ctgctatggg     120

caccccagct ctaccactcc caacctggac cagctggcgg cgggagggct gcggttcaca     180

gacttctacg tgcctgtgtc tctgtgcaca ccctctaggg ccgccctcct gaccggccgg     240

ctcccggttc ggatgggcat gtaccctggc gtcctggtgc ccagctcccg ggggggcctg     300

cccctggagg aggtgaccgt ggccgaagtc ctggctgccc gaggctacct cacaggaatg     360

gccggcaagt ggcaccttgg ggtggggcct gagggggcct tcctgccccc ccatcagggc     420

ttccatcgat ttctaggcat cccgtactcc cacgaccagg gcccctgcca gaacctgacc     480

tgcttcccgc cggccactcc ttgcgacggt ggctgtgacc agggcctggt ccccatccca     540

ctgttggcca acctgtccgt ggaggcgcag cccccctggc tgcccggact agaggcccgc     600

tacatggctt tcgcccatga cctcatggcc gacgcccagc gccaggatcg ccccttcttc     660

ctgtactatg cctctcacca cacccactac cctcagttca gtgggcagag ctttgcagag     720

cgttcaggcc gcgggccatt tggggactcc ctgatggagc tggatgcagc tgtggggacc     780

ctgatgacag ccatagggga cctggggctg cttgaagaga cgctggtcat cttcactgca     840

gacaatggac ctgagaccat gcgtatgtcc cgaggcggct gctccggtct cttgcggtgt     900

ggaaagggaa cgacctacga gggcggtgtc cgagagcctg ccttggcctt ctggccaggt 960
catatcgctc ccggcgtgac ccacgagctg gccagctccc tggacctgct gcctaccctg 1020
gcagccctgg ctggggcccc actgcccaat gtcaccttgg atggctttga cctcagcccc 1080
ctgctgctgg gcacaggcaa gagccctcgg cagtctctct tcttctaccc gtcctaccca 1140
gacgaggtcc gtggggtttt tgctgtgcgg actggaaagt acaaggctca cttcttcacc 1200
cagggctctg cccacagtga taccactgca gaccctgcct gccacgcctc cagctctctg 1260
actgctcatg agcccccgct gctctatgac ctgtccaagg accctggtga gaactacaac 1320
ctgctggggg gtgtggccgg ggccacccca gaggtgctgc aagccctgaa acagcttcag 1380
ctgctcaagg cccagttaga cgcagctgtg accttcggcc ccagccaggt ggcccggggc 1440
gaggaccccg ccctgcagat ctgctgtcat cctggctgca ccccccgccc agcttgctgc 1500
cattgcccag atccccatgc ctga 1524

<210> 64
<211> 507
<212> PRT
<213> Homo sapiens

<400> 64

Met Gly Ala Pro Arg Ser Leu Leu Leu Ala Leu Ala Ala Gly Leu Ala
1               5                   10                  15

Val Ala Arg Pro Pro Asn Ile Val Leu Ile Phe Ala Asp Asp Leu Gly
            20                  25                  30

Tyr Gly Asp Leu Gly Cys Tyr Gly His Pro Ser Ser Thr Thr Pro Asn
        35                  40                  45

Leu Asp Gln Leu Ala Ala Gly Gly Leu Arg Phe Thr Asp Phe Tyr Val
    50                  55                  60

Pro Val Ser Leu Cys Thr Pro Ser Arg Ala Ala Leu Leu Thr Gly Arg
65                  70                  75                  80

Leu Pro Val Arg Met Gly Met Tyr Pro Gly Val Leu Val Pro Ser Ser
                85                  90                  95

Arg Gly Gly Leu Pro Leu Glu Glu Val Thr Val Ala Glu Val Leu Ala
            100                 105                 110

Ala Arg Gly Tyr Leu Thr Gly Met Ala Gly Lys Trp His Leu Gly Val
        115                 120                 125

Gly Pro Glu Gly Ala Phe Leu Pro Pro His Gln Gly Phe His Arg Phe
    130                 135                 140

Leu Gly Ile Pro Tyr Ser His Asp Gln Gly Pro Cys Gln Asn Leu Thr

145                 150                 155                 160

Cys Phe Pro Pro Ala Thr Pro Cys Asp Gly Gly Cys Asp Gln Gly Leu
                165                 170                 175

Val Pro Ile Pro Leu Leu Ala Asn Leu Ser Val Glu Ala Gln Pro Pro
            180                 185                 190

Trp Leu Pro Gly Leu Glu Ala Arg Tyr Met Ala Phe Ala His Asp Leu
        195                 200                 205

Met Ala Asp Ala Gln Arg Gln Asp Arg Pro Phe Phe Leu Tyr Tyr Ala
    210                 215                 220

Ser His His Thr His Tyr Pro Gln Phe Ser Gly Gln Ser Phe Ala Glu
225                 230                 235                 240

Arg Ser Gly Arg Gly Pro Phe Gly Asp Ser Leu Met Glu Leu Asp Ala
                245                 250                 255

Ala Val Gly Thr Leu Met Thr Ala Ile Gly Asp Leu Gly Leu Leu Glu
            260                 265                 270

Glu Thr Leu Val Ile Phe Thr Ala Asp Asn Gly Pro Glu Thr Met Arg
        275                 280                 285

Met Ser Arg Gly Gly Cys Ser Gly Leu Leu Arg Cys Gly Lys Gly Thr
    290                 295                 300

Thr Tyr Glu Gly Gly Val Arg Glu Pro Ala Leu Ala Phe Trp Pro Gly
305                 310                 315                 320

His Ile Ala Pro Gly Val Thr His Glu Leu Ala Ser Ser Leu Asp Leu
                325                 330                 335

Leu Pro Thr Leu Ala Ala Leu Ala Gly Ala Pro Leu Pro Asn Val Thr
            340                 345                 350

Leu Asp Gly Phe Asp Leu Ser Pro Leu Leu Leu Gly Thr Gly Lys Ser
        355                 360                 365

Pro Arg Gln Ser Leu Phe Phe Tyr Pro Ser Tyr Pro Asp Glu Val Arg
    370                 375                 380

Gly Val Phe Ala Val Arg Thr Gly Lys Tyr Lys Ala His Phe Phe Thr
385                 390                 395                 400

Gln Gly Ser Ala His Ser Asp Thr Thr Ala Asp Pro Ala Cys His Ala
                405                 410                 415

Ser Ser Ser Leu Thr Ala His Glu Pro Pro Leu Leu Tyr Asp Leu Ser

420                 425                 430

Lys Asp Pro Gly Glu Asn Tyr Asn Leu Leu Gly Gly Val Ala Gly Ala
        435                 440                 445

Thr Pro Glu Val Leu Gln Ala Leu Lys Gln Leu Gln Leu Leu Lys Ala
    450                 455                 460

Gln Leu Asp Ala Ala Val Thr Phe Gly Pro Ser Gln Val Ala Arg Gly
465                 470                 475                 480

Glu Asp Pro Ala Leu Gln Ile Cys Cys His Pro Gly Cys Thr Pro Arg
                485                 490                 495

Pro Ala Cys Cys His Cys Pro Asp Pro His Ala
            500                 505

<210>  65
<211>  2010
<212>  DNA
<213>  Homo sapiens

<400>  65
atggctgcag ccgcgggttc ggcgggccgc gccgcggtgc ccttgctgct gtgtgcgctg      60

ctggcgcccg gcggcgcgta cgtgctcgac gactccgacg ggctgggccg ggagttcgac     120

ggcatcggcg cggtcagcgg cggcggggca acctcccgac ttctagtaaa ttacccagag     180

ccctatcgtt ctcagatatt ggattatctc tttaagccga attttggtgc ctctttgcat     240

attttaaaag tggaaatagg tggtgatggg cagacaacag atggcactga gccctcccac     300

atgcattatg cactagatga gaattatttc cgaggatacg agtggtggtt gatgaaagaa     360

gctaagaaga ggaatcccaa tattacactc attgggttgc catggtcatt ccctggatgg     420

ctgggaaaag gtttcgactg gccttatgtc aatcttcagc tgactgccta ttatgtcgtg     480

acctggattg tgggcgccaa gcgttaccat gatttggaca ttgattatat tggaatttgg     540

aatgagaggt catataatgc caattatatt aagatattaa gaaaaatgct gaattatcaa     600

ggtctccagc gagtgaaaat catagcaagt gataatctct gggagtccat ctctgcatcc     660

atgctccttg atgccgaact cttcaaggtg gttgatgtta taggggctca ttatcctgga     720

acccattcag caaaagatgc aaagttgact gggaagaagc tttggtcttc tgaagacttt     780

agcactttaa atagtgacat gggtgcaggc tgctggggtc gcattttaaa tcagaattat     840

atcaatggct atatgacttc cacaatcgca tggaatttag tggctagtta ctatgaacag     900

ttgccttatg ggagatgcgg gttgatgacg gcccaggagc catggagtgg gcactacgtg     960

gtagaatctc ctgtctgggt atcagctcat accactcagt ttactcaacc tggctggtat    1020

tacctgaaga cagttggcca tttagagaaa ggaggaagct acgtagctct gactgatggc    1080

ttagggaacc tcaccatcat cattgaaacc atgagtcata aacattctaa gtgcatacgg    1140

ccatttcttc cttatttcaa tgtgtcacaa caatttgcca cctttgttct taagggatct    1200

tttagtgaaa taccagagct acaggtatgg tataccaaac ttggaaaaac atccgaaaga 1260

tttcttttta agcagctgga ttctctatgg ctccttgaca gcgatggcag tttcacactg 1320

agcctgcatg aagatgagct gttcacactc accactctca ccactggtcg caaaggcagc 1380

tacccgcttc ctccaaaatc ccagcccttc ccaagtacct ataaggatga tttcaatgtt 1440

gattacccat tttttagtga agctccaaac tttgctgatc aaactggtgt atttgaatat 1500

tttacaaata ttgaagaccc tggcgagcat cacttcacgc tacgccaagt tctcaaccag 1560

agacccatta cgtgggctgc cgatgcatcc aacacaatca gtattatagg agactacaac 1620

tggaccaatc tgactacaaa gtgtgatgtt tacatagaga cccctgacac aggaggtgtg 1680

ttcattgcag gaagagtaaa taaaggtggt attttgatta gaagtgccag aggaattttc 1740

ttctggattt ttgcaaatgg atcttacagg gttacaggtg atttagctgg atggattata 1800

tatgctttag gacgtgttga agttacagca aaaaaatggt atacactcac gttaactatt 1860

aagggtcatt tcgcctctgg catgctgaat gacaagtctc tgtggacaga catccctgtg 1920

aattttccaa agaatggctg ggctgcaatt ggaactcact cctttgaatt tgcacagttt 1980

gacaactttc ttgtggaagc cacacgctaa 2010

<210> 66
<211> 669
<212> PRT
<213> Homo sapiens

<400> 66

Met Ala Ala Ala Ala Gly Ser Ala Gly Arg Ala Ala Val Pro Leu Leu
1               5                   10                  15

Leu Cys Ala Leu Leu Ala Pro Gly Gly Ala Tyr Val Leu Asp Asp Ser
            20                  25                  30

Asp Gly Leu Gly Arg Glu Phe Asp Gly Ile Gly Ala Val Ser Gly Gly
        35                  40                  45

Gly Ala Thr Ser Arg Leu Leu Val Asn Tyr Pro Glu Pro Tyr Arg Ser
    50                  55                  60

Gln Ile Leu Asp Tyr Leu Phe Lys Pro Asn Phe Gly Ala Ser Leu His
65                  70                  75                  80

Ile Leu Lys Val Glu Ile Gly Gly Asp Gly Gln Thr Thr Asp Gly Thr
                85                  90                  95

Glu Pro Ser His Met His Tyr Ala Leu Asp Glu Asn Tyr Phe Arg Gly
            100                 105                 110

Tyr Glu Trp Trp Leu Met Lys Glu Ala Lys Lys Arg Asn Pro Asn Ile
        115                 120                 125

Thr Leu Ile Gly Leu Pro Trp Ser Phe Pro Gly Trp Leu Gly Lys Gly
    130                 135                 140

Phe Asp Trp Pro Tyr Val Asn Leu Gln Leu Thr Ala Tyr Tyr Val Val
145                 150                 155                 160

Thr Trp Ile Val Gly Ala Lys Arg Tyr His Asp Leu Asp Ile Asp Tyr
                165                 170                 175

Ile Gly Ile Trp Asn Glu Arg Ser Tyr Asn Ala Asn Tyr Ile Lys Ile
            180                 185                 190

Leu Arg Lys Met Leu Asn Tyr Gln Gly Leu Gln Arg Val Lys Ile Ile
        195                 200                 205

Ala Ser Asp Asn Leu Trp Glu Ser Ile Ser Ala Ser Met Leu Leu Asp
    210                 215                 220

Ala Glu Leu Phe Lys Val Val Asp Val Ile Gly Ala His Tyr Pro Gly
225                 230                 235                 240

Thr His Ser Ala Lys Asp Ala Lys Leu Thr Gly Lys Lys Leu Trp Ser
                245                 250                 255

Ser Glu Asp Phe Ser Thr Leu Asn Ser Asp Met Gly Ala Gly Cys Trp
            260                 265                 270

Gly Arg Ile Leu Asn Gln Asn Tyr Ile Asn Gly Tyr Met Thr Ser Thr
        275                 280                 285

Ile Ala Trp Asn Leu Val Ala Ser Tyr Tyr Glu Gln Leu Pro Tyr Gly
    290                 295                 300

Arg Cys Gly Leu Met Thr Ala Gln Glu Pro Trp Ser Gly His Tyr Val
305                 310                 315                 320

Val Glu Ser Pro Val Trp Val Ser Ala His Thr Thr Gln Phe Thr Gln
                325                 330                 335

Pro Gly Trp Tyr Tyr Leu Lys Thr Val Gly His Leu Glu Lys Gly Gly
            340                 345                 350

Ser Tyr Val Ala Leu Thr Asp Gly Leu Gly Asn Leu Thr Ile Ile Ile
        355                 360                 365

Glu Thr Met Ser His Lys His Ser Lys Cys Ile Arg Pro Phe Leu Pro
    370                 375                 380

Tyr Phe Asn Val Ser Gln Gln Phe Ala Thr Phe Val Leu Lys Gly Ser
385                 390                 395                 400

Phe Ser Glu Ile Pro Glu Leu Gln Val Trp Tyr Thr Lys Leu Gly Lys
                405                 410                 415

Thr Ser Glu Arg Phe Leu Phe Lys Gln Leu Asp Ser Leu Trp Leu Leu
            420                 425                 430

Asp Ser Asp Gly Ser Phe Thr Leu Ser Leu His Glu Asp Glu Leu Phe
        435                 440                 445

Thr Leu Thr Thr Leu Thr Thr Gly Arg Lys Gly Ser Tyr Pro Leu Pro
    450                 455                 460

Pro Lys Ser Gln Pro Phe Pro Ser Thr Tyr Lys Asp Asp Phe Asn Val
465                 470                 475                 480

Asp Tyr Pro Phe Phe Ser Glu Ala Pro Asn Phe Ala Asp Gln Thr Gly
                485                 490                 495

Val Phe Glu Tyr Phe Thr Asn Ile Glu Asp Pro Gly Glu His His Phe
            500                 505                 510

Thr Leu Arg Gln Val Leu Asn Gln Arg Pro Ile Thr Trp Ala Ala Asp
        515                 520                 525

Ala Ser Asn Thr Ile Ser Ile Ile Gly Asp Tyr Asn Trp Thr Asn Leu
    530                 535                 540

Thr Thr Lys Cys Asp Val Tyr Ile Glu Thr Pro Asp Thr Gly Gly Val
545                 550                 555                 560

Phe Ile Ala Gly Arg Val Asn Lys Gly Gly Ile Leu Ile Arg Ser Ala
                565                 570                 575

Arg Gly Ile Phe Phe Trp Ile Phe Ala Asn Gly Ser Tyr Arg Val Thr
            580                 585                 590

Gly Asp Leu Ala Gly Trp Ile Ile Tyr Ala Leu Gly Arg Val Glu Val
        595                 600                 605

Thr Ala Lys Lys Trp Tyr Thr Leu Thr Leu Thr Ile Lys Gly His Phe
    610                 615                 620

Ala Ser Gly Met Leu Asn Asp Lys Ser Leu Trp Thr Asp Ile Pro Val
625                 630                 635                 640

Asn Phe Pro Lys Asn Gly Trp Ala Ala Ile Gly Thr His Ser Phe Glu
                645                 650                 655

Phe Ala Gln Phe Asp Asn Phe Leu Val Glu Ala Thr Arg
            660                 665

<210>  67
<211>  1611
<212>  DNA
<213>  Homo sapiens

<400>  67
atggagtttt caagtccttc cagagaggaa tgtcccaagc ctttgagtag ggtaagcatc      60

atggctggca gcctcacagg attgcttcta cttcaggcag tgtcgtgggc atcaggtgcc     120

cgcccctgca tccctaaaag cttcggctac agctcggtgg tgtgtgtctg caatgccaca     180

tactgtgact cctttgaccc cccgaccttt cctgcccttg gtaccttcag ccgctatgag     240

agtacacgca gtgggcgacg gatggagctg agtatggggc ccatccaggc taatcacacg     300

ggcacaggcc tgctactgac cctgcagcca gaacagaagt tccagaaagt gaagggattt     360

ggaggggcca tgacagatgc tgctgctctc aacatccttg ccctgtcacc ccctgcccaa     420

aatttgctac ttaaatcgta cttctctgaa gaaggaatcg gatataacat catccgggta     480

cccatggcca gcagcgactt ctccatccgc acctacacct atgcagacac ccctgatgat     540

ttccagttgc acaacttcag cctcccagag gaagatacca agctcaagat acccctgatt     600

caccgagccc tgcagttggc ccagcgtccc gtttcactcc ttgccagccc ctggacatca     660

cccacttggc tcaagaccaa tggagcggtg aatgggaagg ggtcactcaa gggacagccc     720

ggagacatct accaccagac ctgggccaga tactttgtga agttcctgga tgcctatgct     780

gagcacaagt tacagttctg ggcagtgaca gctgaaaatg agccttctgc tgggctgttg     840

agtggatacc ccttccagag cctgggcttc acccctgaac atcagcgaga cttcattgcc     900

cgtgacctag gtcctaccct cgccaacagt actcaccaca atgtccgcct actcatgctg     960

gatgaccaac gcttgctgct gccccactgg gcaaaggtgg tactgacaga cccagaagca    1020

gctaaatatg ttcatggcat tgctgtacat tggtacctgg actttctggc tccagccaaa    1080

gccaccctag gggagacaca ccgcctgttc cccaacacca tgctctttgc ctcagaggcc    1140

agcgtgggct ccaagttctg ggagcagagt gtgcggctag gctcctggga tcgagggatg    1200

cagtacagcc acagcatcat cacgaacctc ctgtaccatg tggtcggctg gaccgactgg    1260

aaccttgccc tgaaccccga aggaggaccc aattgggtgc gtaactttgt cgacagtccc    1320

atcattgtag acatcaccaa ggacacgttt tacaaacagc ccatgttcta ccaccttggc    1380

cacttcagca agttcattcc tgagggctcc cagagagtgg ggctggttgc cagtcagaag    1440

aacgacctgg acgcagtggc actgatgcat cccgatggct ctgctgttgt ggtcgtgcta    1500

aaccgctcct ctaaggatgt gcctcttacc atcaaggatc ctgctgtggg cttcctggag    1560

acaatctcac ctggctactc cattcacacc tacctgtggc gtcgccagtg a             1611

<210>  68
<211>  538
<212>  PRT
<213>  Homo sapiens

<400>  68

Ala Ser Met Glu Phe Ser Ser Pro Ser Arg Glu Glu Cys Pro Lys Pro
1               5                   10                  15

Leu Ser Arg Val Ser Ile Met Ala Gly Ser Leu Thr Gly Leu Leu Leu
            20                  25                  30

Leu Gln Ala Val Ser Trp Ala Ser Gly Ala Arg Pro Cys Ile Pro Lys
        35                  40                  45

Ser Phe Gly Tyr Ser Ser Val Val Cys Val Cys Asn Ala Thr Tyr Cys
    50                  55                  60

Asp Ser Phe Asp Pro Pro Thr Phe Pro Ala Leu Gly Thr Phe Ser Arg
65                  70                  75                  80

Tyr Glu Ser Thr Arg Ser Gly Arg Arg Met Glu Leu Ser Met Gly Pro
                85                  90                  95

Ile Gln Ala Asn His Thr Gly Thr Gly Leu Leu Leu Thr Leu Gln Pro
            100                 105                 110

Glu Gln Lys Phe Gln Lys Val Lys Gly Phe Gly Gly Ala Met Thr Asp
        115                 120                 125

Ala Ala Ala Leu Asn Ile Leu Ala Leu Ser Pro Pro Ala Gln Asn Leu
    130                 135                 140

Leu Leu Lys Ser Tyr Phe Ser Glu Glu Gly Ile Gly Tyr Asn Ile Ile
145                 150                 155                 160

Arg Val Pro Met Ala Ser Ser Asp Phe Ser Ile Arg Thr Tyr Thr Tyr
                165                 170                 175

Ala Asp Thr Pro Asp Asp Phe Gln Leu His Asn Phe Ser Leu Pro Glu
            180                 185                 190

Glu Asp Thr Lys Leu Lys Ile Pro Leu Ile His Arg Ala Leu Gln Leu
        195                 200                 205

Ala Gln Arg Pro Val Ser Leu Leu Ala Ser Pro Trp Thr Ser Pro Thr
    210                 215                 220

Trp Leu Lys Thr Asn Gly Ala Val Asn Gly Lys Gly Ser Leu Lys Gly
225                 230                 235                 240

Gln Pro Gly Asp Ile Tyr His Gln Thr Trp Ala Arg Tyr Phe Val Lys
                245                 250                 255

Phe Leu Asp Ala Tyr Ala Glu His Lys Leu Gln Phe Trp Ala Val Thr
            260                 265                 270

Ala Glu Asn Glu Pro Ser Ala Gly Leu Leu Ser Gly Tyr Pro Phe Gln
        275                 280                 285

Ser Leu Gly Phe Thr Pro Glu His Gln Arg Asp Phe Ile Ala Arg Asp
    290                 295                 300

Leu Gly Pro Thr Leu Ala Asn Ser Thr His His Asn Val Arg Leu Leu
305                 310                 315                 320

Met Leu Asp Asp Gln Arg Leu Leu Leu Pro His Trp Ala Lys Val Val
                325                 330                 335

Leu Thr Asp Pro Glu Ala Ala Lys Tyr Val His Gly Ile Ala Val His
            340                 345                 350

Trp Tyr Leu Asp Phe Leu Ala Pro Ala Lys Ala Thr Leu Gly Glu Thr
        355                 360                 365

His Arg Leu Phe Pro Asn Thr Met Leu Phe Ala Ser Glu Ala Ser Val
    370                 375                 380

Gly Ser Lys Phe Trp Glu Gln Ser Val Arg Leu Gly Ser Trp Asp Arg
385                 390                 395                 400

Gly Met Gln Tyr Ser His Ser Ile Ile Thr Asn Leu Leu Tyr His Val
                405                 410                 415

Val Gly Trp Thr Asp Trp Asn Leu Ala Leu Asn Pro Glu Gly Gly Pro
            420                 425                 430

Asn Trp Val Arg Asn Phe Val Asp Ser Pro Ile Ile Val Asp Ile Thr
        435                 440                 445

Lys Asp Thr Phe Tyr Lys Gln Pro Met Phe Tyr His Leu Gly His Phe
    450                 455                 460

Ser Lys Phe Ile Pro Glu Gly Ser Gln Arg Val Gly Leu Val Ala Ser
465                 470                 475                 480

Gln Lys Asn Asp Leu Asp Ala Val Ala Leu Met His Pro Asp Gly Ser
                485                 490                 495

Ala Val Val Val Val Leu Asn Arg Ser Ser Lys Asp Val Pro Leu Thr
            500                 505                 510

Ile Lys Asp Pro Ala Val Gly Phe Leu Glu Thr Ile Ser Pro Gly Tyr
        515                 520                 525

Ser Ile His Thr Tyr Leu Trp Arg Arg Gln
    530                 535

<210> 69
<211> 1320
<212> DNA
<213> Homo sapiens

<400> 69
atgcagctga ggaacccaga actacatctg ggctgcgcgc ttgcgcttcg cttcctggcc 60

ctcgtttcct gggacatccc tggggctaga gcactggaca atggattggc aaggacgcct 120

accatgggct ggctgcactg ggagcgcttc atgtgcaacc ttgactgcca ggaagagcca 180

gattcctgca tcagtgagaa gctcttcatg gagatggcag agctcatggt ctcagaaggc 240

tggaaggatg caggttatga gtacctctgc attgatgact gttggatggc tccccaaaga 300

gattcagaag gcagacttca ggcagaccct cagcgctttc ctcatgggat tcgccagcta 360

gctaattatg ttcacagcaa aggactgaag ctagggattt atgcagatgt tgggaataaa 420

acctgcgcag gcttccctgg gagttttgga tactacgaca ttgatgccca gacctttgct 480

gactggggag tagatctgct aaaatttgat ggttgttact gtgacagttt ggaaaatttg 540

gcagatggtt ataagcacat gtccttggcc ctgaatagga ctggcagaag cattgtgtac 600

tcctgtgagt ggcctcttta tatgtggccc tttcaaaagc ccaattatac agaaatccga 660

cagtactgca atcactggcg aaattttgct gacattgatg attcctggaa aagtataaag 720

agtatcttgg actggacatc ttttaaccag gagagaattg ttgatgttgc tggaccaggg 780

ggttggaatg acccagatat gttagtgatt ggcaactttg gcctcagctg gaatcagcaa 840

gtaactcaga tggccctctg ggctatcatg gctgctcctt tattcatgtc taatgacctc 900

cgacacatca gccctcaagc caaagctctc cttcaggata aggacgtaat tgccatcaat 960

caggacccct tgggcaagca agggtaccag cttagacagg gagacaactt tgaagtgtgg 1020

gaacgacctc tctcaggctt agcctgggct gtagctatga taaaccggca ggagattggt 1080

ggacctcgct cttataccat cgcagttgct tccctgggta aaggagtggc ctgtaatcct 1140

gcctgcttca tcacacagct cctccctgtg aaaaggaagc tagggttcta tgaatggact 1200

tcaaggttaa gaagtcacat aaatcccaca ggcactgttt tgcttcagct agaaaataca 1260

atgcagatgt cattaaaaga cttactttaa atgcagatgt cattaaaaga cttactttaa 1320

<210> 70
<211> 429
<212> PRT
<213> Homo sapiens

<400> 70

<210>  71
<211>  92
<212>  PRT
<213>  Homo sapiens

<400>  71

Met Gln Pro Ser Ser Leu Leu Pro Leu Ala Leu Cys Leu Leu Ala Ala
1               5                   10                  15

Pro Ala Gly Ser Ser Lys Pro Gln Ala Leu Ala Thr Pro Asn Lys Glu
            20                  25                  30

Glu His Gly Lys Arg Lys Lys Lys Gly Lys Gly Leu Gly Lys Lys Arg
        35                  40                  45

Asp Pro Cys Leu Arg Lys Tyr Lys Asp Phe Cys Ile His Gly Glu Cys
    50                  55                  60

Lys Tyr Val Lys Glu Leu Arg Ala Pro Ser Cys Ile Cys His Pro Gly
65                  70                  75                  80

Tyr His Gly Glu Arg Cys His Gly Leu Ser Gly Ser
                85                  90

<210>  72
<211>  47
<212>  PRT
<213>  Homo sapiens

<400>  72

Met Gln Pro Ser Ser Leu Leu Pro Leu Ala Leu Cys Leu Leu Ala Ala
1               5                   10                  15

Pro Ala Gly Ser Gly Lys Arg Lys Lys Lys Gly Lys Gly Leu Gly Lys
            20                  25                  30

Lys Arg Asp Pro Ser Leu Arg Lys Tyr Lys Asp Phe Ser Gly Ser
        35                  40                  45

## Description

### FIELD OF THE INVENTION

This invention is in the field of biology and medicine in particular human therapeutics, more in particular in the field of lysosomal storage diseases (LSDs) which are a group of approximately 40 rare inherited metabolic disorders that result from defects in lysosomal function. Lysosomal storage diseases result when a specific component of lysosomes which are organelles in the body's cells malfunctions.

### BACKGROUND OF THE INVENTION

Lysosomal storage diseases (LSDs) are a group of approximately 40 rare inherited metabolic disorders that result from defects in lysosomal function.
Tay-Sachs disease was the first of these disorders to be described, in 1881, followed by Gaucher disease in 1882 and Fabry disease in 1898. In the late 1950s and early 1960s, de Duve and colleagues, using cell fractionation techniques, cytological studies and biochemical analyses, identified and characterized the lysosome as a cellular organelle responsible for intracellular digestion and recycling of macromolecules. Pompe disease was the first disease to be identified as a LSD in 1963 (α-glucosidase deficiency).

Lysosomal storage disorders are caused by lysosomal dysfunction usually as a consequence of deficiency of a single enzyme required for the metabolism of lipids, proteins or carbohydrates. Worldwilde, individual LSDs occur with incidences of less than 1:100.000, however, as a group the incidence is about 1:5000 - 1:10.000.
Lysosomal disorders are caused by partial or complete loss of function of lysosomal proteins, mostly lysosomal enzymes. When this happens, substances accumulate in the cell. In other words, when the lysosome doesn't function normally, excess products destined for breakdown and recycling are stored in the cell.
Lysosomal storage diseases affect mostly children and they often die at a young and unpredictable age, many within a few months or years of birth. Many other children die of this disease following years of suffering from various symptoms of their particular disorder. The symptoms of lysosomal storage disease vary, depending on the particular disorder and other environmental and genetic factors. Usually, early onset forms are associated with a severe phenotype whereas late onset forms show a milder phenotype. Typical symptoms can include developmental delay, movement disorders, seizures, dementia, deafness and/or blindness. Some people with lysosomal storage disease have enlarged livers (hepatomegaly) and enlarged spleens (splenomegaly), pulmonary and cardiac problems, and bones that grow abnormally.

The lysosomal storage diseases are generally classified by the nature of the primary stored material involved, and can be broadly broken into the following: (ICD-10 codes are provided), (i) (E75) lipid storage disorders, mainly sphingolipidoses (including Gaucher's and Niemann-Pick diseases), (ii) (E75.0-E75.1) gangliosidosis (including Tay-Sachs disease), (iii) (E75.2) leukodystrophies, (iv) (E76.0) mucopolysaccharidoses (including Hunter syndrome and Hurler disease), (v) (E77) glycoprotein storage disorders and (vi) (E77.0-E77.1) mucolipidoses.

Alternatively to the protein targets, lysosomal storage diseases may be classified by the type of protein that is deficient and is causing build-up.

| **Type of defect protein** | **Disease examples** | **Deficient protein** |
|---|---|---|
| lysosomal hydrolases primarily | Sphingolipidoses (e.g., gangliosidosis, Gaucher and Niemann-Pick disease) | Various |
| Posttranslational modification of enzymes | Multiple sulfatase deficiency | Multiple sulfatases |
| Membrane transport proteins | Mucolipidosis type II and IIIA | N-acetylglucosamine-1-phosphate transferase |
| Enzyme protecting proteins | Galactosialidosis | Cathepsin A |
| Soluble nonenzymatic proteins | GM2-AP deficiency, variant AB | GM2-AP |
| Transmembrane proteins | SAP deficiency | Sphingolipid activator proteins |
| | Niemann-Pick disease, type C | NPC1 and NPC2 |
| | Salla disease | Sialin |

There are no cures for lysosomal storage diseases and treatment is mostly symptomatic, although bone marrow transplantation and enzyme replacement therapy (ERT) have been tried with some success (Clarke JT, Iwanochko RM (2005) "Enzyme replacement therapy of Fabry disease". Mol. Neurobiol. 32 (1): 43-50 and Bruni S, Loschi L, Incerti C, Gabrielli O, Coppa GV (2007) "Update on treatment of lysosomal storage diseases", Acta Myol 26 (1): 87-92). In addition, umbilical cord blood transplantation is being performed at specialized centres for a number of these diseases. In addition, substrate reduction therapy, a method used to decrease the accumulation of storage material, is currently being evaluated for some of these diseases. Also enzyme replacement therapy is being attempted however; success rates are low because the enzymes are poorly internalized in particular by neurons. Hence, there is a great need for a therapy for treating individuals that have a lysosomal storage disease. Such an approach would need to overcome in particular the problem of poor internalization as it was recently shown for example that in rat brain the turnover of mannose-6-phosphate is much lower in the central nervous system than in other tissues.

### SUMMARY OF THE INVENTION

The inventors of the present invention have astonishingly found that certain chimeric molecules can solve the above mentioned problem. The present invention therefore, relates to a chimeric molecule, comprising (i) a targeting moiety that binds to heparin or heparan sulfate proteoglycans, (ii) a lysosomal peptide or protein and (iii) wherein the targeting moiety is a neurotrophic growth factor and/or, wherein the targeting moiety comprises one of the following consensus sequences BBXB, BXBB, BBXXB, BXXBB, BBXXXB or BXXXBB and wherein B represents an arginine, lysine or histidine amino acid and X represents any amino acid, with the proviso that the targeting moiety is at least thirteen amino acids long.

The invention also relates to a polynucleotide encoding the chimeric molecule according to the invention as well as a pharmaceutical composition comprising a chimeric molecule according to the invention. The chimeric molecule according to the invention is also claimed for the use in the treatment of a disease. In one aspect of the invention the disease is a lysosomal storage disease.

Herein, a "chimeric molecule" is a molecule (preferably a biopolymer) containing molecule portions derived from two different origins, in a preferred embodiment, e.g. from two different genes.

Herein, a "mutant" sequence is defined as DNA, RNA or amino acid sequence differing from but having sequence identity with the native or disclosed sequence. Depending on the particular sequence, the degree of sequence identity between the native or disclosed sequence and the mutant sequence is preferably greater than 50% (e.g. 60%, 70%, 80%, 90%, 95%, 99% or more, calculated using the Smith-Waterman algorithm as described above). As used herein, an "allelic variant" of a nucleic acid molecule, or region, for which nucleic acid sequence is provided herein is a nucleic acid molecule, or region, that occurs essentially at the same locus in the genome of another or second isolate, and that, due to natural variation caused by, for example, mutation or recombination, has a similar but not identical nucleic acid sequence. A coding region allelic variant typically encodes a protein having similar activity to that of the protein encoded by the gene to which it is being compared. An allelic variant can also comprise an alteration in the 5' or 3' untranslated regions of the gene, such as in regulatory control regions (e.g. see US patent 5,753,235).

### FIGURE CAPTIONS

**Fig. 1**
   Purification of the TPP1-FGF2 fusion protein: Coomassie-stained PAGE gel with the 86 kDa TPP1 fusion protein in lane 2 after cation exchange chromatography.
**Fig. 2**
   Autocatalytic processing of the TPP1-FGF2 fusion protein: A Coomassie-stained PAGE gel demonstrating the pH-dependent auto-processing of the 86 kDa TPP1-FGF2 fusion protein after 10 min (10') and 90 min (90'), respectively. B Activity of the TPP1-FGF2 fusion protein during auto-processing.
**Fig. 3**

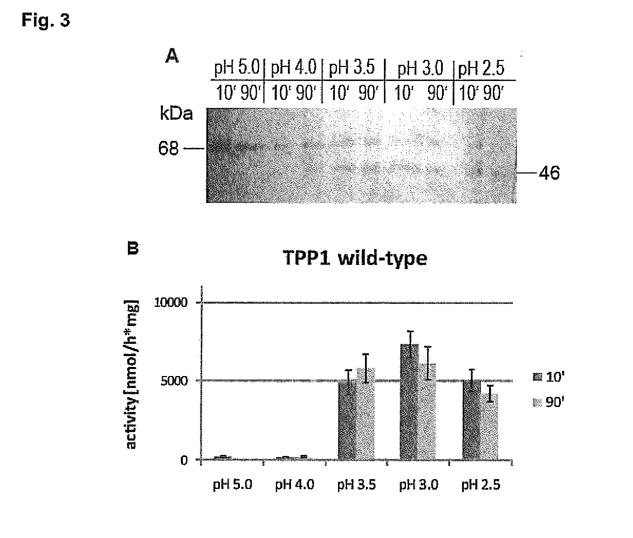

   Fig. 3 illustrates the respective auto-processing of the TPP1 wild-type. Interestingly, the TPP1-FGF2 fusion proteins showed a three times higher enzymatic activity than the processed TPP1 wild-type. Since after 10 min of incubation the N-terminal part of TPP1 is preferably cleaved off while the C-terminal part comprising the FGF2 tag is unaffected, it is concluded that the FGF2 tag improves the TPP1 activity. After 90 minutes incubation at room temperature the FGF2 tag is largely cleaved off and the activity is comparable to that of the TPP1 wild-type.
   The TPP1-FGF2 fusion protein is significantly more active at pH of 4.0 after a 10 minute (10 times higher) or a 90 minute incubation (5 times higher), respectively, than the TPP1 wild-type. This implies that the FGF2-Tag increases the TPP1 auto-processing at natural lysosomal pH environment (pH 4-5). The *in vitro* auto-activation at pH 3.5 is not physiological and does not represent the *in vivo* conditions. *In vivo,* other interacting compounds such as glycosaminoglycans may increase auto-processing at higher pH (pH 4-5).
**Fig. 4**
   Cellular uptake and intracellular activation of the TPP1-FGF2 fusion protein **(A)** and the TPP1 wild-type (TPP1-WT) protein **(B),** respectively. After 48 h of incubation with 0.4 to 0.5 µM TPP1-FGF2 fusion protein or TPP1 wild-type protein, respectively, the activity in the cell lysates of human NT2 cells was determined. TPP1-FGF2 fusion protein treated cells had a six times higher activity than the TPP1 wild-type treated NT2 cells. By adding 1 mM heparin (H) the cellular uptake was reduced to less than 30 %, whereas the addition of mannose-6-phosphate (MP) led to a 50% reduction of the uptake of the TPP1-FGF2 fusion protein. The combined addition of H and MP (HMP) led to a reduction to 16% of the cellular uptake/activity of the TPP1-FGF2 fusion protein. For the TPP1 wild-type protein, the highest reduction was observed for MP alone.
**Fig. 5**
   Survival times of *tpp1*-/- mice under intraventricular injections of either 10 µg TPP1 wild-type or TPP1-FGF2 fusion protein once per week, respectively. Injections were performed from the 30th day of life of the mice.
**Fig. 6**

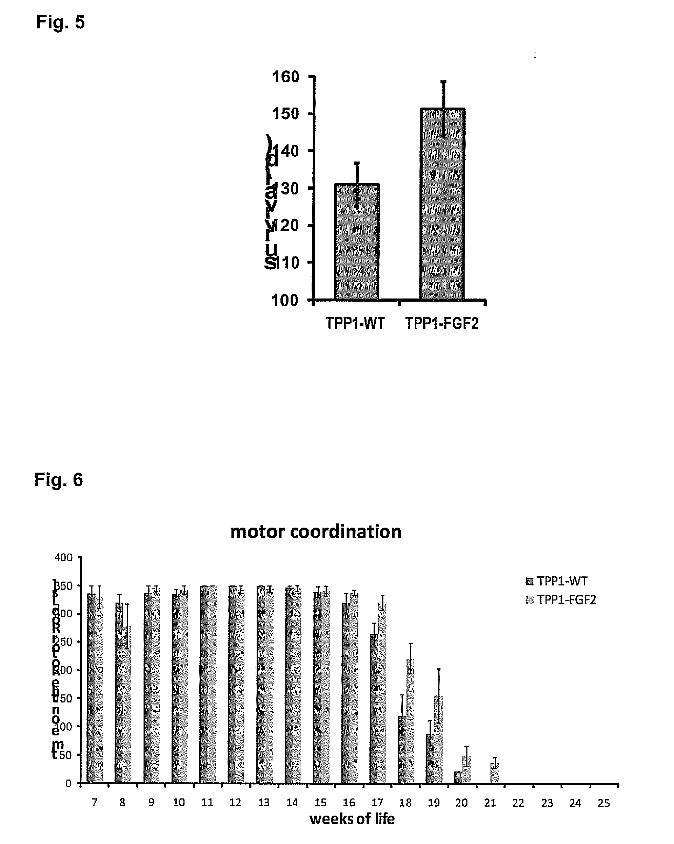

   Fig. 6 shows testing of the motor coordination of TPP1 wild-type (TPP1-WT) and TPP1-FGF2 fusion protein treated *tpp1*-/- mice. The time the mice spent on the Rotor Rod before falling down is plotted.

### DETAILED DESCRIPTION OF THE INVENTION

The inventors of the present invention have astonishingly found that certain chimeric molecules can solve the above mentioned problem. The present invention therefore, relates to a chimeric molecule, comprising (i) a targeting moiety that binds to heparin or heparan sulfate proteoglycans, (ii) a lysosomal peptide or protein and (iii) wherein the targeting moiety is a neurotrophic growth factor and/or, wherein the targeting moiety comprises one of the following consensus sequences BBXB, BXBB, BBXXB, BXXBB, BBXXXB or BXXXBB and wherein B represents an arginine, lysine or histidine amino acid and X represents any amino acid, with the proviso that the targeting moiety is at least thirteen amino acids long.

Preferably, the targeting moiety contains at least 7 basic amino acids selected from arginine, lysine and histidine.

It was astonishingly found that chimeric polypeptides according to the invention, such as TPP1-FGF1 fusion proteins showed a significantly higher life expectancy in mice (tpp1-/- mice ) as compared to mice treated with the TPP1 wild-type protein.

Moreover, *tpp1*-/- mice treated with TPP1-FGF2-fusion proteins showed a delayed course of illness in comparison to *tpp1*-/- mice treated with the TPP1 wild-type. Also motor coordination with the so called Rotor Rod was greatly improved in mice treated with the TPP1-FGF2 fusion protein.

In a preferred embodiment the chimeric molecule of the invention comprises a targeting moiety selected from the group of
(i) annexin II comprising the amino acid sequence according to SEQ ID NO. 1 (KIRSEFKKKYGKSLYY),
(ii) vitronectin comprising the amino acid sequence according to SEQ ID NO. 2 (QRFRHRNRKGYRSQRG),
(iii) ApoB comprising the amino acid sequence according to SEQ ID NO. 3 (KFIIPSPKRPVKLLSG),
(iv) bFGF comprising the amino acid sequence according to SEQ ID NO. 4 (GHFKDPKRLYCKNGGF),
(v) NCAM comprising the amino acid sequence according to SEQ ID NO. 5 (DGGSPIRHYLIKYKAK),
(vi) Protein C inhibitor comprising the amino acid sequence according to SEQ ID NO. 6 (GLSEKTLRKWLKMFKK),
(vii) AT-III comprising the amino acid sequence according to SEQ ID NO. 7 (KLNCRLYRKANKSSKL),
(viii) ApoE comprising the amino acid sequence according to SEQ ID NO. 8 (SHLRKLRKRLLRDADD),
(ix) Fibrin comprising the amino acid sequence according to SEQ ID NO. 9 (GHRPLDKKREEAPSLR),
(x) hGDNF comprising the amino acid sequence according to SEQ ID NO. 10 (SRGKGRRGQRGKNRG),
(xi) B-thromboglobulin comprising the amino acid sequence according to SEQ ID NO. 11 (PDAPRIKKIVQKKLAG)
(xii) Insulin-like growth factor-binding protein-3 comprising the amino acid sequence according to SEQ ID NO. 12 (DKKGFYKKKQCRPSKG),
(xiii) Antp comprising the amino acid sequence according to SEQ ID NO. 13 (RQIKIWFQNRRMKWKK) and
(xiv) human clock comprising the amino acid sequence according to SEQ ID NO. 14 (KRVSRNKSEKKRR).

In one embodiment the growth factor is modified and and lysosomal targeting is improved.

In a preferred embodiment the chimeric molecule of the invention is a molecule wherein the targeting moiety and the enzyme moiety are covalently linked to each other.

Ideally, the chimeric molecule is a single polypeptide chain.

Expression systems for such peptide chains are for example those used with mammalian cells, baculoviruses, and plants.

### Mammalian Systems

Mammalian expression systems are known in the art. A mammalian promoter is any DNA sequence capable of binding mammalian RNA polymerase and initiating the downstream (3') transcription of a coding sequence (e.g. structural gene) into mRNA. A promoter will have a transcription initiating region, which is usually placed proximal to the 5' end of the coding sequence, and a TATA box, usually located 25-30 base pairs (bp) upstream of the transcription initiation site. The TATA box is thought to direct RNA polymerase II to begin RNA synthesis at the correct site. A mammalian promoter will also contain an upstream promoter element, usually located within 100 to 200 bp upstream of the TATA box. An upstream promoter element determines the rate at which transcription is initiated and can act in either orientation (Sambrook et al. (1989) "Expression of Cloned Genes in Mammalian Cells." In Molecular Cloning: A Laboratory Manual, 2nd ed.).

Mammalian viral genes are often highly expressed and have a broad host range; therefore sequences encoding mammalian viral genes provide particularly useful promoter sequences. Examples include the SV40 early promoter, mouse mammary tumor virus LTR promoter, adenovirus major late promoter (Ad MLP), and herpes simplex virus promoter. In addition, sequences derived from non-viral genes, such as the murine metallotheionein gene, also provide useful promoter sequences. Expression may be either constitutive or regulated (inducible), depending on the promoter can be induced with glucocorticoid in hormone-responsive cells. The presence of an enhancer element (enhancer), combined with the promoter elements described above, will usually increase expression levels. An enhancer is a regulatory DNA sequence that can stimulate transcription up to 1000-fold when linked to homologous or heterologous promoters, with synthesis beginning at the normal RNA start site. Enhancers are also active when they are placed upstream or downstream from the transcription initiation site, in either normal or flipped orientation, or at a distance of more than 1000 nucleotides from the promoter (Maniatis et al. (1987) Science 236: 1237; Alberts et al. (1989) Molecular Biology of the Cell, 2nd ed.). Enhancer elements derived from viruses may be particularly useful, because they usually have a broader host range. Examples include the SV40 early gene enhancer (Dijkema et al (1985) EMBO J. 4: 761) and the enhancer/promoters derived from the long terminal repeat (LTR) of the Rous Sarcoma Virus (Gorman et al. (1982b) Proc. Natl. Acad. Sci. 79: 6777) and from human cytomegalovirus (Boshart et al. (1985) Cell 41: 521). Additionally, some enhancers are regulatable and become active only in the presence of an inducer, such as a hormone or metal ion (Sassone-Corsi and Borelli (1986) Trends Genet. 2: 215; Maniatis et al. (1987) Science 236: 1237). A DNA molecule may be expressed intracellularly in mammalian cells. A promoter sequence may be directly linked with the DNA molecule, in which case the first amino acid at the N-terminus of the recombinant protein will always be a methionine, which is encoded by the ATG start codon. If desired, the N-terminus may be cleaved from the protein by in vitro incubation with cyanogen bromide. Alternatively, foreign proteins can also be secreted from the cell into the growth media by creating chimeric DNA molecules that encode a fusion protein comprised of a leader sequence fragment that provides for secretion of the foreign protein in mammalian cells. Preferably, there are processing sites encoded between the leader fragment and the foreign gene that can be cleaved either in vivo or in vitro. The leader sequence fragment usually encodes a signal peptide comprised of hydrophobic amino acids which direct the secretion of the protein from the cell. The adenovirus triparite leader is an example of a leader sequence that provides for secretion of a foreign protein in mammalian cells. Usually, transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-transcriptional cleavage and polyadenylation (Birnstiel et al. (1985) Cell 41: 349; Proudfoot and Whitelaw (1988) "Termination and 3' end processing of eukaryotic RNA" in Transcription and splicing (ed. B.D. Hames and D.M. Glover); Proudfoot (1989) Trends Biochem. Sci. 14: 105). These sequences direct the transcription of an mRNA which can be translated into the polypeptide encoded by the DNA. Examples of transcription terminator/polyadenylation signals include those derived from SV40 (Sambrook et al (1989) "Expression of cloned genes in cultured mammalian cells." In Molecular Cloning: A Laboratory Manual). Usually, the above described components, comprising a promoter, polyadenylation signal, and transcription termination sequence are put together into expression constructs. Enhancers, introns with functional splice donor and acceptor sites, and leader sequences may also be included in an expression construct, if desired. Expression constructs are often maintained in a replicon, such as an extrachromosomal element (e.g. plasmids) capable of stable maintenance in a host, such as mammalian cells or bacteria. Mammalian replication systems include those derived from animal viruses, which require trans-acting factors to replicate. For example, plasmids containing the replication systems of papovaviruses, such as SV40 (Gluzman (1981) Cell 23: 175) or polyomavirus, replicate to extremely high copy number in the presence of the appropriate viral T antigen. Additional examples of mammalian replicons include those derived from bovine papillomavirus and Epstein-Barr virus. Additionally, the replicon may have two replication systems, thus allowing it to be maintained, for example, in mammalian cells for expression and in a prokaryotic host for cloning and amplification. Examples of such mammalian-bacteria shuttle vectors include pMT2 (Kaufman et al. (1989) Mol. Cell. Biol. 9: 9469) and pHEBO (Shimizu et al. (1986) Mol. Cell. Biol. 6: 1074). The transformation procedure used depends upon the host to be transformed. Methods for introduction of heterologous polynucleotides into mammalian cells are known in the art and include dextran-mediated transfection, calcium phosphate precipitation, polybrene mediated transfection, protoplast fusion, electroporation, encapsulation of the polynucleotide (s) in liposomes, and direct microinjection of the DNA into nuclei. Mammalian cell lines available as hosts for expression are known in the art and include many immortalized cell lines available from the American Type Culture Collection (ATCC), including but not limited to, Chinese hamster ovary (CHO) cells, HeLa cells, baby hamster kidney (BHK) cells, monkey kidney cells (COS), human hepatocellular carcinoma cells (e.g. Hep G2), and a number of other cell lines.

### Baculovirus Systems

The polynucleotide encoding the protein can also be inserted into a suitable insect expression vector, and is operably linked to the control elements within that vector. Vector construction employs techniques which are known in the art. Generally, the components of the expression system include a transfer vector, usually a bacterial plasmid, which contains both a fragment of the baculovirus genome, and a convenient restriction site for insertion of the heterologous gene or genes to be expressed; a wild type baculovirus with a sequence homologous to the baculovirus-specific fragment in the transfer vector (this allows for the homologous recombination of the heterologous gene in to the baculovirus genome); and appropriate insect host cells and growth media. After inserting the DNA sequence encoding the protein into the transfer vector, the vector and the wild type viral genome are transfected into an insect host cell where the vector and viral genome are allowed to recombine. The packaged recombinant virus is expressed and recombinant plaques are identified and purified. Materials and methods for baculovirus/insect cell expression systems are commercially available in kit form from, inter alia, Invitrogen, San Diego CA ("MaxBac" kit). These techniques are generally known to those skilled in the art and fully described in Summers and Smith, Texas Agricultural Experiment Station Bulletin No. 1555 (1987) (hereinafter "Summers and Smith").
Prior to inserting the DNA sequence encoding the protein into the baculovirus genome, the above described components, comprising a promoter, leader (if desired), coding sequence, and transcription termination sequence, are usually assembled into an intermediate transplacement construct (transfer vector). This may contain a single gene and operably linked regulatory elements; multiple genes, each with its owned set of operably linked regulatory elements; or multiple genes, regulated by the same set of regulatory elements. Intermediate transplacement constructs are often maintained in a replicon, such as an extra-chromosomal element (e.g. plasmids) capable of stable maintenance in a host, such as a bacterium. The replicon will have a replication system, thus allowing it to be maintained in a suitable host for cloning and amplification. Currently, the most commonly used transfer vector for introducing foreign genes into AcNPV is pAc373. Many other vectors, known to those of skill in the art, have also been designed. These include, for example, pVL985 (which alters the polyhedrin start codon from ATG to ATT, and which introduces a BamHI cloning site 32 basepairs downstream from the ATT; see Luckow and Summers, Virology (1989) 17:31. The plasmid usually also contains the polyhedrin polyadenylation signal (Miller et al. (1988) Ann. Rev. Microbiol., 42: 177) and a prokaryotic ampicillin-resistance (amp) gene and origin of replication for selection and propagation in *E. coli.* Baculovirus transfer vectors usually contain a baculovirus promoter. A baculovirus promoter is any DNA sequence capable of binding a baculovirus RNA polymerase and initiating the downstream (5' to 3') transcription of a coding sequence (e.g. structural gene) into mRNA. A promoter will have a transcription initiation region which is usually placed proximal to the 5' end of the coding sequence. This transcription initiation region usually includes an RNA polymerase binding site and a transcription initiation site. A baculovirus transfer vector may also have a second domain called an enhancer, which, if present, is usually distal to the structural gene. Expression may be either regulated or constitutive.
Structural genes, abundantly transcribed at late times in a viral infection cycle, provide particularly useful promoter sequences. Examples include sequences derived from the gene encoding the viral polyhedron protein, Friesen et al., (1986) "The Regulation of Baculovirus Gene Expression," in: The Molecular Biology of Baculoviruses (ed. Walter Doerfler); EPO Publ. Nos. 127 839 and 155 476; and the gene encoding the p10 protein, Vlak et al., (1988), J. Gen. Virol. 69:765. DNA encoding suitable signal sequences can be derived from genes for secreted insect or baculovirus proteins, such as the baculovirus polyhedrin gene (Carbonell et al. (1988) Gene, 73:409). Alternatively, since the signals for mammalian cell posttranslational modifications (such as signal peptide cleavage, proteolytic cleavage, and phosphorylation) appear to be recognized by insect cells, and the signals required for secretion and nuclear accumulation also appear to be conserved between the invertebrate cells and vertebrate cells, leaders of non-insect origin, such as those derived from genes encoding human-interferon, Maeda et al., (1985), Nature 315:592; human gastrin-releasing
peptide, Lebacq-Verheyden et al., (1988), Molec. Cell. Biol. 8: 3129; human IL-2, Smith et al., (1985) Proc. Natl. Acad. Sci. USA, 82:8404; mouse IL-3, (Miyajima et al., (1987) Gene 58:273; and human glucocerebrosidase, Martin et al. (1988) DNA, 7:99, can also be used to provide for secretion in insects. A recombinant polypeptide or polyprotein may be expressed intracellularly or, if it is expressed with the proper regulatory sequences, it can be secreted. Good intracellular expression of non-fused foreign proteins usually requires heterologous genes that ideally have a short leader sequence containing suitable translation initiation signals preceding an ATG start signal. If desired, methionine at the N-terminus may be cleaved from the mature protein by in vitro incubation with cyanogen bromide.
Alternatively, recombinant polyproteins or proteins which are not naturally secreted can be secreted from the insect cell by creating chimeric DNA molecules that encode a fusion protein comprised of a leader sequence fragment that provides for secretion of the foreign protein in insects. The leader sequence fragment usually encodes a signal peptide comprised of hydrophobic amino acids which direct the translocation of the protein into the endoplasmic reticulum.
After insertion of the DNA sequence and/or the gene encoding the expression product precursor of the protein, an insect cell host is co-transformed with the heterologous DNA of the transfer vector and the genomic DNA of wild type baculovirus -- usually by co-transfection. The promoter and transcription termination sequence of the construct will usually comprise a 2-5kb section of the baculovirus genome. Methods for introducing heterologous DNA into the desired site in the baculovirus virus are known in the art. (See Summers and Smith supra; Ju et al, (1987); Smith et al., Mol. Cell. Biol. (1983) 3: 2156; and Luckow and Summers (1989)). For example, the insertion can be into a gene such as the polyhedrin gene, by homologous double crossover recombination; insertion can also be into a restriction enzyme site engineered into the desired baculovirus gene. Miller et al., (1989), Bioessays 4: 91.The DNA sequence, when cloned in place of the polyhedrin gene in the expression vector, is flanked both 5' and 3' by polyhedrin-specific sequences and is positioned downstream of the polyhedrin promoter. The newly formed baculovirus expression vector is subsequently packaged into an infectious recombinant baculovirus. Homologous recombination occurs at low frequency (between about 1% and about 5%); thus, the majority of the virus produced after cotransfection is still wild-type virus. Therefore, a method is necessary to identify recombinant viruses. An advantage of the expression system is a visual screen allowing recombinant viruses to be distinguished. The polyhedrin protein, which is produced by the native virus, is produced at very high levels in the nuclei of infected cells at late times after viral infection. Accumulated polyhedrin protein forms occlusion bodies that also contain embedded particles. These occlusion bodies, up to 15 m in size, are highly refractile, giving them a bright shiny appearance that is readily visualized under the light microscope. Cells infected with recombinant viruses lack occlusion bodies. To distinguish recombinant virus from wild-type virus, the transfection supernatant is plaqued onto a monolayer of insect cells by techniques known to those skilled in the art. Namely, the plaques are screened under the light microscope for the presence (indicative of wild-type virus) or absence (indicative of recombinant virus) of occlusion bodies. "Current Protocols in Microbiology" Vol. 2 (Ausubel et al. eds) at 16.8 (Supp. 10, 1990); Summers and Smith, supra; Miller et al. (1989). Recombinant baculovirus expression vectors have been developed for infection into several insect cells. For example, recombinant baculoviruses have been developed for, inter alia: Aedes aegypti, Autographa californica, Bombyx mori, Drosophila melanogaster, Spodoptera frugiperda, and Trichoplusia ni (WO 89/046699; Carbonell et al., (1985) J. Virol. 56: 153; Wright (1986) Nature 321: 718; Smith et al., (1983) Mol. Cell. Biol. 3: 2156; and see generally, Fraser, et al. (1989) In Vitro Cell. Dev. Biol. 25: 225). Cells and cell culture media are commercially available for both direct and fusion expression of heterologous polypeptides in a baculovirus/expression system; cell culture technology is generally known to those skilled in the art. See, e.g. Summers and Smith supra. The modified insect cells may then be grown in an appropriate nutrient medium, which allows for stable maintenance of the plasmid (s) present in the modified insect host. Where the expression product gene is under inducible control, the host may be grown to high density, and expression induced. Alternatively, where expression is constitutive, the product will be continuously expressed into the medium and the nutrient medium must be continuously circulated, while removing the product of interest and augmenting depleted nutrients. The product may be purified by such techniques as chromatography, e.g. HPLC, affinity chromatography, ion exchange chromatography, etc.; electrophoresis; density gradient centrifugation; solvent extraction, etc. As appropriate, the product may be further purified, as required, so as to remove substantially any insect proteins which are also present in the medium, so as to provide a product which is at least substantially free of host debris, e.g. proteins, lipids and polysaccharides. In order to obtain protein expression, recombinant host cells derived from the transformants are incubated under conditions which allow expression of the recombinant protein encoding sequence. These conditions will vary, dependent upon the host cell selected. However, the conditions are readily ascertainable to those of ordinary skill in the art, based upon what is known in the art.

### Plant Systems

There are many plant cell culture and whole plant genetic expression systems known in the art. Exemplary plant cellular genetic expression systems include those described in patents, such as: US 5,693,506; US 5,659,122; and US 5,608,143. Additional examples of genetic expression in plant cell culture has been described by Zenk, Phytochemistry 30: 3861-3863 (1991). Typically, using techniques known in the art, a desired polynucleotide sequence is inserted into an expression cassette comprising genetic regulatory elements designed for operation in plants. The expression cassette is inserted into a desired expression vector with companion sequences upstream and downstream from the expression cassette suitable for expression in a plant host. The companion sequences will be of plasmid or viral origin and provide necessary characteristics to the vector to permit the vectors to move DNA from an original cloning host, such as bacteria, to the desired plant host. The basic bacterial/plant vector construct will preferably provide a broad host range prokaryote replication origin; a prokaryote selectable marker; and, for Agrobacterium transformations, T DNA sequences for Agrobacterium-mediated transfer to plant chromosomes. Where the heterologous gene is not readily amenable to detection, the construct will preferably also have a selectable marker gene suitable for determining if a plant cell has been transformed. A general review of suitable markers, for example for the members of the grass family, is found in Wilmink and Dons, 1993, Plant Mol. Biol. Reptr, 11 (2):165-185. Sequences suitable for permitting integration of the heterologous sequence into the plant genome are also recommended. These might include transposon sequences and the like for homologous recombination as well as Ti sequences which permit random insertion of a heterologous expression cassette into a plant genome. Suitable prokaryote selectable markers include resistance toward antibiotics such as ampicillin or tetracycline. Other DNA sequences encoding additional functions may also be present in the vector, as is known in the art. The nucleic acid molecules of the subject invention may be included into an expression cassette for expression of the protein (s) of interest. Usually, there will be only one expression cassette, although two or more are feasible. The recombinant expression cassette will contain in addition to the heterologous protein encoding sequence the following elements, a promoter region, plant 5' untranslated sequences, initiation codon depending upon whether or not the structural gene comes equipped with one, and a transcription and translation termination sequence. Unique restriction enzyme sites at the 5' and 3' ends of the cassette allow for easy insertion into a pre-existing vector. A heterologous coding sequence may be for any protein relating to the present invention. The sequence encoding the protein of interest will encode a signal peptide which allows processing and translocation of the protein, as appropriate, and will usually lack any sequence which might result in the binding of the desired protein of the invention to a membrane. Since, for the most part, the transcriptional initiation region will be for a gene which is expressed and translocated during germination, by employing the signal peptide which provides for translocation, one may also provide for translocation of the protein of interest. In this way, the protein(s) of interest will be translocated from the cells in which they are expressed and may be efficiently harvested. Typically secretion in seeds are across the aleurone or scutellarepithelium layer into the endosperm of the seed. While it is not required that the protein be secreted from the cells in which the protein is produced, this facilitates the isolation and purification of the recombinant protein. Since the ultimate expression of the desired gene product will be in a eucaryotic cell it is desirable to determine whether any portion of the cloned gene contains sequences which will be processed out as introns by the host's splicosome machinery. If so, site-directed mutagenesis of the "intron" region may be conducted to prevent losing a portion of the genetic message as a false intron code, Reed and Maniatis, Cell 41:95-105, 1985. The vector can be microinjected directly into plant cells by use of micropipettes to mechanically transfer the recombinant DNA. Crossway, Mol. Gen. Genet, 202:179-185, 1985. The genetic material may also be transferred into the plant cell by using polyethylene glycol, Krens, et al., Nature, 296, 72-74, 1982. Another method of introduction of nucleic acid segments is high velocity ballistic penetration by small particles with the nucleic acid either within the matrix of small beads or particles, or on the surface, Klein, et al., Nature, 327, 70-73, 1987 and Knudsen and Muller, 1991, Planta, 185:330-336 teaching particle bombardment of barley endosperm to create transgenic barley. Yet another method of introduction would be fusion of protoplasts with other entities, either minicells, cells, lysosomes or other fusible lipidsurfaced bodies, Fraley, et al., Proc. Natl. Acad. Sci. USA, 79, 1859-1863, 1982. The vector may also be introduced into the plant cells by electroporation. (Fromm et al., Proc. Natl Acad. Sci. USA 82: 5824, 1985). In this technique, plant protoplasts are electroporated in the presence of plasmids containing the gene construct. Electrical impulses of high field strength reversibly permeabilize biomembranes allowing the introduction of the plasmids. Electroporated plant protoplasts reform the cell wall, divide, and form plant callus. All plants from which protoplasts can be isolated and cultured to give whole regenerated plants can be transformed by the present invention so that whole plants are recovered which contain the transferred gene. It is known that practically all plants can be regenerated from cultured cells or tissues, including but not limited to all major species of sugarcane, sugar beet, cotton, fruit and other trees, legumes and vegetables. Some suitable plants include, for example, species from the genera Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hererocallis, Nemesia, Pelargonium, Panicum, Pennisetum, Ranunculus, Senecio, Salpiglossis, Cucumis, Browaalia, Glycine, Lolium, Zea, Triticum, Sorghum, and Datura. Means for regeneration vary from species to species of plants, but generally a suspension of transformed protoplasts containing copies of the heterologous gene is first provided. Callus tissue is formed and shoots may be induced from callus and subsequently rooted. Alternatively, embryo formation can be induced from the protoplast suspension. These embryos germinate as natural embryos to form plants. The culture media will generally contain various amino acids and hormones, such as auxin and cytokinins. It is also advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.
In some plant cell culture systems, the desired protein of the invention may be excreted or alternatively, the protein may be extracted from the whole plant. Where the desired protein of the invention is secreted into the medium, it may be collected. Alternatively, the embryos and embryoless-half seeds or other plant tissue may be mechanically disrupted to release any secreted protein between cells and tissues. The mixture may be suspended in a buffer solution to retrieve soluble proteins. Conventional protein isolation and purification methods will be then used to purify the recombinant protein. Parameters of time, temperature pH, oxygen, and volumes will be adjusted through routine methods to optimize expression and recovery of heterologous protein.

Preferred molecules according to the invention are disclosed in Tables 1 to 7 below.

In one embodiment the following tags are added at the C-terminus of the lysosomal proteins (see Table 1). Ideally, they contain linker sequences between the lysosomal protein and the tag. The C-terminal tags are fused with the lysosomal proteins in such a way that the tags replace the stop codon of the lysosomal proteins. In case C-terminal amino acids are omitted it is indicated.

**Table 1 - Antp and CLOCK**

| | | |
|---|---|---|
| Linker preceding the tags "Antp" and "CLOCK" | AGATCCCCCGGG | SEQ ID NO. 15 |
| Liner preceding the tags "Antp" and "CLOCK" | GGATCCCCCGGG | SEQ ID NO.16 |
| Antp cDNA of tag; | CGCCAGATAAAGATTTGGTTCCAGAATCGGCG CATGAAGTGGAAGAAGTAA | SEQ ID NO.17 |
| Antp amino acid sequence; | RQIKIWFQNRRMKWKK | SEQ ID NO.18 |
| Human CLOCK cDNA tag; | AAAAGAGTATCTAGAAACAAATCTGAAAAGAA ACGTAGATAA | SEQ ID NO.19 |
| Human CLOCK amino acid sequence tag; | KRVSRNKSEKKRR | SEQ ID NO.20 |

The following tags are derived from the human basic fibroblast growth factor (FGF2) and possess an N-terminal linker (AGATCCGTCGACATCGAAGGTAGAGGCATT or GGATCCGTCGACATCGAAGGTAGAGGCATT) containing the factor Xa cleavage site "IEGR" (Table 2).

**Table 2 - FGF2 and variants thereof**

| | | |
|---|---|---|
| N-terminal linker | AGATCCGTCGACATCGAAGGTAGAGGCATT | SEQ ID NO.21 |
| N-terminal linker | GGATCCGTCGACATCGAAGGTAGAGGCATT | SEQ ID NO.22 |
| FGF2 variant 1 (base substitution G206C and G260C (small letter) leading to amino acid substitution s C69S and C87S) cDNA: | CCCGCCTTGCCCGAGGATGGCGGCAGCGGCGC CTTCCCGCCCGGCCACTTCAAGGACCCCAAGC GGCTGTACTGCAAAAACGGGGGCTTCTTCCTG CGCATCCACCCCGACGGCCGAGTTGACGGGGT CCGGGAGAAGAGCGACCCTCACATCAAGCTAC | SEQ ID NO.23 |
| | AACTTCAAGCAGAAGAGAGAGGAGTTGTGTCT ATCAAAGGAGTGTcTGCTAACCGTTACCTGGC TATGAAGGAAGATGGAAGATTACTGGCTTCTA AATcTGTTACGGATGAGTGTTTCTTTTTTGAA CGATTGGAATCTAATAACTACAATACTTACCG GTCAAGGAAATACACCAGTTGGTATGTGGCAC TGAAACGAACTGGGCAGTATAAACTTGGCTCC AAAACAGGACCTGGGCAGAAAGCTATACTTTT TCTTCCAATGTCTGCTAAGAGCTGA | |
| Amino acid sequence of FGF2 variant 1 (base substitution G206C and G260C (small letter) leading to amino acid substitution s C69S and C87S) | PALPEDGGSGAFPPGHFKDPKRLYCKNGGFFL RIHPDGRVDGVREKSDPHIKLQLQAEERGVVS IKGVSANRYLAMKEDGRLLASKSVTDECFFFE RLESNNYNTYRSRKYTSWYVALKRTGQYKLGS KTGPGQKAILFLPMSAK | SEQ ID NO.24 |
| FGF2 variant 2 (same as variant 1 plus reduced FGFR binding ) cDNA | CCCGCCTTGCCCGAGGATGGCGGCAGCGGCGC CTTCCCGCCCGGCCACTTCAAGGACCCCAAGC GGCTGTACTGCAAAAACGGGGGCTTCTTCCTG CGCATCCACCCCGACGGCCGAGTTGACGGGGT CCGGGAGAAGAGCGACCCTCACATCAAGCTAC AACTTCAAGCAGAAGAGAGAGGAGTTGTGTCT ATCAAAGGAGTGTCTGCTAACCGTTACCTGGC TATGAAGGAAGATGGAAGATTACTGGCTTCTA AATCTGTTACGGATGAGTGTTTCTTTTTTGCA CGATTGGAATCTAATAACTACAATACTTACCG GTCAAGGAAATACACCAGTTGGTATGTGGCAC TGAAACGAACTGGGCAGTATAAACTTGGCTCC AAAACAGGACCTGGGCAGAAAGCTATACTTTT TCTTCCAATGTCTGCTAAGAGCTGA | SEQ ID NO.25 |
| FGF2 variant 2 amino acid sequence; | PALPEDGGSGAFPPGHFKDPKRLYCKNGGFFL RIHPDGRVDGVREKSDPHIKLQLQAEERGVVS IKGVSANRYLAMKEDGRLLASKSVTDECFFFA RLESNNYNTYRSRKYTSWYVALKRTGQYKLGS KTGPGQKAILFLPMSAKS | SEQ ID NO.26 |
| FGF2 variant 3 (same as variant 1 plus reduced nuclear translocation) cDNA; | CCCGCCTTGCCCGAGGATGGCGGCAGCGGCGC CTTCCCGCCCGGCCACTTCAAGGACCCCAAGC GGCTGTACTGCAAAAACGGGGGCTTCTTCCTG CGCATCCACCCCGACGGCCGAGTTGACGGGAC AAGGGACAGGAGCGACCAGCACATTCAGCTGC AGCTCAGTGCAGAAGAGAGAGGAGTTGTGTCT | SEQ ID NO.27 |
| | ATCAAAGGAGTGTCTGCTAACCGTTACCTGGC TATGAAGGAAGATGGAAGATTACTGGCTTCTA AATCTGTTACGGATGAGTGTTTCTTTTTTGAA CGATTGGAATCTAATAACTACAATACTTACCG GTCAAGGAAATACACCAGTTGGTATGTGGCAC TGAAACGAACTGGGCAGTATAAACTTGGCTCC AAAACAGGACCTGGGCAGAAAGCTATACTTTT TCTTCCAATGTCTGCTAAGAGCTGA | |
| FGF2 variant 3 amino acid sequence; | PALPEDGGSGAFPPGHFKDPKRLYCKNGGFFL RIHPDGRVDGTRDRSDQHIQLQLSAEERGVVS IKGVSANRYLAMKEDGRLLASKSVTDECFFFE RLESNNYNTYRSRKYTSWYVALKRTGQYKLGS KTGPGQKAILFLPMSAKS | SEQ ID NO.28 |
| FGF2 variant 4 (same as variant 1 plus reduced FGFR binding and reduced nuclear translocatio n) cDNA: | CCCGCCTTGCCCGAGGATGGCGGCAGCGGCGC CTTCCCGCCCGGCCACTTCAAGGACCCCAAGC GGCTGTACTGCAAAAACGGGGGCTTCTTCCTG CGCATCCACCCCGACGGCCGAGTTGACGGGAC AAGGGACAGGAGCGACCAGCACATTCAGCTGC AGCTCAGTGCAGAAGAGAGAGGAGTTGTGTCT ATCAAAGGAGTGTCTGCTAACCGTTACCTGGC TATGAAGGAAGATGGAAGATTACTGGCTTCTA AATCTGTTACGGATGAGTGTTTCTTTTTTGCA CGATTGGAATCTAATAACTACAATACTTACCG GTCAAGGAAATACACCAGTTGGTATGTGGCAC TGAAACGAACTGGGCAGTATAAACTTGGCTCC AAAACAGGACCTGGGCAGAAAGCTATACTTTT TCTTCCAATGTCTGCTAAGAGCTGA | SEQ ID NO.29 |
| FGF2 variant 4 amino acid sequence | PALPEDGGSGAFPPGHFKDPKRLYCKNGGFFL RIHPDGRVDGTRDRSDQHIQLQLSAEERGVVS IKGVSANRYLAMKEDGRLLASKSVTDECFFFA RLESNNYNTYRSRKYTSWYVALKRTGQYKLGS KTGPGQKAILFLPMSAKS | SEQ ID NO.30 |

The following sequences demonstrate the C-terminal tags fused to the cDNA of human tripeptidyl peptidase 1 (TPP1) (Table 3).

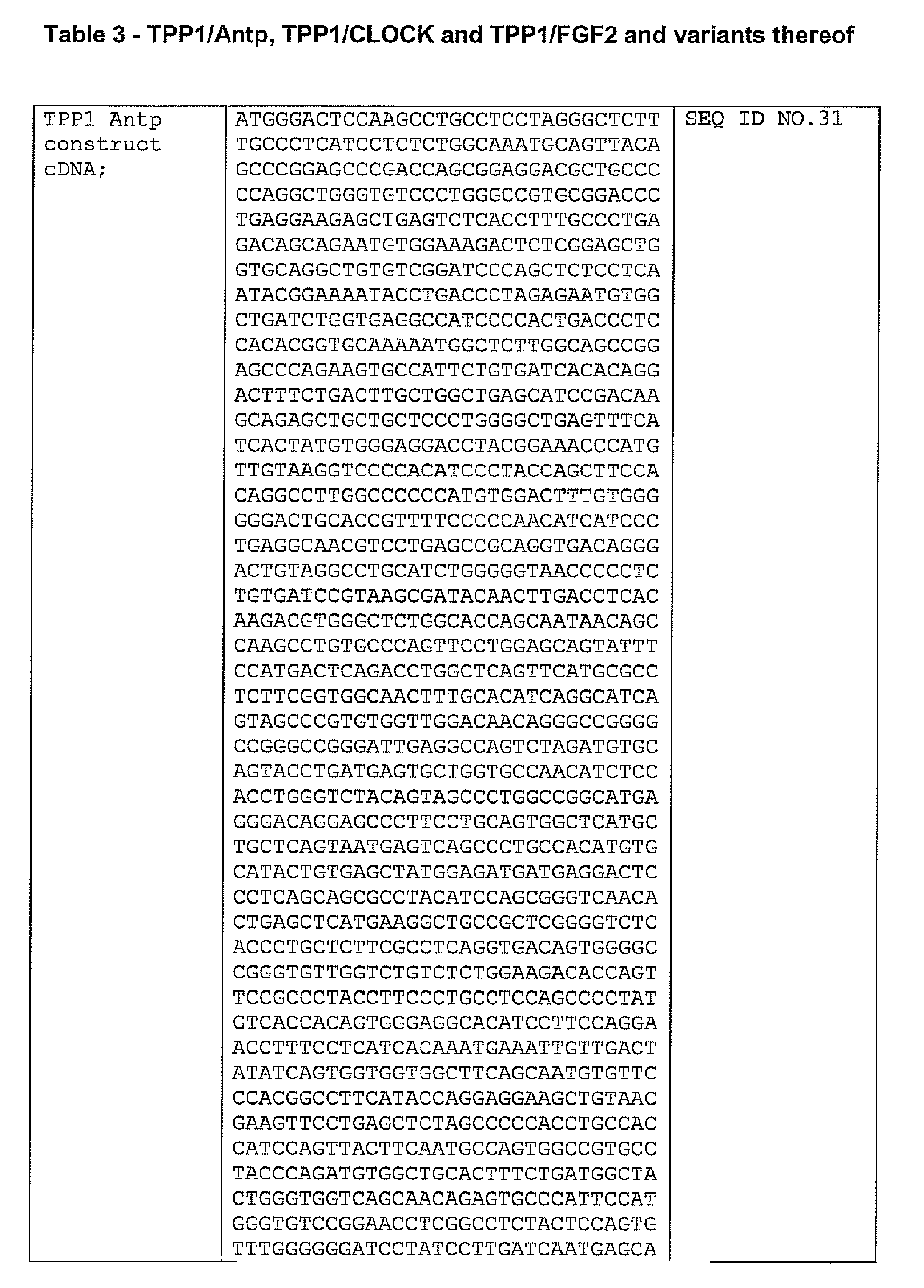

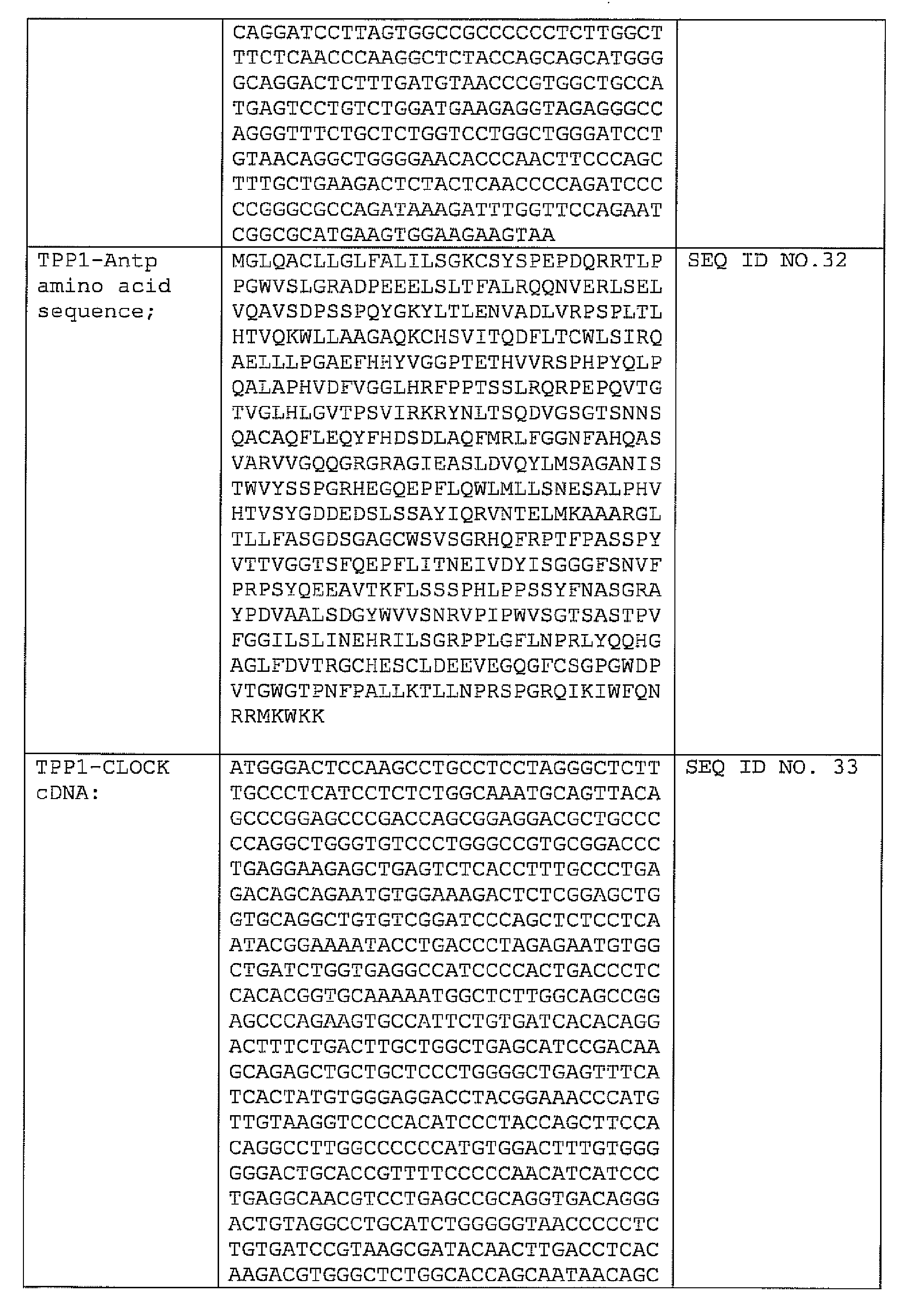

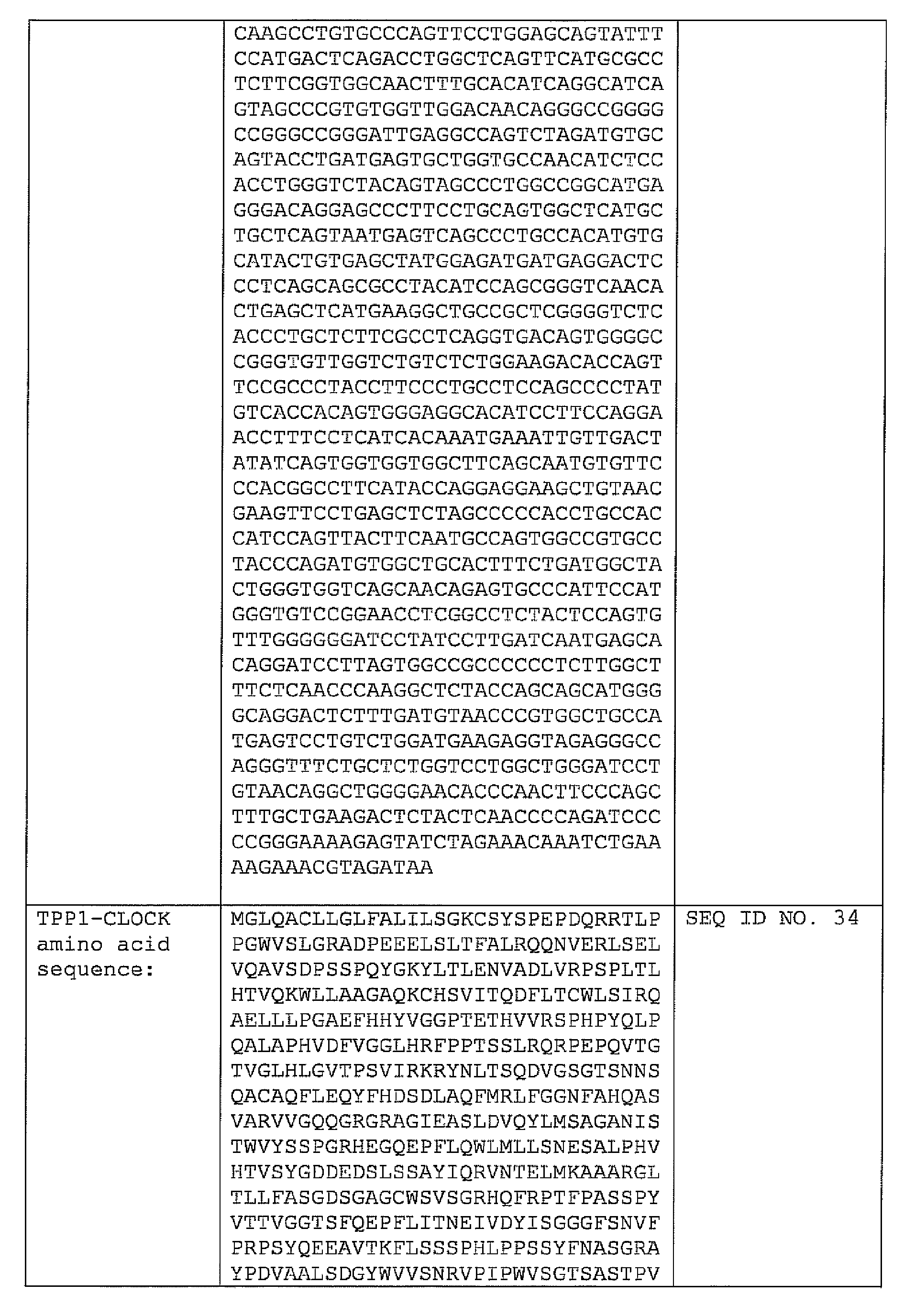

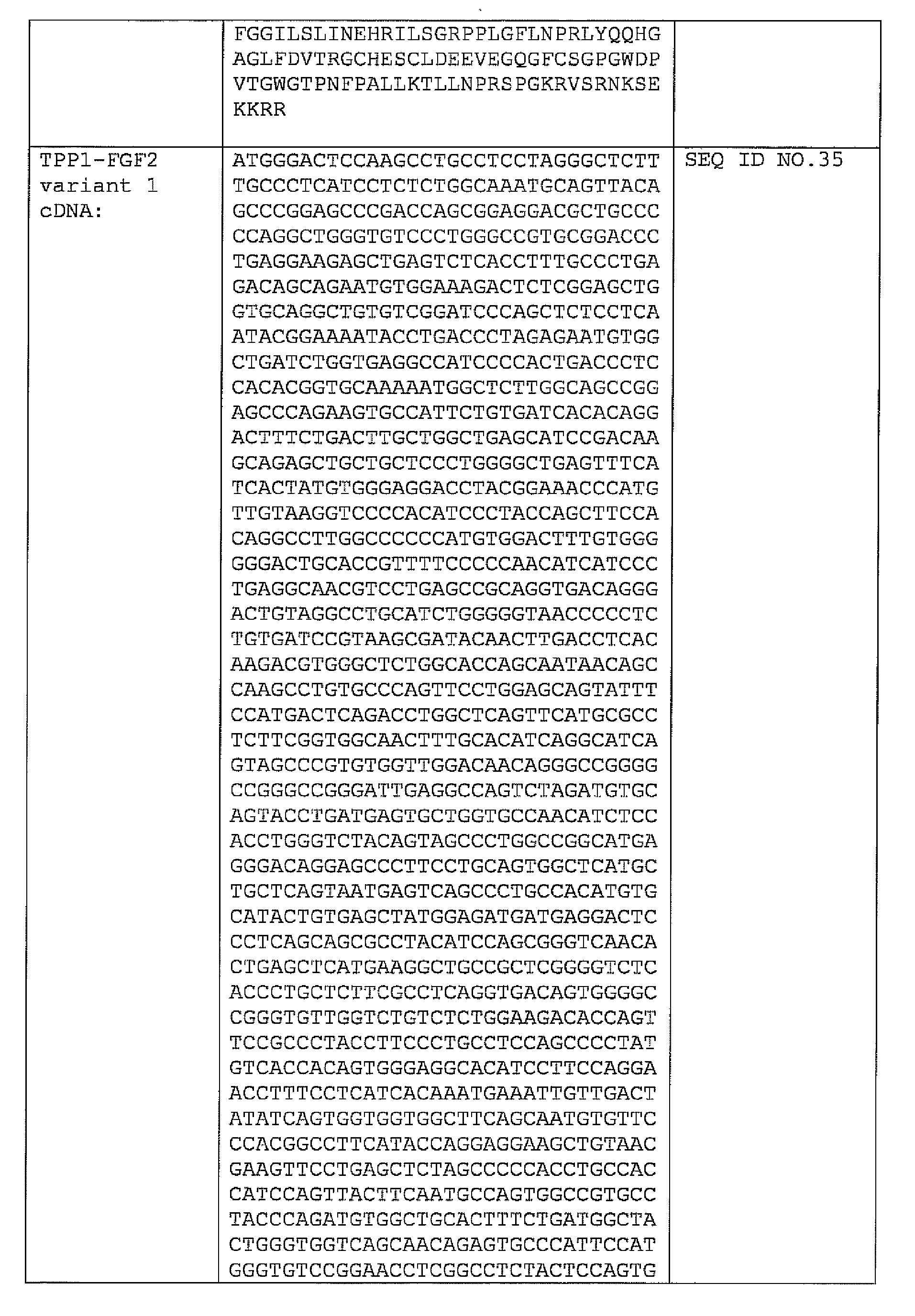

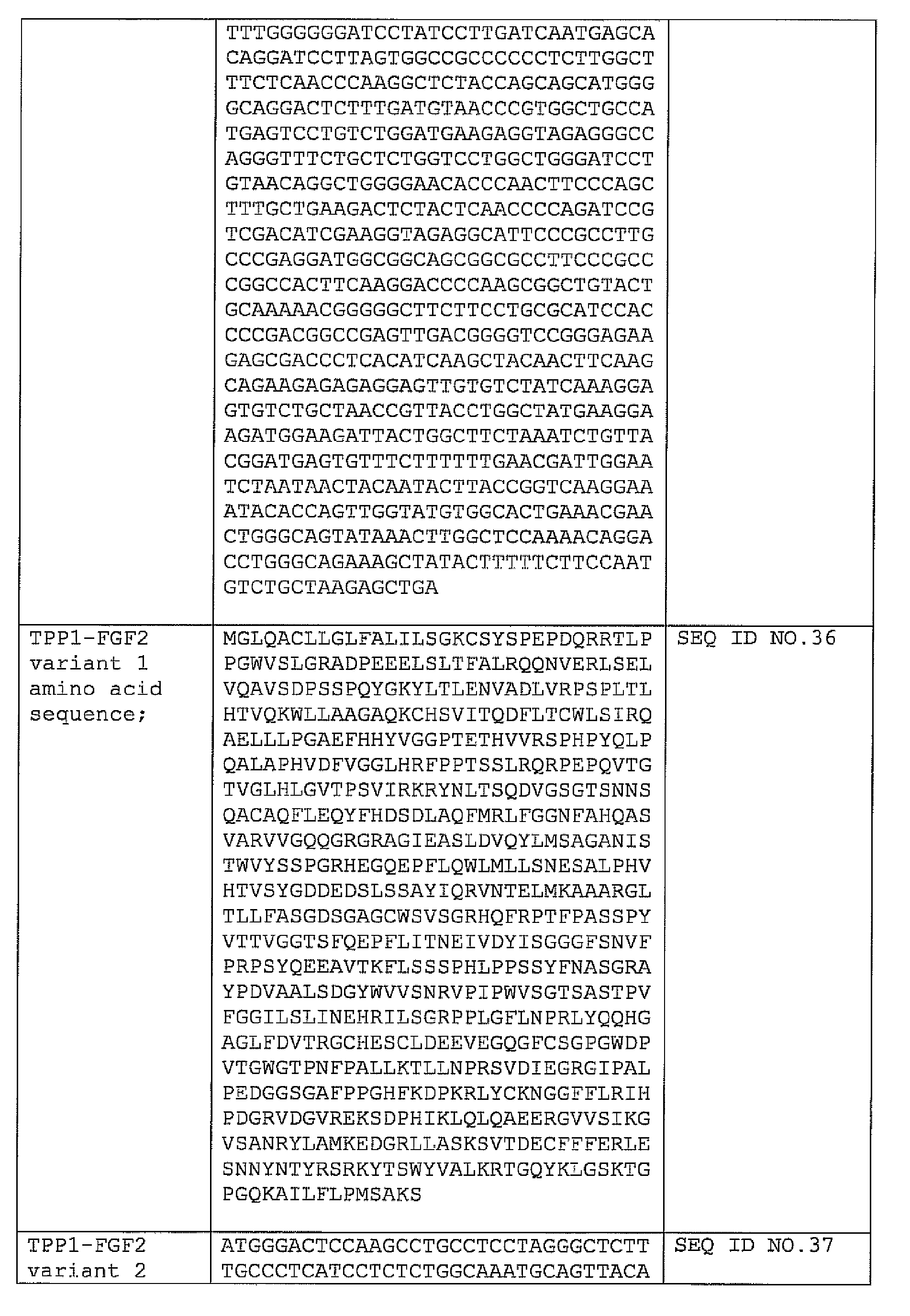

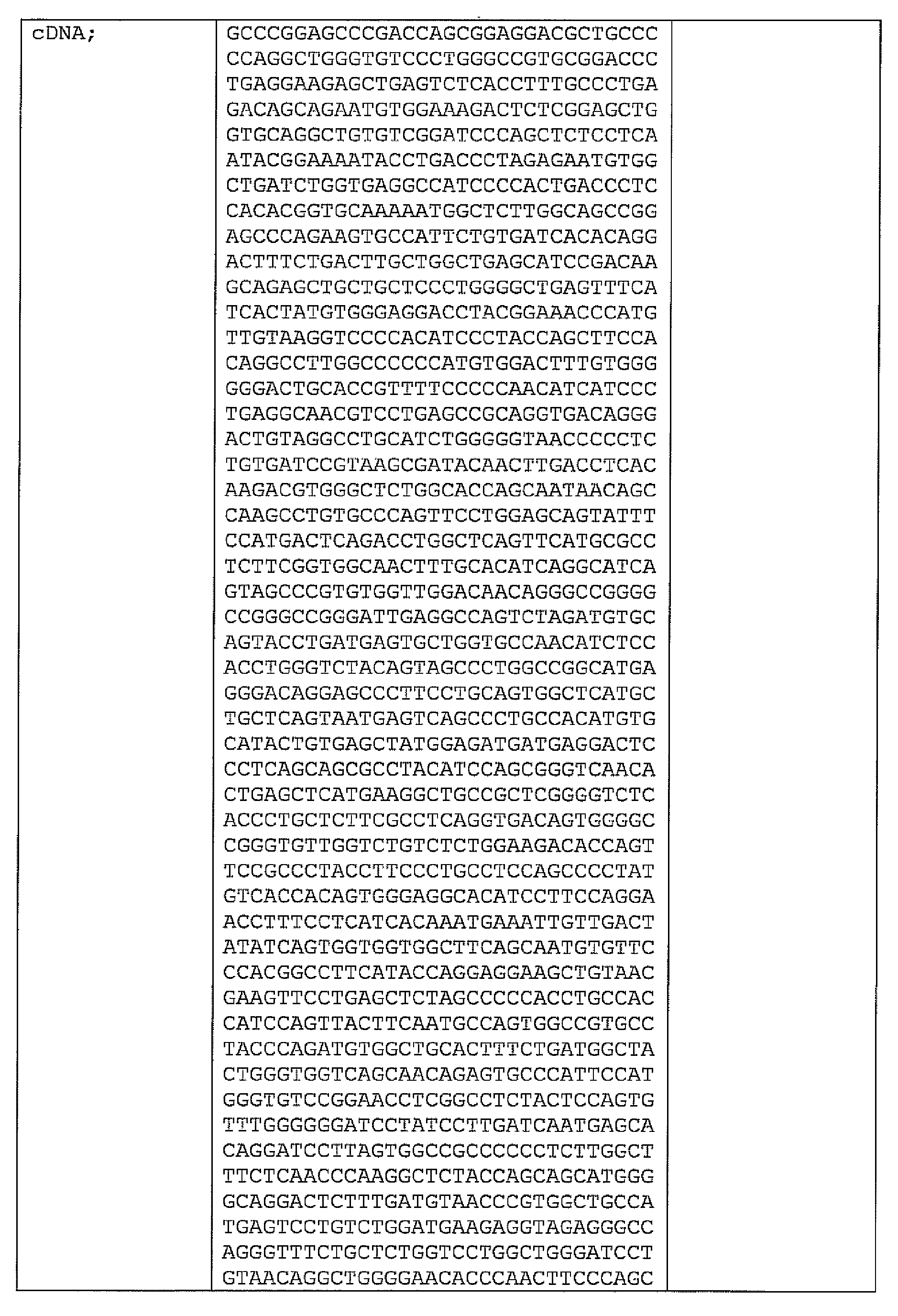

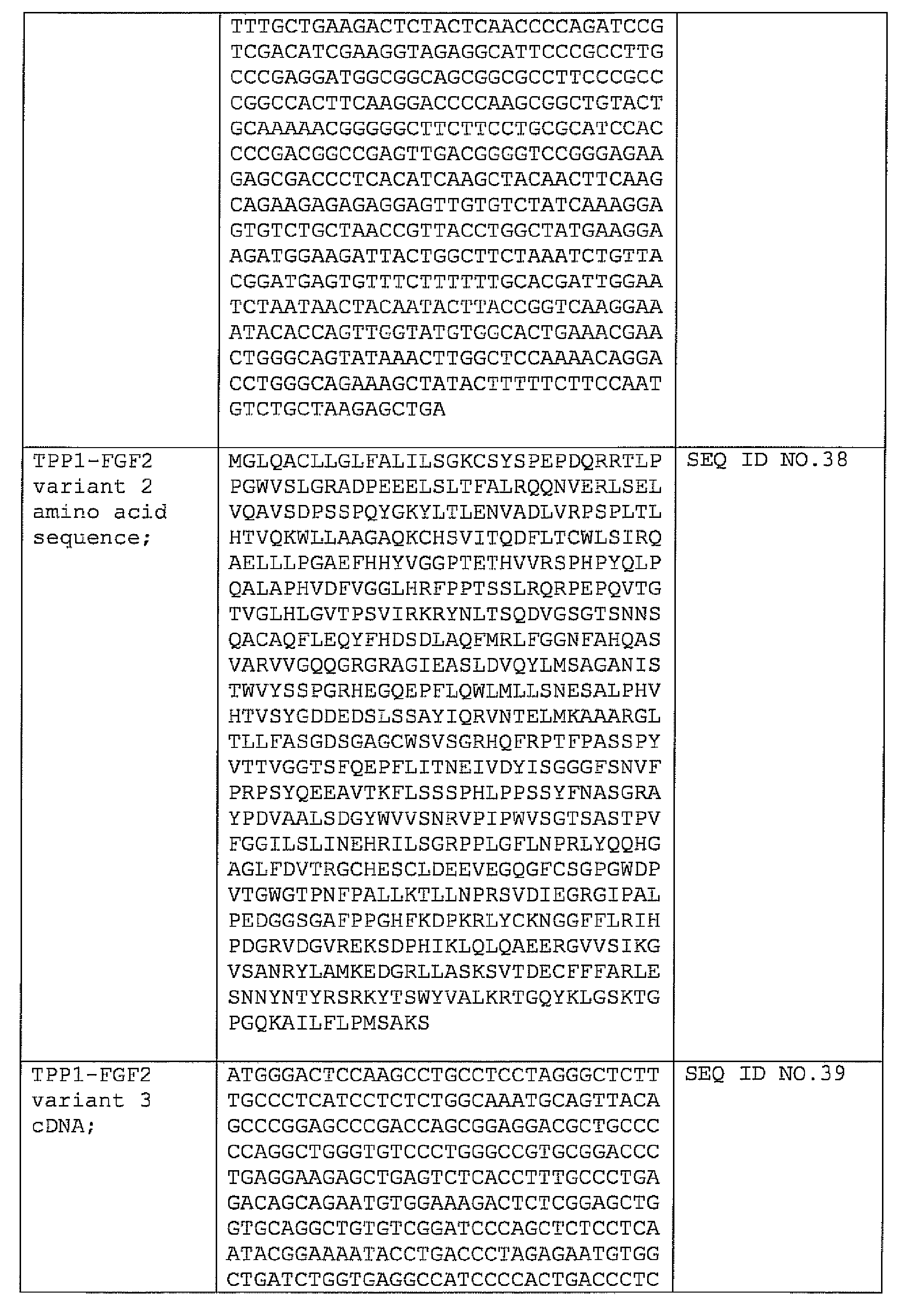

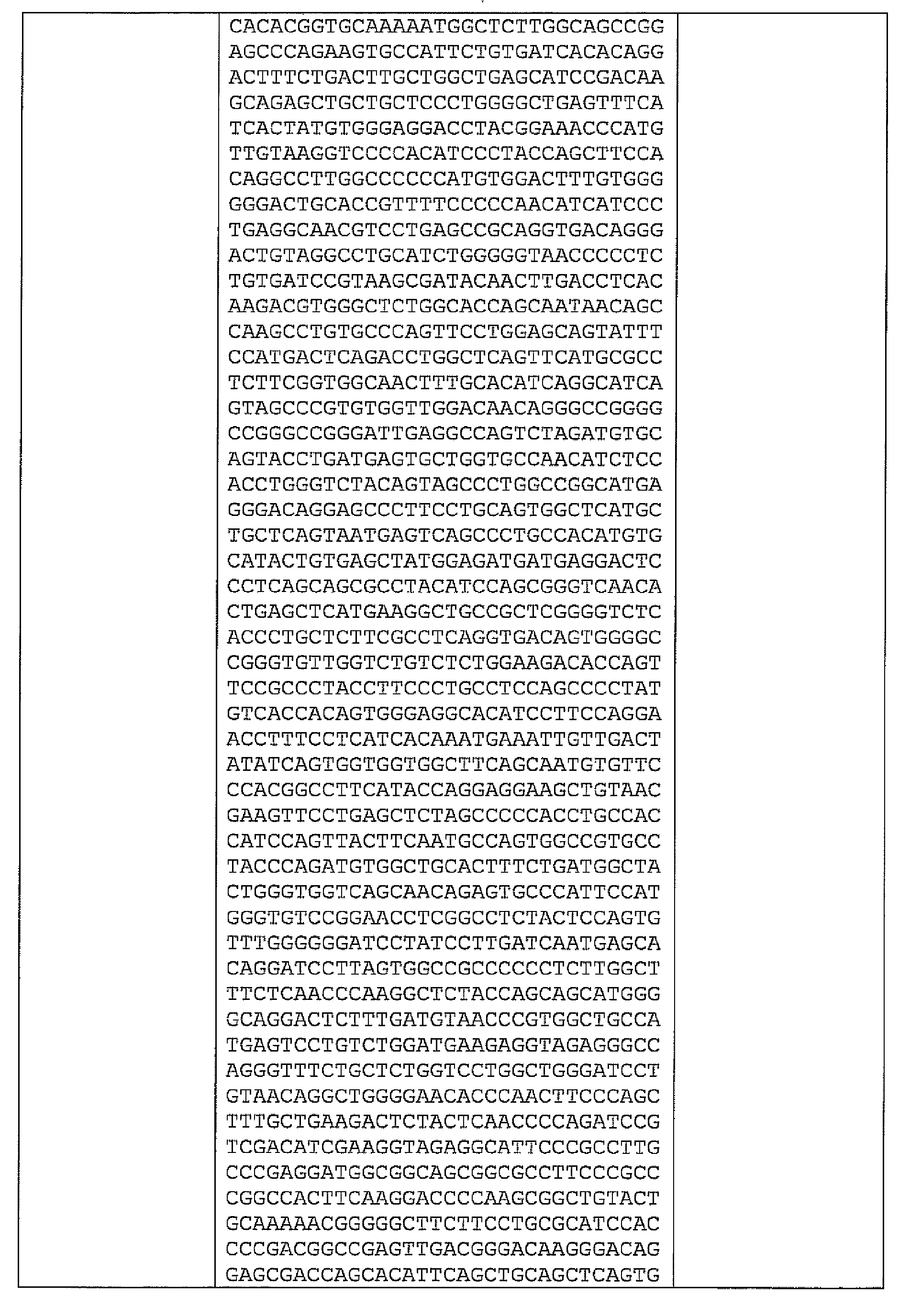

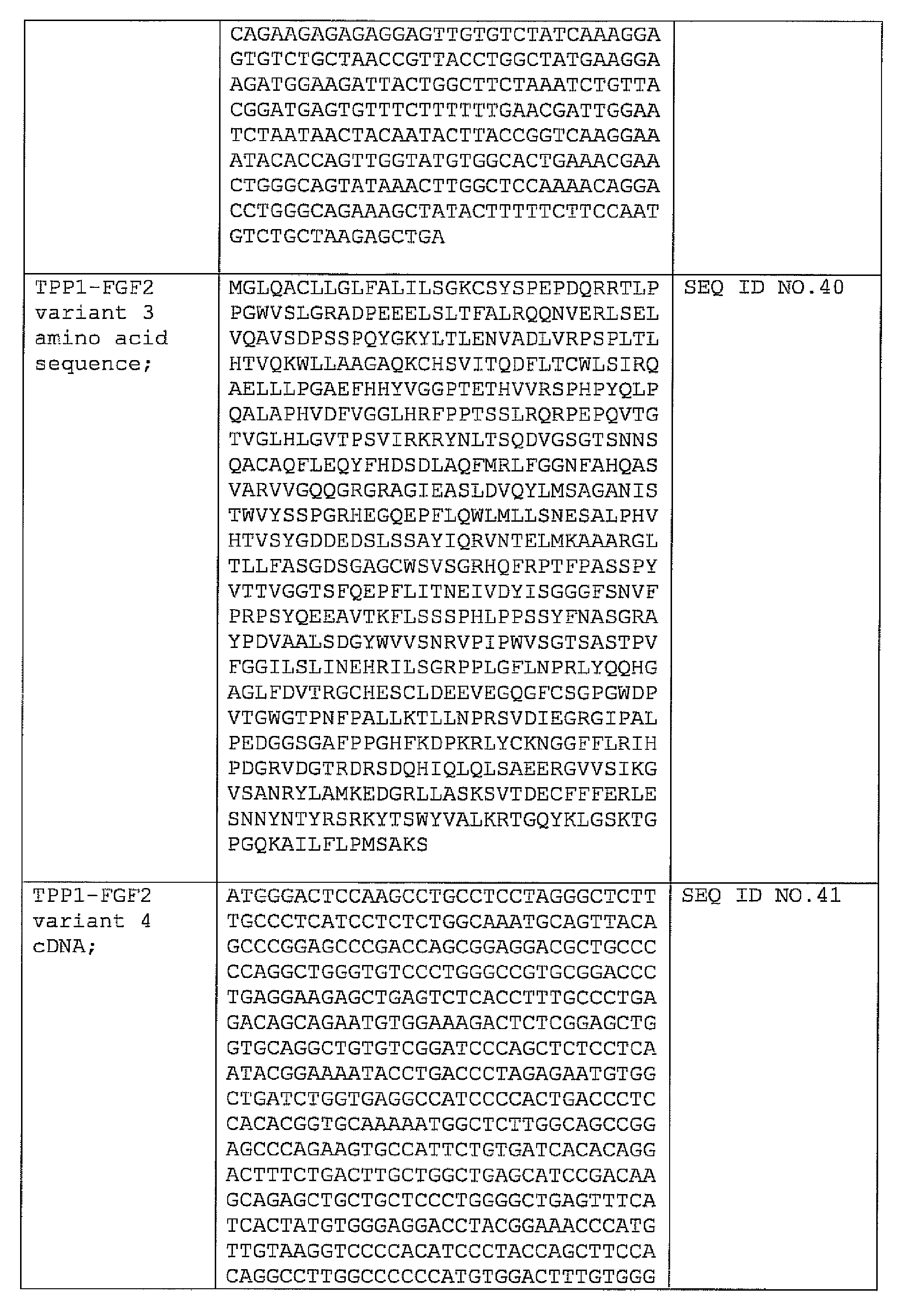

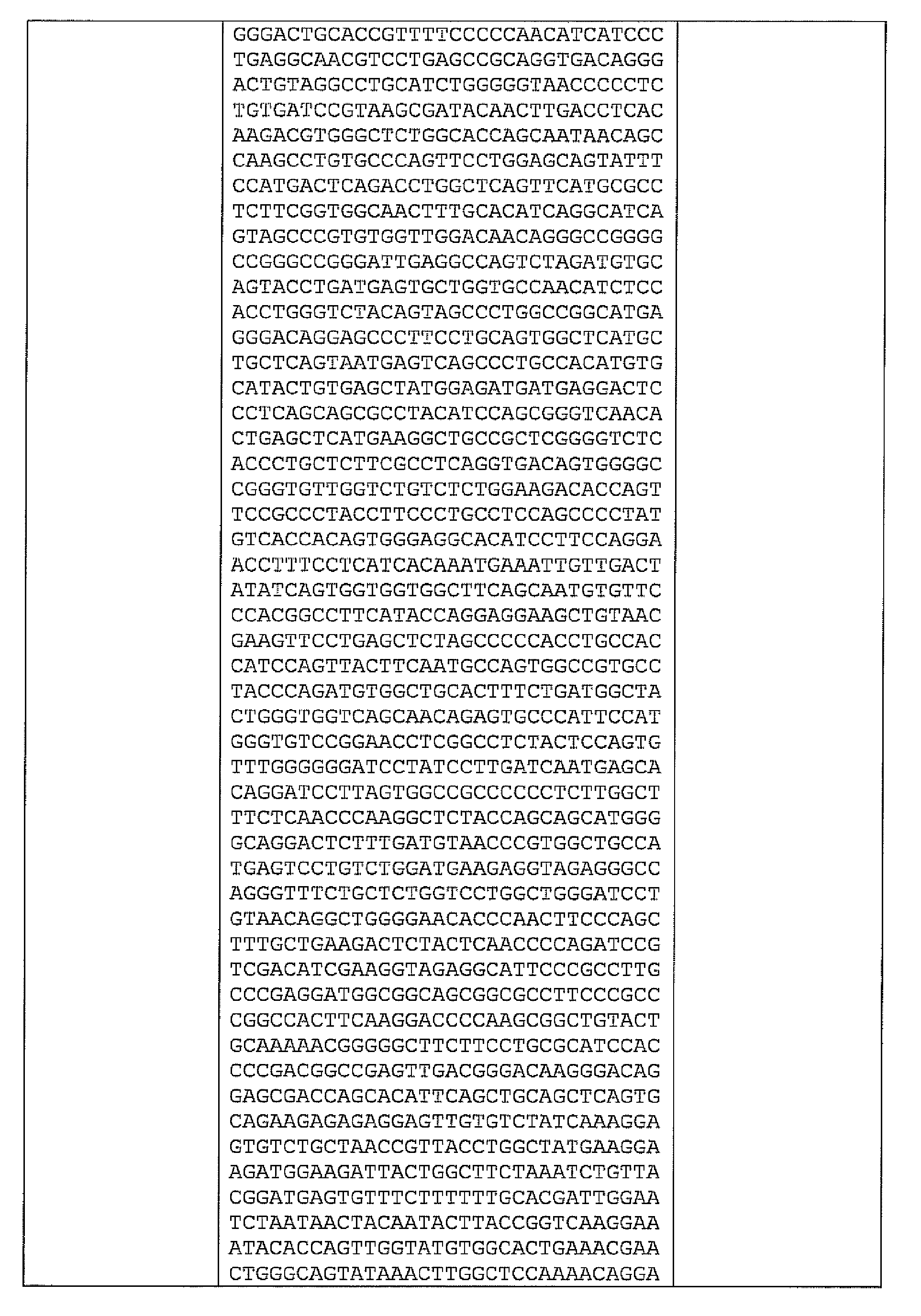

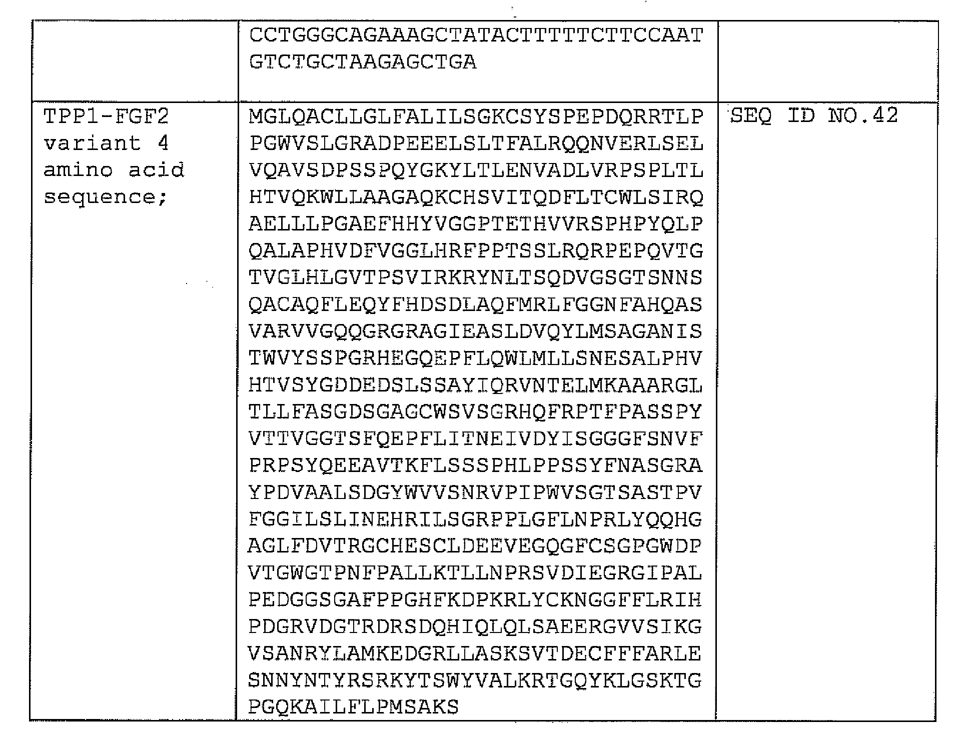

The following tags (Table 4) are derived from the human heparin-binding epidermal growth factor (HB-EGF). They are added at the N-terminus of the lysosomal proteins and replace the signal peptide of the lysosomal proteins.

Two different HB-EGF tags were designed. The last nucleotide "T" of HB1 and HB2 is alternatively replaced by "C":

**Table 4 HB1 and HB2 tags**

| | | |
|---|---|---|
| HB1 cDNA | ATGCAGCCCTCCAGCCTTCTGCCGCTCGCCCT CTGCCTGCTGGCTGCACCCGCCGGATCTTCCA AGCCACAAGCACTGGCCACACCAAACAAGGAG GAGCACGGGAAAAGAAAGAAGAAAGGCAAGGG GCTAGGGAAGAAGAGGGACCCATGTCTTCGGA AATACAAGGACTTCTGCATCCATGGAGAATGC AAATATGTGAAGGAGCTCCGGGCTCCCTCCTG CATCTGCCACCCGGGTTACCATGGAGAGAGGT | SEQ ID NO.43 |
| | GTCATGGGCTGAGCGGATCT | |
| HB2 cDNA | ATGCAGCCCTCCAGCCTTCTGCCGCTCGCCCT CTGCCTGCTGGCTGCACCCGCCGGATCTGGGA AAAGAAAGAAGAAAGGCAAGGGGCTAGGGAAG AAGAGGGACCCATCTCTTCGGAAATACAAGGA CTTCTCCGGATCT | SEQ ID NO.44 |
| HB1 amino acid sequence | MQPSSLLPLALCLLAAPAGSSKPQALATPNKE EHGKRKKKGKGLGKKRDPCLRKYKDFCIHGEC KYVKELRAPSCICHPGYHGERCHGLSGS | SEQ ID NO. 71 |
| HB2 amino acid sequence | MQPSSLLPLALCLLAAPAGSGKRKKKGKGLGK KRDPSLRKYKDFSGS | SEQ ID NO. 72 |

The following sequences (Table 5) disclose the N-terminal tags fused to the cDNA of human sulfamidase (hSGSH)

**Table 5 - HB1/SGSH and HB2/SGSH**

| | | |
|---|---|---|
| HB1-SGSH cDNA: | ATGCAGCCCTCCAGCCTTCTGCCGCTCGCCCT CTGCCTGCTGGCTGCACCCGCCGGATCTTCCA AGCCACAAGCACTGGCCACACCAAACAAGGAG GAGCACGGGAAAAGAAAGAAGAAAGGCAAGGG GCTAGGGAAGAAGAGGGACCCATGTCTTCGGA AATACAAGGACTTCTGCATCCATGGAGAATGC AAATATGTGAAGGAGCTCCGGGCTCCCTCCTG CATCTGCCACCCGGGTTACCATGGAGAGAGGT GTCATGGGCTGAGCGGATCTCGTCCCCGGAAC GCACTGCTGCTCCTCGCGGATGACGGAGGCTT TGAGAGTGGCGCGTACAACAACAGCGCCATCG CCACCCCGCACCTGGACGCCTTGGCCCGCCGC AGCCTCCTCTTTCGCAATGCCTTCACCTCGGT CAGCAGCTGCTCTCCCAGCCGCGCCAGCCTCC TCACTGGCCTGCCCCAGCATCAGAATGGGATG TACGGGCTGCACCAGGACGTGCACCACTTCAA CTCCTTCGACAAGGTGCGGAGCCTGCCGCTGC TGCTCAGCCAAGCTGGTGTGCGCACAGGCATC ATCGGGAAGAAGCACGTGGGGCCGGAGACCGT GTACCCGTTTGACTTTGCGTACACGGAGGAGA ATGGCTCCGTCCTCCAGGTGGGGCGGAACATC ACTAGAATTAAGCTGCTCGTCCGGAAATTCCT | SEQ ID NO.45 |
| | GCAGACTCAGGATGACCGGCCTTTCTTCCTCT ACGTCGCCTTCCACGACCCCACCGCTGTGG CACTCCCAGCCCCAGTACGGAACCTTCTGTGA GAAGTTTGGCAACGGAGAGAGCGGCATGGGTC GTATCCCAGACTGGACCCCCCAGGCCTACGAC CCACTGGACGTGCTGGTGCCTTACTTCGTCCC CAACACCCCGGCAGCCCGAGCCGACCTGGCCG CTCAGTACACCACCGTAGGCCGCATGGACCAA GGAGTTGGACTGGTGCTCCAGGAGCTGCGTGA CGCCGGTGTCCTGAACGACACACTGGTGATCT TCACGTCCGACAACGGGATCCCCTTCCCCAGC GGCAGGACCAACCTGTACTGGCCGGGCACTGC TGAACCCTTACTGGTGTCATCCCCGGAGCACC CAAAACGCTGGGGCCAAGTCAGCGAGGCCTAC GTGAGCCTCCTAGACCTCACGCCCACCATCTT GGATTGGTTCTCGATCCCGTACCCCAGCTACG CCATCTTTGGCTCGAAGACCATCCACCTCACT GGCCGGTCCCTCCTGCCGGCGCTGGAGGCCGA GCCCCTCTGGGCCACCGTCTTTGGCAGCCAGA GCCACCACGAGGTCACCATGTCCTACCCCATG CGCTCCGTGCAGCACCGGCACTTCCGCCTCGT GCACAACCTCAACTTCAAGATGCCCTTTCCCA TCGACCAGGACTTCTACGTCTCACCCACCTTC CAGGACCTCCTGAACCGCACTACAGCTGGTCA GCCCACGGGCTGGTACAAGGACCTCCGTCATT ACTACTACCGGGCGCGCTGGGAGCTCTACGAC CGGAGCCGGGACCCCCACGAGACCCAGAACCT GGCCACCGACCCGCGCTTTGCTCAGCTTCTGG AGATGCTTCGGGACCAGCTGGCCAAGTGGCAG TGGGAGACCCACGACCCCTGGGTGTGCGCCCC CGACGGCGTCCTGGAGGAGAAGCTCTCTCCCC AGTGCCAGCCCCTCCACAATGAGCTGTAA | |
| HB1-SGSH amino acid sequence; | MQPSSLLPLALCLLAAPAGSSKPQALATPNKE EHGKRKKKGKGLGKKRDPCLRKYKDFCIHGEC KYVKELRAPSCICHPGYHGERCHGLSGSRPRN ALLLLADDGGFESGAYNNSAIATPHLDALARR SLLFRNAFTSVSSCSPSRASLLTGLPQHQNGM YGLHQDVHHFNSFDKVRSLPLLLSQAGVRTGI IGKKHVGPETVYPFDFAYTEENGSVLQVGRNI TRIKLLVRKFLQTQDDRPFFLYVAFHDPHRCG HSQPQYGTFCEKFGNGESGMGRIPDWTPQAYD PLDVLVPYFVPNTPAARADLAAQYTTVGRMDQ GVGLVLQELRDAGVLNDTLVIFTSDNGIPFPS GRTNLYWPGTAEPLLVSSPEHPKRWGQVSEAY VSLLDLTPTILDWFSIPYPSYAIFGSKTIHLT GRSLLPALEAEPLWATVFGSQSHHEVTMSYPM RSVQHRHFRLVHNLNFKMPFPIDQDFYVSPTF QDLLNRTTAGQPTGWYKDLRHYYYRARWELYD RSRDPHETQNLATDPRFAQLLEMLRDQLAKWQ WETHDPWVCAPDGVLEEKLSPQCQPLHNEL | SEQ ID NO.46 |
| HB2-SGSH cDNA: | ATGCAGCCCTCCAGCCTTCTGCCGCTCGCCCT CTGCCTGCTGGCTGCACCCGCCGGATCTGGGA AAAGAAAGAAGAAAGGCAAGGGGCTAGGGAAG AAGAGGGACCCATCTCTTCGGAAATACAAGGA CTTCTCCGGATCTCGTCCCCGGAACGCACTGC | SEQ ID NO.47 |
| | TGCTCCTCGCGGATGACGGAGGCTTTGAGAGT GGCGCGTACAACAACAGCGCCATCGCCACCCC GCACCTGGACGCCTTGGCCCGCCGCAGCCTCC TCTTTCGCAATGCCTTCACCTCGGTCAGCAGC TGCTCTCCCAGCCGCGCCAGCCTCCTCACTGG CCTGCCCCAGCATCAGAATGGATGTACGGGC TGCACCAGGACGTGCACCACTTCAACTCCTTC GACAAGGTGCGGAGCCTGCCGCTGCTGCTCAG CCAAGCTGGTGTGCGCACAGGCATCATCGGGA AGAAGCACGTGGGGCCGGAGACCGTGTACCCG TTTGACTTTGCGTACACGGAGGAGAATGGCTC CGTCCTCCAGGTGGGGCGGAACATCACTAGAA TTAAGCTGCTCGTCCGGAAATTCCTGCAGACT CAGGATGACCGGCCTTTCTTCCTCTACGTCGC CTTCCACGACCCCCACCGCTGTGGGCACTCCC AGCCCCAGTACGGAACCTTCTGTGAGAAGTTT GGCAACGGAGAGAGCGGCATGGGTCGTATCCC AGACTGGACCCCCCAGGCCTACGACCCACTGG ACGTGCTGGTGCCTTACTTCGTCCCCAACACC CCGGCAGCCCGAGCCGACCTGGCCGCTCAGTA CACCACCGTAGGCCGCATGGACCAAGGAGTTG GACTGGTGCTCCAGGAGCTGCGTGACGCCGGT GTCCTGAACGACACACTGGTGATCTTCACGTC CGACAACGGGATCCCCTTCCCCAGCGGCAGGA CCAACCTGTACTGGCCGGGCACTGCTGAACCC TTACTGGTGTCATCCCCGGAGCACCCAAAACG CTGGGGCCAAGTCAGCGAGGCCTACGTGAGCC TCCTAGACCTCACGCCCACCATCTTGGATTGG TTCTCGATCCCGTACCCCAGCTACGCCATCTT TGGCTCGAAGACCATCCACCTCACTGGCCGGT CCCTCCTGCCGGCGCTGGAGGCCGAGCCCCTC TGGGCCACCGTCTTTGGCAGCCAGAGCCACCA CGAGGTCACCATGTCCTACCCCATGCGCTCCG TGCAGCACCGGCACTTCCGCCTCGTGCACAAC CTCAACTTCAAGATGCCCTTTCCCATCGACCA GGACTTCTACGTCTCACCCACCTTCCAGGACC TCCTGAACCGCACTACAGCTGGTCAGCCCACG GGCTGGTACAAGGACCTCCGTCATTACTACTA CCGGGCGCGCTGGGAGCTCTACGACCGGAGCC GGGACCCCCACGAGACCCAGAACCTGGCCACC GACCCGCGCTTTGCTCAGCTTCTGGAGATGCT TCGGGACCAGCTGGCCAAGTGGCAGTGGGAGA CCCACGACCCCTGGGTGTGCGCCCCCGACGGC GTCCTGGAGGAGAAGCTCTCTCCCCAGTGCCA GCCCCTCCACAATGAGCTGTAA | |
| HB2-SGSH amino acid sequence; | MQPSSLLPLALCLLAAPAGSGKRKKKGKGLGK KRDPSLRKYKDFSGSRPRNALLLLADDGGFES GAYNNSAIATPHLDALARRSLLFRNAFTSVSS CSPSRASLLTGLPQHQNGMYGLHQDVHHFNSF DKVRSLPLLLSQAGVRTGIIGKKHVGPETVYP FDFAYTEENGSVLQVGRNITRIKLLVRKFLQT QDDRPFFLYVAFHDPHRCGHSQPQYGTFCEKF GNGESGMGRIPDWTPQAYDPLDVLVPYFVPNT PAARADLAAQYTTVGRMDQGVGLVLQELRDAG VLNDTLVIFTSDNGIPFPSGRTNLYWPGTAEP LLVSSPEHPKRWGQVSEAYVSLLDLTPTILDW | SEQ ID NO.48 |
| | FSIPYPSYAIFGSKTIHLTGRSLLPALEAEPL WATVFGSQSHHEVTMSYPMRSVQHRHFRLVHN LNFKMPFPIDQDFYVSPTFQDLLNRTTAGQPT GWYKDLRHYYYRARWELYDRSRDPHETQNLAT DPRFAQLLEMLRDQLAKWQWETHDPWVCAPDG VLEEKLSPQCQPLHNEL | |

Combined N-terminal and C-terminal heparin/heparan sulfate binding tags were constructed correspondingly. The combined N-terminal and C-terminal tag is demonstrated for human sulfamidase (SGSH) (Table 6).

**Table 6 - HB2/SGSH/Antp**

| | | |
|---|---|---|
| HB2-SGSH-Antp cDNA; | ATGCAGCCCTCCAGCCTTCTGCCGCTCGCCCT CTGCCTGCTGGCTGCACCCGCCGGATCTGGGA AAAGAAAGAAGAAAGGCAAGGGGCTAGGGAAG AAGAGGGACCCATCTCTTCGGAAATACAAGGA CTTCTCCGGATCTCGTCCCCGGAACGCACTGC TGCTCCTCGCGGATGACGGAGGCTTTGAGAGT GGCGCGTACAACAACAGCGCCATCGCCACCCC GCACCTGGACGCCTTGGCCCGCCGCAGCCTCC TCTTTCGCAATGCCTTCACCTCGGTCAGCAGC TGCTCTCCCAGCCGCGCCAGCCTCCTCACTGG CCTGCCCCAGCATCAGAATGGGATGTACGGGC TGCACCAGGACGTGCACCACTTCAACTCCTTC GACAAGGTGCGGAGCCTGCCGCTGCTGCTCAG CCAAGCTGGTGTGCGCACAGGCATCATCGGGA AGAAGCACGTGGGGCCGGAGACCGTGTACCCG TTTGACTTTGCGTACACGGAGGAGAATGGCTC CGTCCTCCAGGTGGGGCGGAACATCACTAGAA TTAAGCTGCTCGTCCGGAAATTCCTGCAGACT CAGGATGACCGGCCTTTCTTCCTCTACGTCGC CTTCCACGACCCCCACCGCTGTGGGCACTCCC AGCCCCAGTACGGAACCTTCTGTGAGAAGTTT GGCAACGGAGAGAGCGGCATGGGTCGTATCCC AGACTGGACCCCCCAGGCCTACGACCCACTGG ACGTGCTGGTGCCTTACTTCGTCCCCAACACC CCGGCAGCCCGAGCCGACCTGGCCGCTCAGTA CACCACCGTAGGCCGCATGGACCAAGGAGTTG GACTGGTGCTCCAGGAGCTGCGTGACGCCGGT GTCCTGAACGACACACTGGTGATCTTCACGTC CGACAACGGGATCCCCTTCCCCAGCGGCAGGA CCAACCTGTACTGGCCGGGCACTGCTGAACCC TTACTGGTGTCATCCCCGGAGCACCCAAAACG CTGGGGCCAAGTCAGCGAGGCCTACGTGAGCC TCCTAGACCTCACGCCCACCATCTTGGATTGG TTCTCGATCCCGTACCCCAGCTACGCCATCTT TGGCTCGAAGACCATCCACCTCACTGGCCGGT CCCTCCTGCCGGCGCTGGAGGCCGAGCCCCTC TGGGCCACCGTCTTTGGCAGCCAGAGCCACCA | SEQ ID NO.49 |
| | CGAGGTCACCATGTCCTACCCCATGCGCTCCG TGCAGCACCGGCACTTCCGCCTCGTGCACAAC CTCAACTTCAAGATGCCCTTTCCCATCGACCA GGACTTCTACGTCTCACCCACCTTCCAGGACC TCCTGAACCGCACTACAGCTGGTCAGCCCACG GGCTGGTACAAGGACCTCCGTCATTACTACTA CCGGGCGCGCTGGGAGCTCTACGACCGGAGCC GGGACCCCCACGAGACCCAGAACCTGGCCACC GACCCGCGCTTTGCTCAGCTTCTGGAGATGCT TCGGGACCAGCTGGCCAAGTGGCAGTGGGAGA CCCACGACCCCTGGGTGTGCGCCCCCGACGGC GTCCTGGAGGAGAAGCTCTCTCCCCAGTGCCA GCCCCTCCACAATGAGCTGAGATCCCCCGGGC GCCAGATAAAGATTTGGTTCCAGAATCGGCGC ATGAAGTGGAAGAAGTAA | |
| HB2-SGSH-Antp amino acid sequence | MQPSSLLPLALCLLAAPAGSGKRKKKGKGLGK KRDPSLRKYKDFSGSRPRNALLLLADDGGFES GAYNNSAIATPHLDALARRSLLFRNAFTSVSS CSPSRASLLTGLPQHQNGMYGLHQDVHHFNSF DKVRSLPLLLSQAGVRTGIIGKKHVGPETVYP FDFAYTEENGSVLQVGRNITRIKLLVRKFLQT QDDRPFFLYVAFHDPHRCGHSQPQYGTFCEKF GNGESGMGRIPDWTPQAYDPLDVLVPYFVPNT PAARADLAAQYTTVGRMDQGVGLVLQELRDAG VLNDTLVIFTSDNGIPFPSGRTNLYWPGTAEP LLVSSPEHPKRWGQVSEAYVSLLDLTPTILDW FSIPYPSYAIFGSKTIHLTGRSLLPALEAEPL WATVFGSQSHHEVTMSYPMRSVQHRHFRLVHN LNFKMPFPIDQDFYVSPTFQDLLNRTTAGQPT GWYKDLRHYYYRARWELYDRSRDPHETQNLAT DPRFAQLLEMLRDQLAKWQWETHDPWVCAPDG VLEEKLSPQCQPLHNELRSPGRQIKIWFQNRR MKWKK | SEQ ID NO.50 |

The following lysosomal proteins were fused to the above described heparin/heparin sulfate binding tags (Table 7).

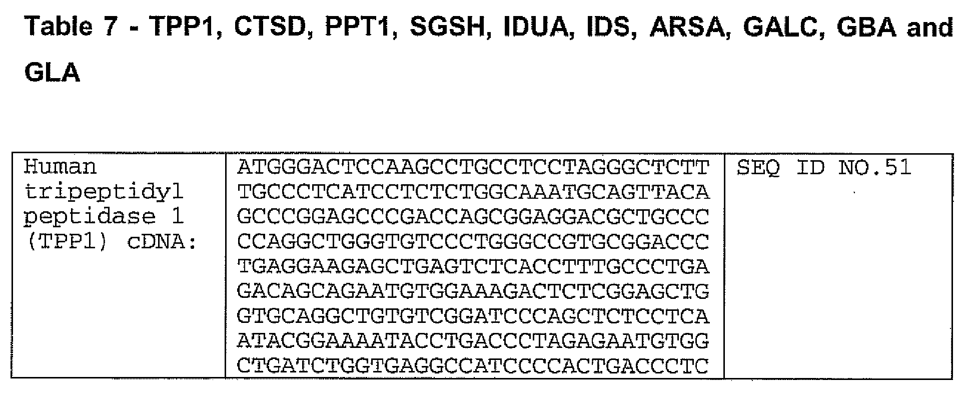

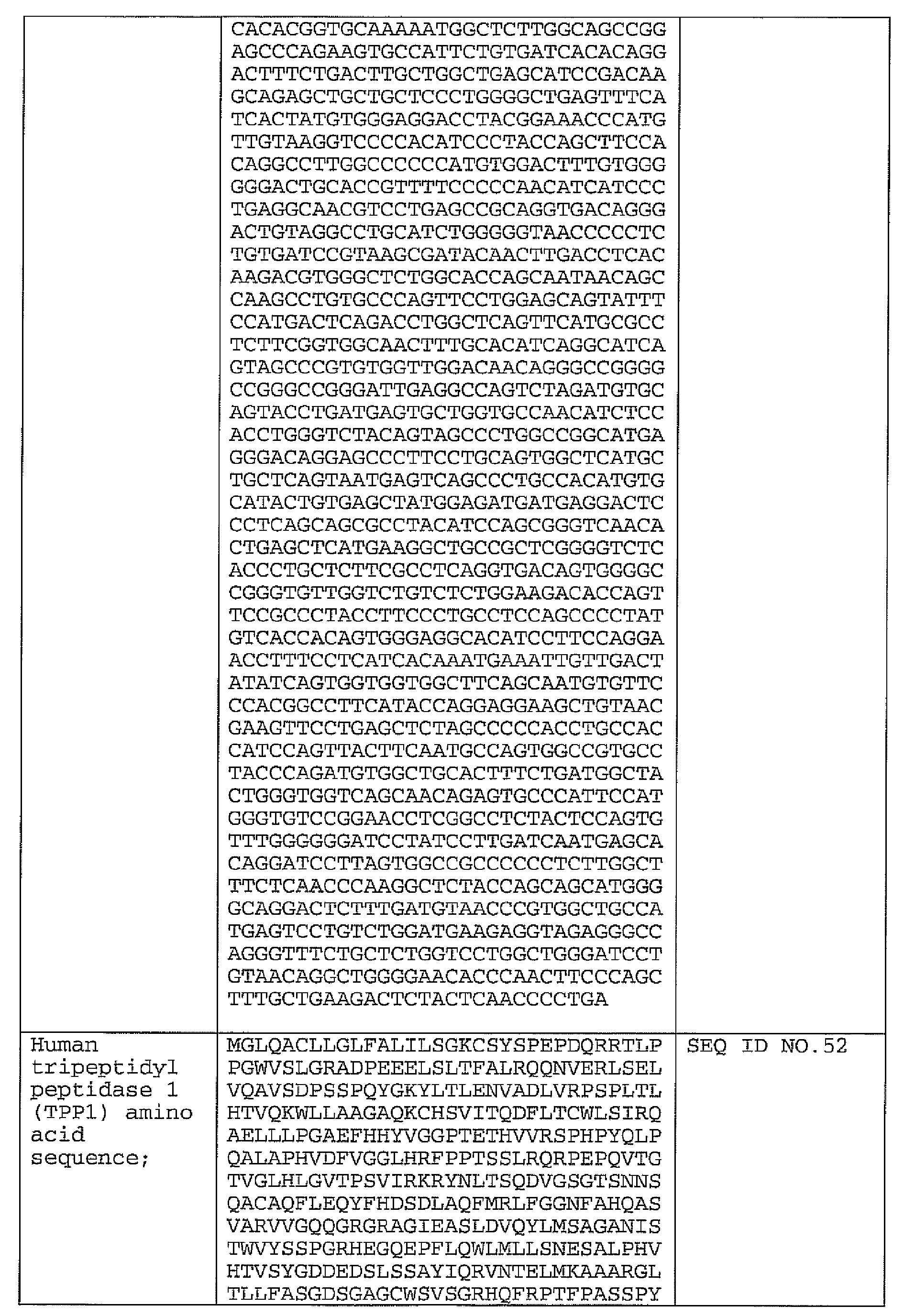

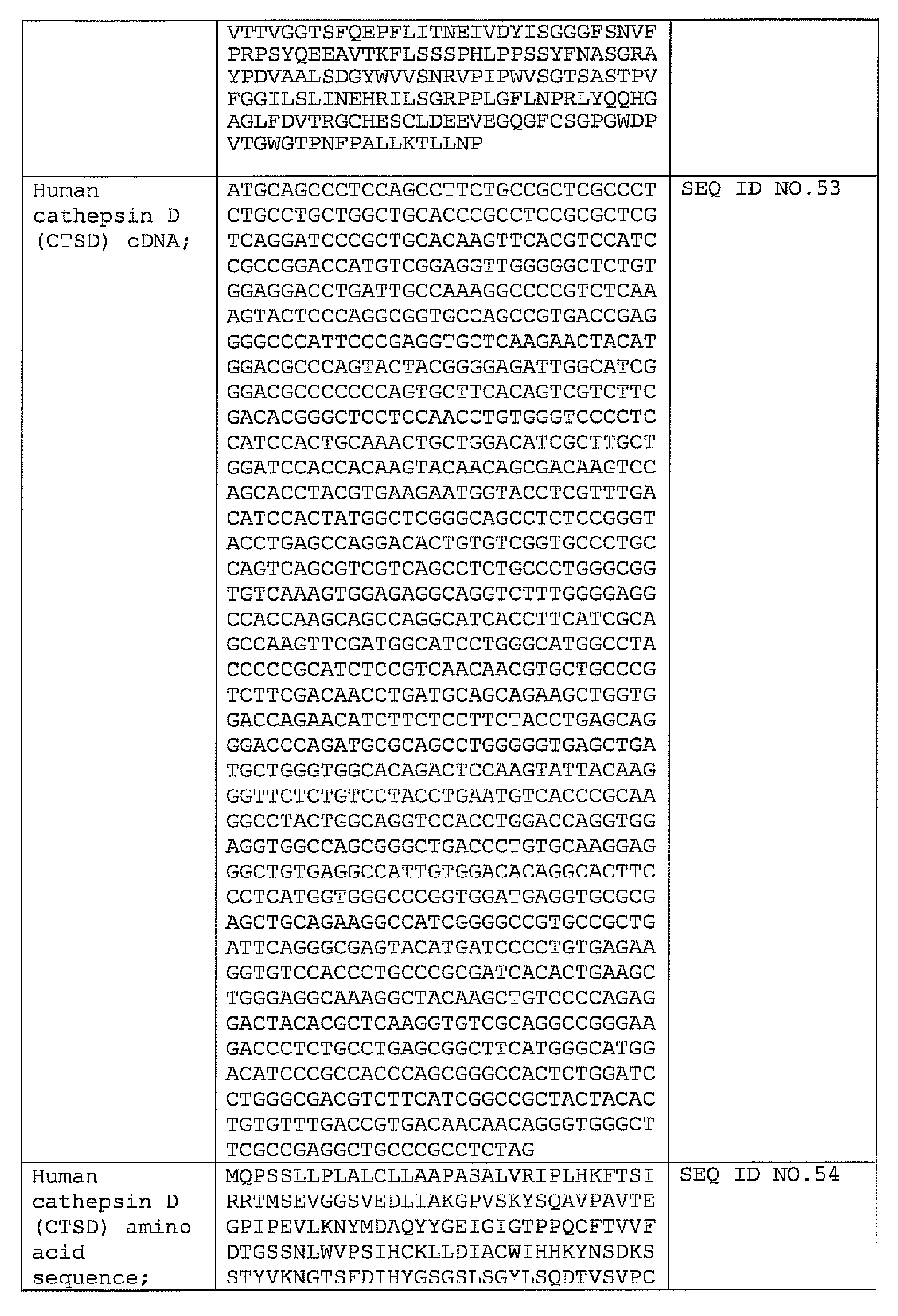

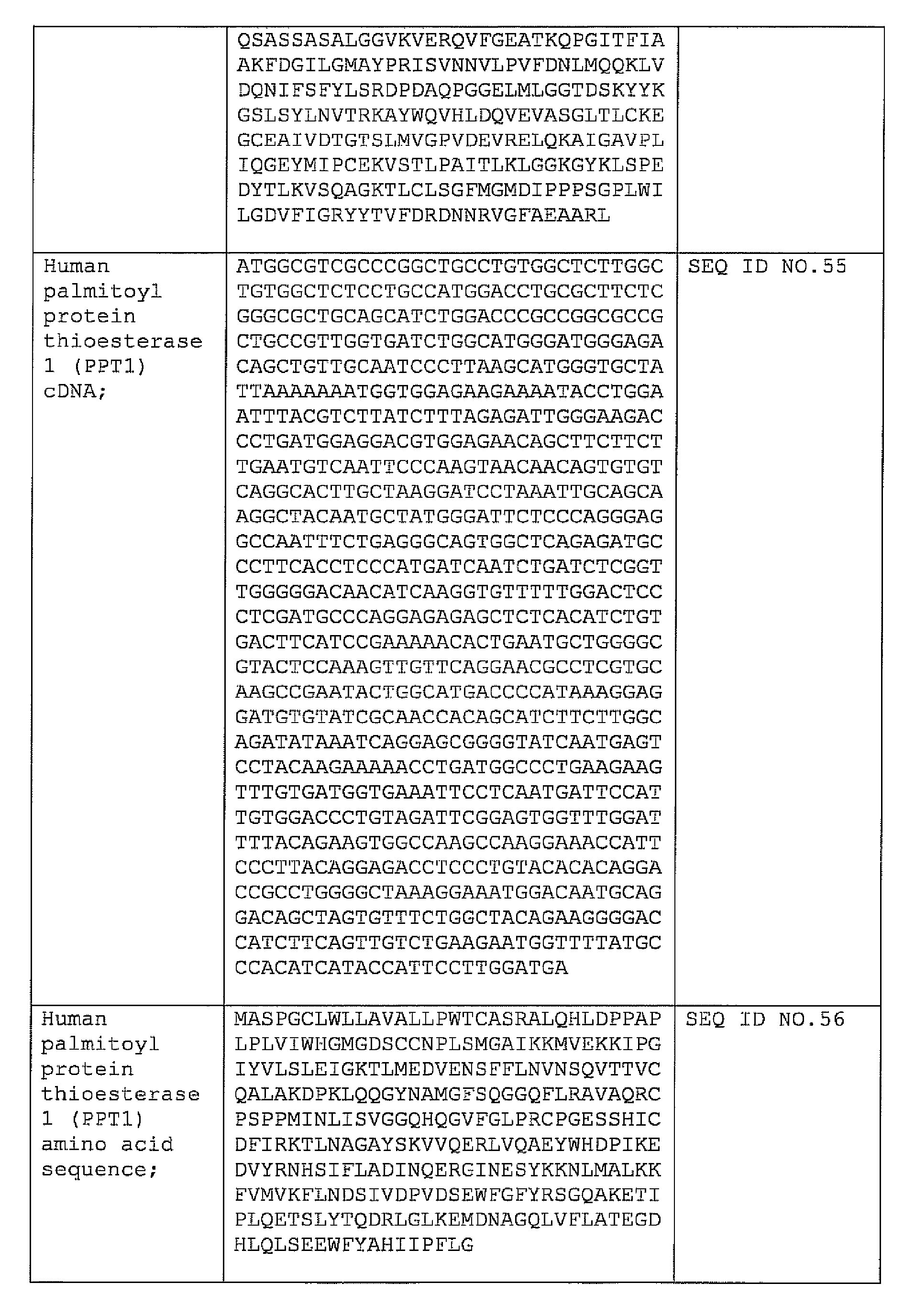

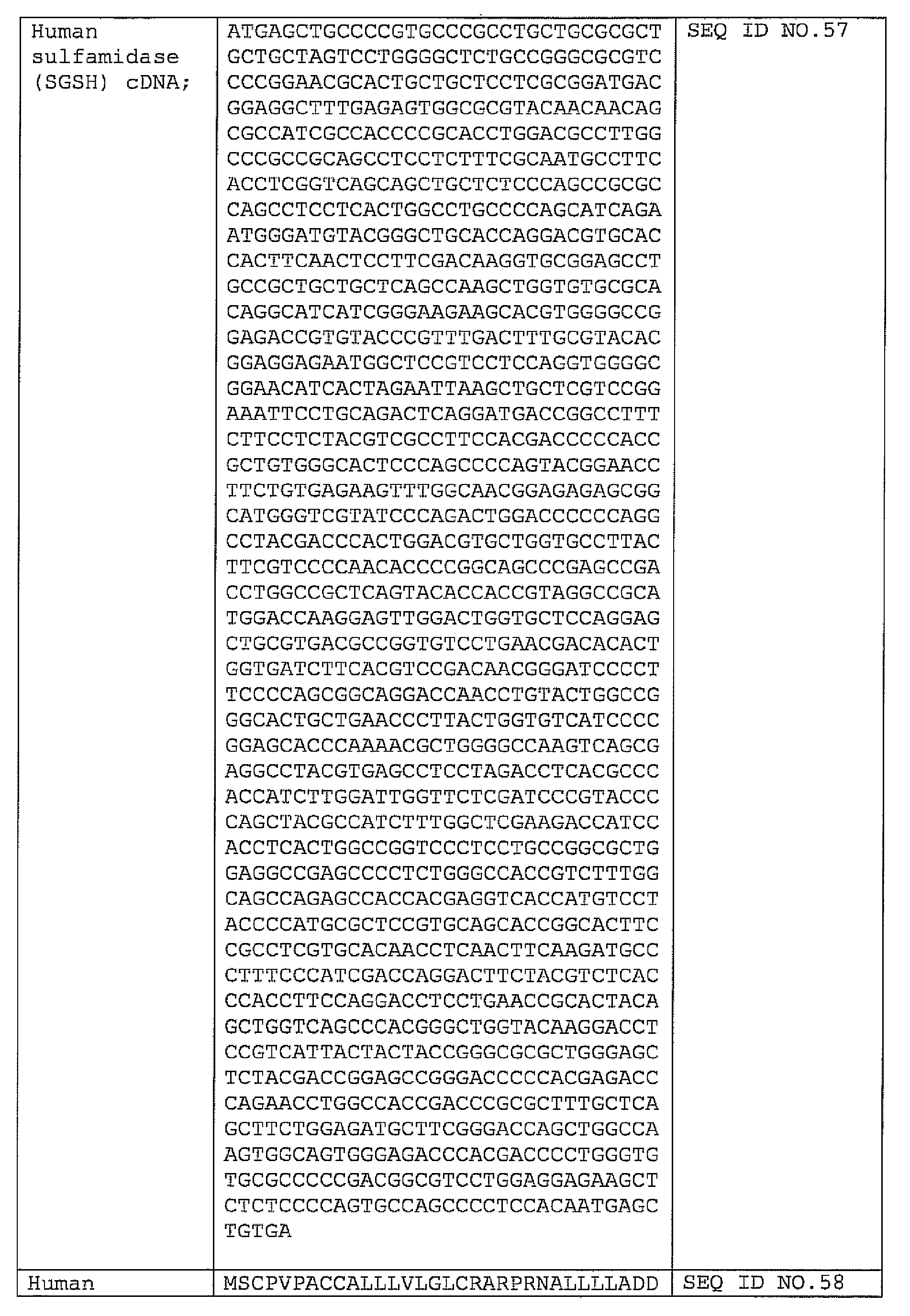

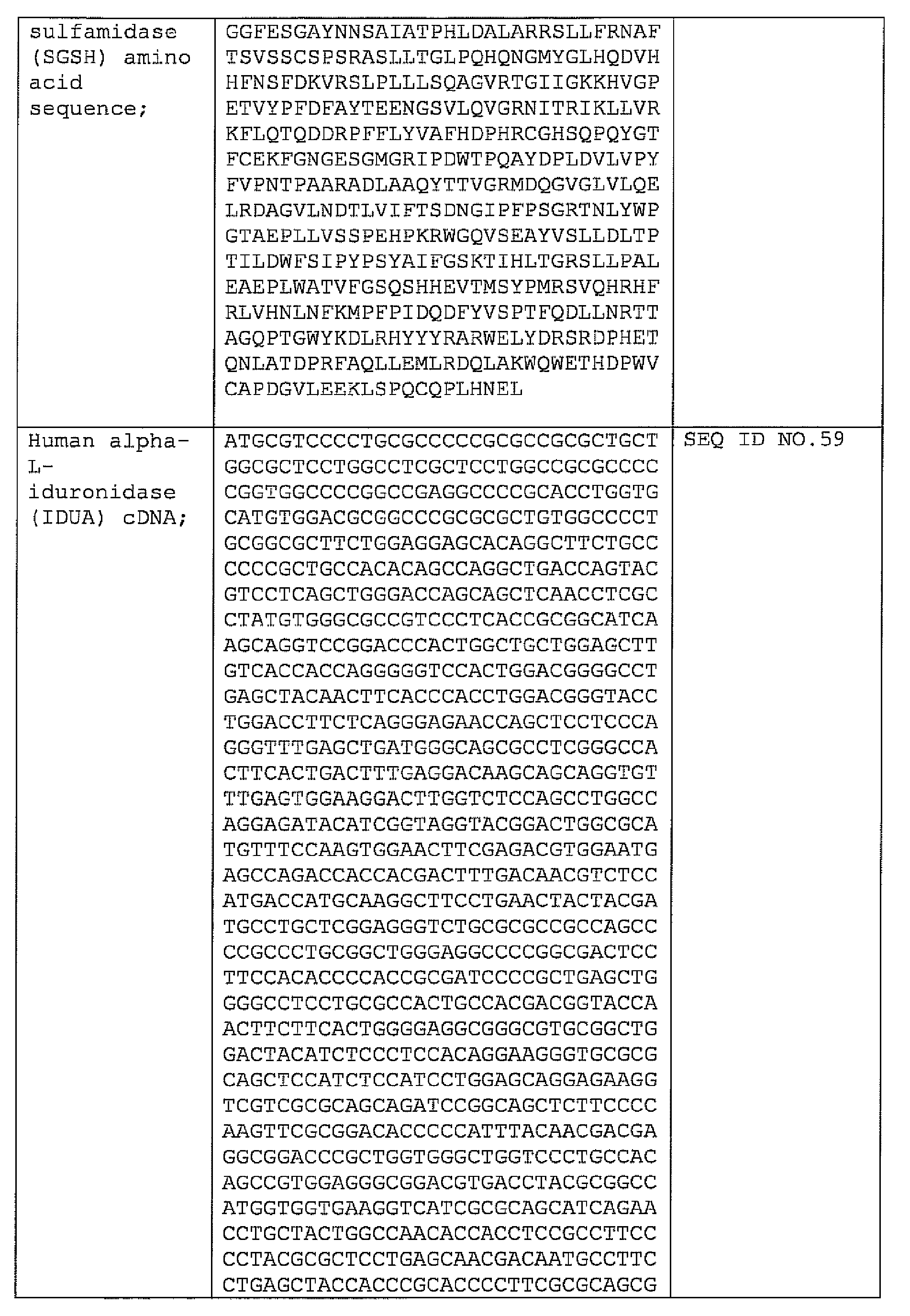

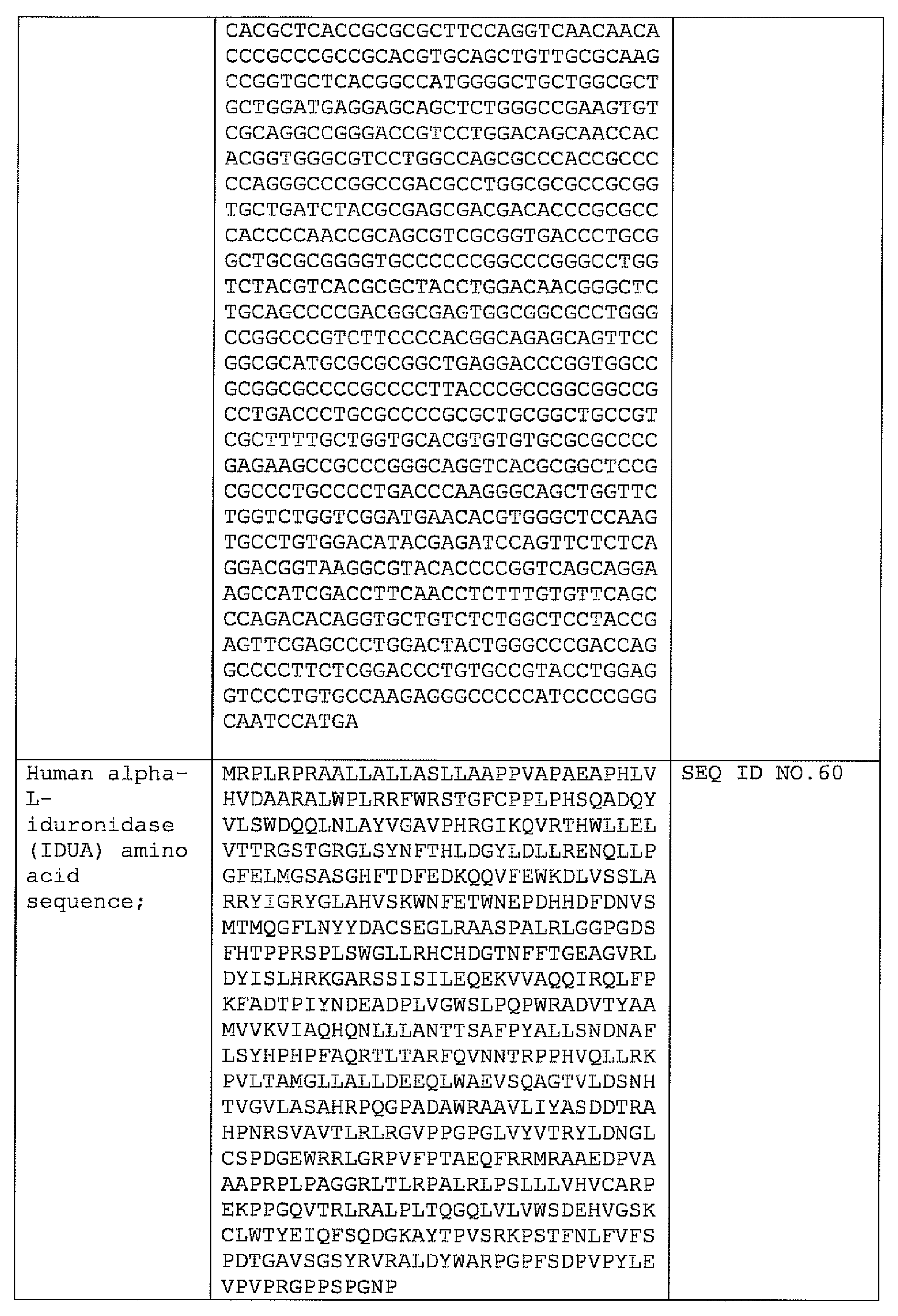

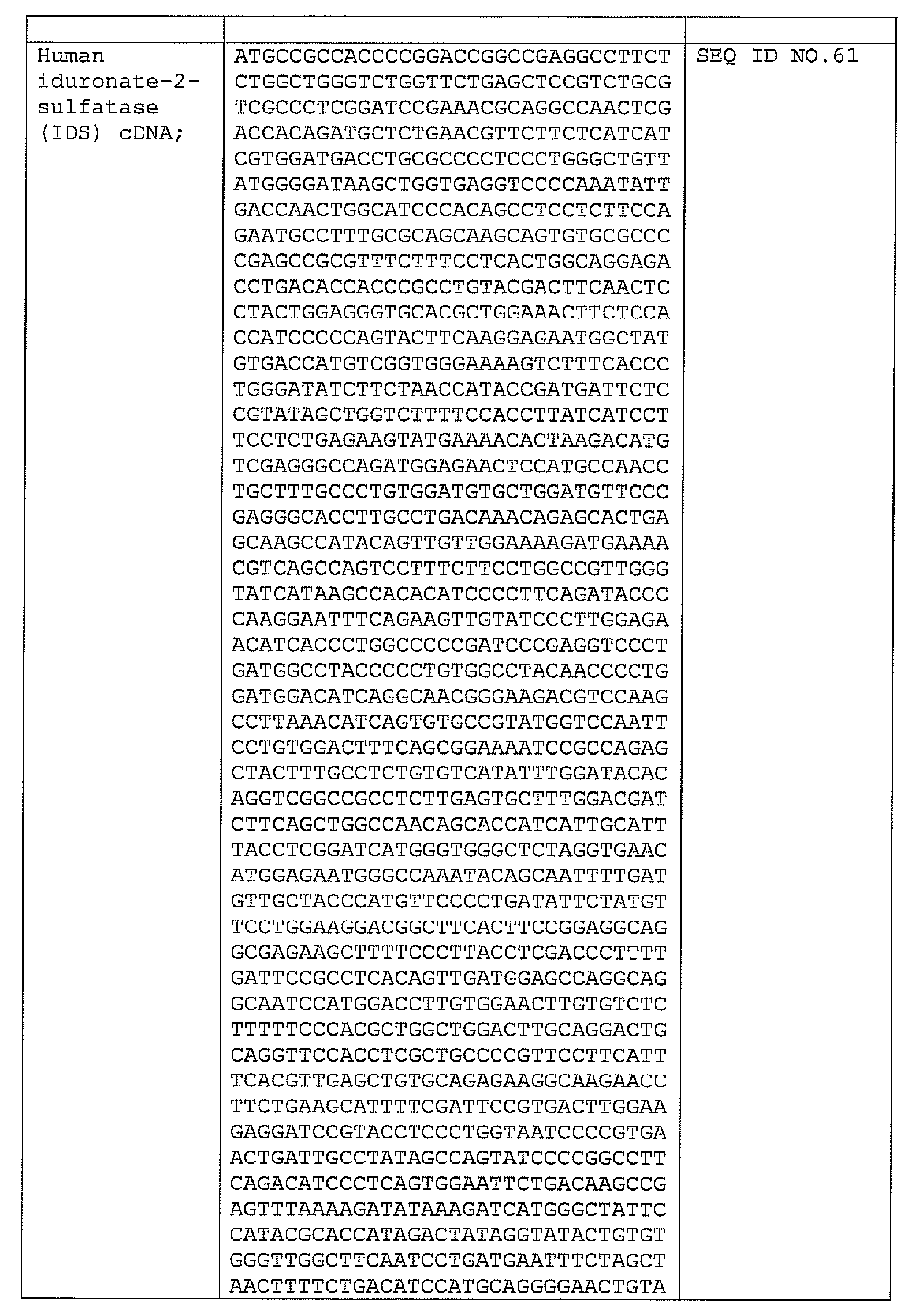

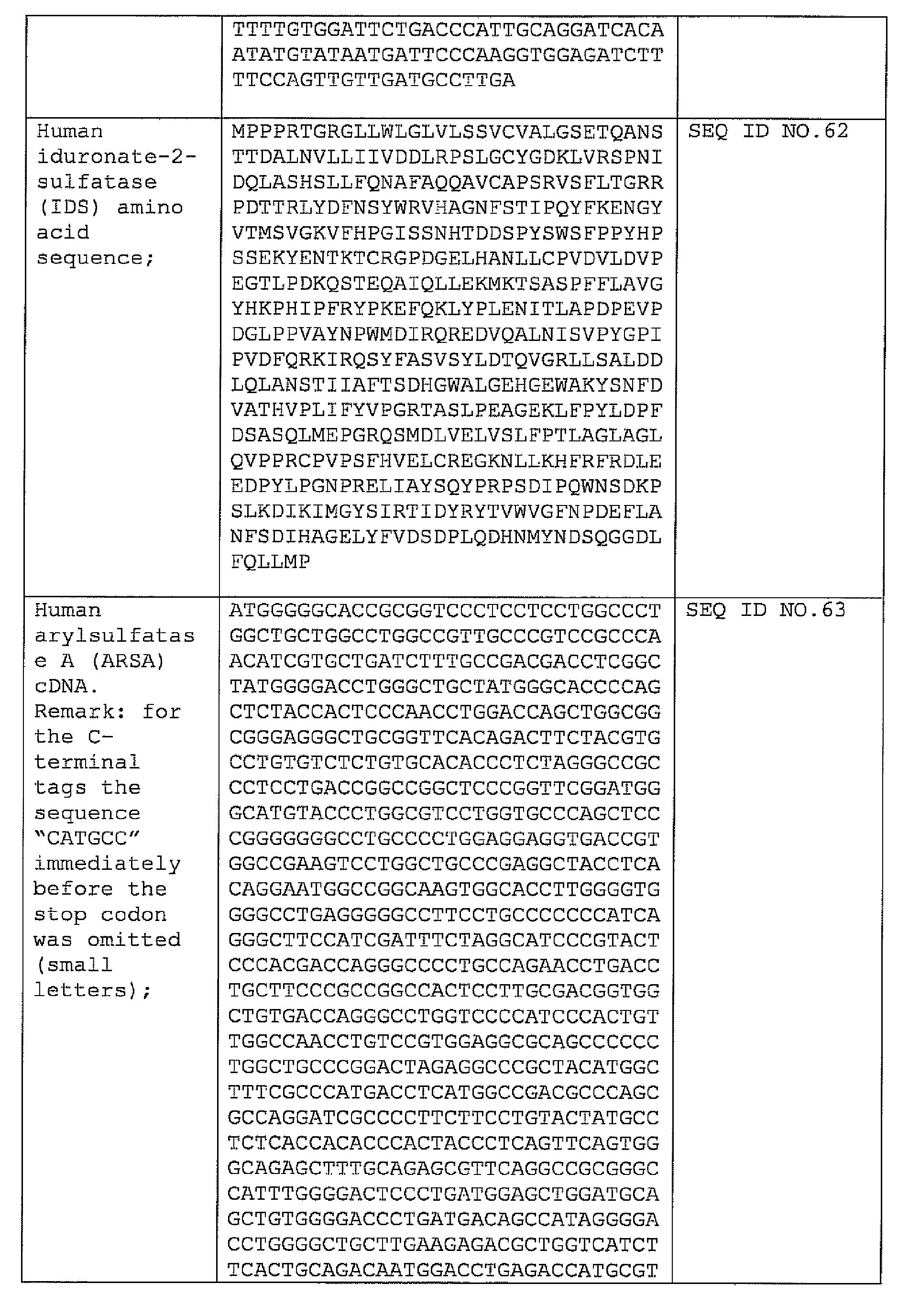

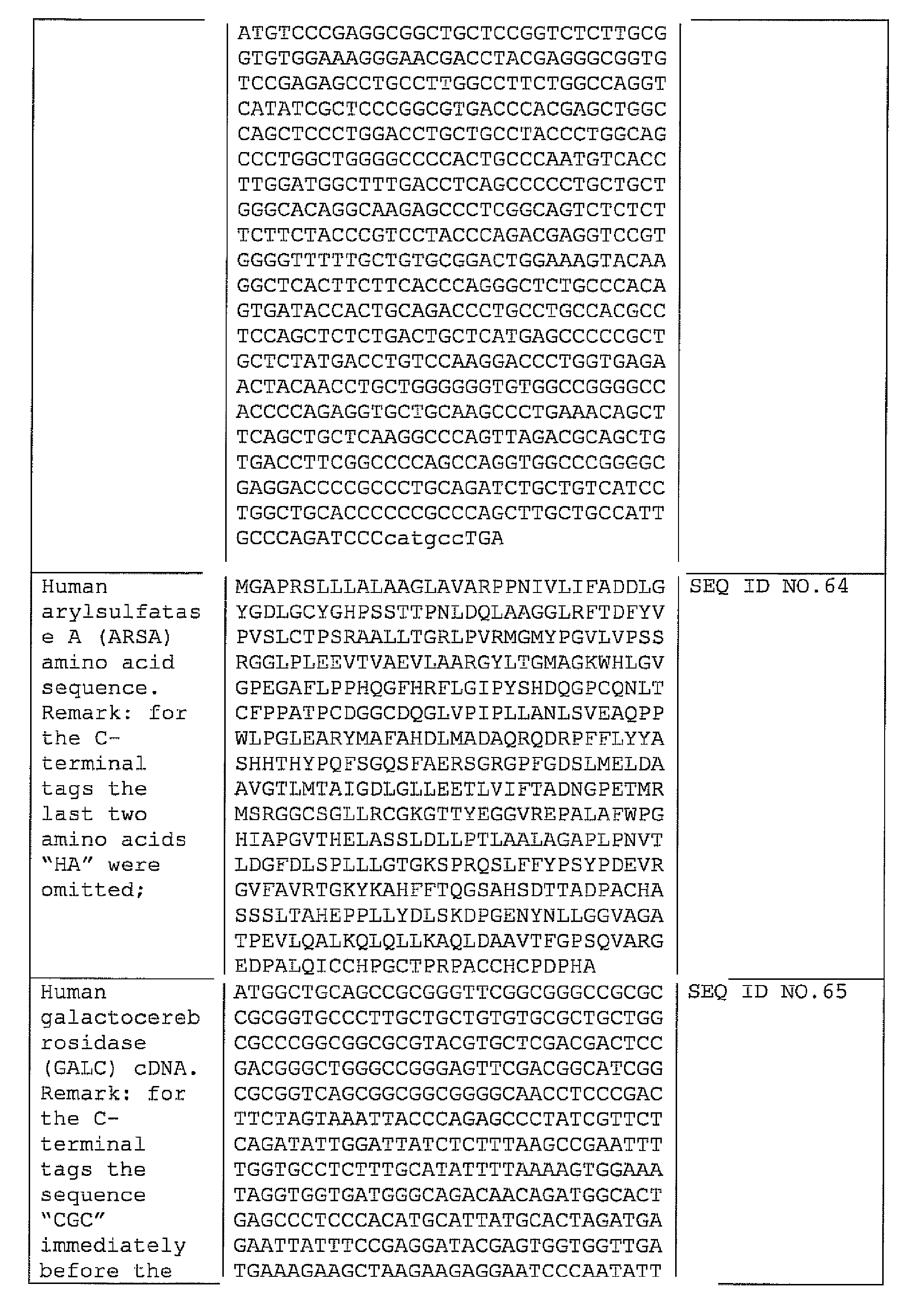

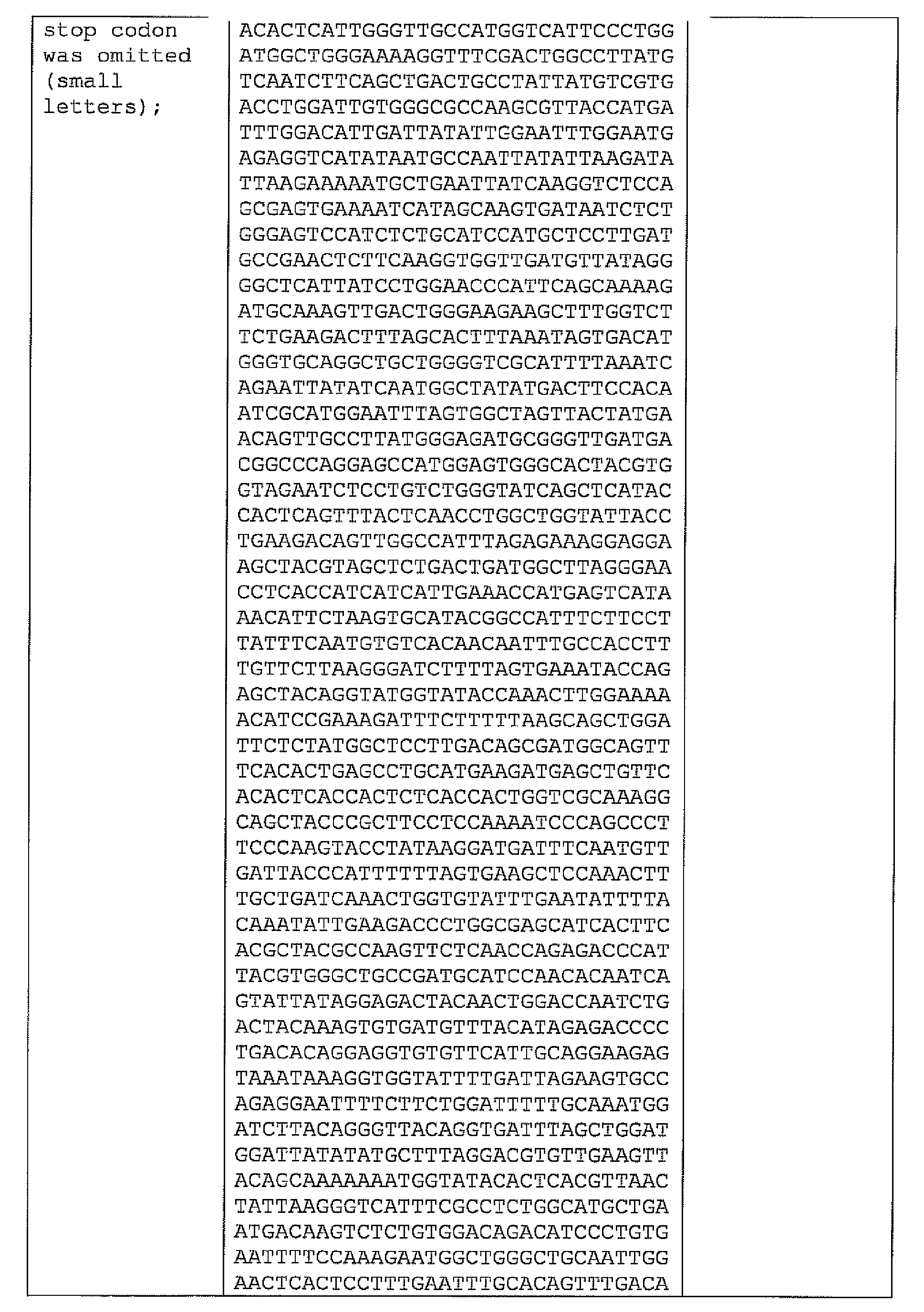

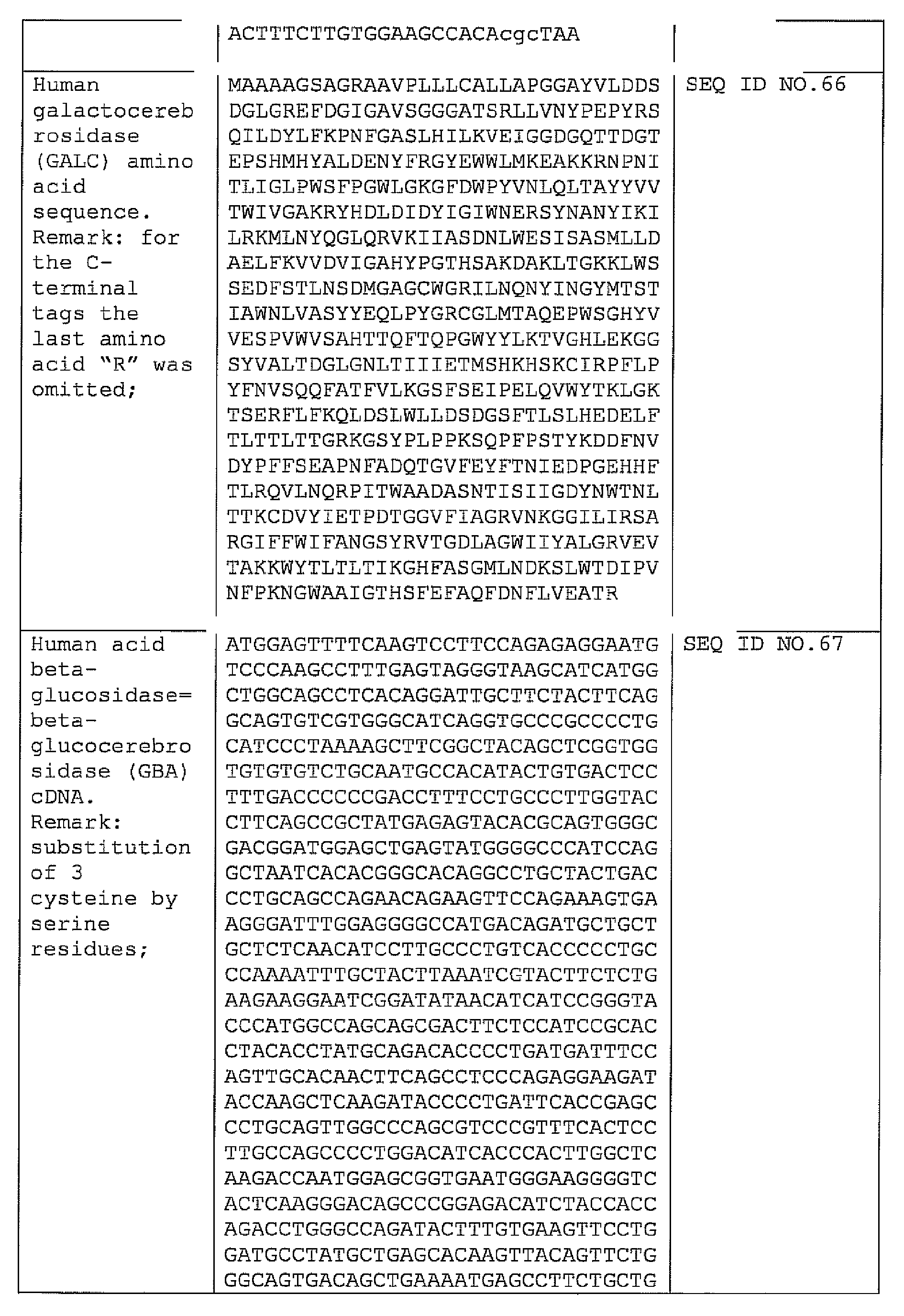

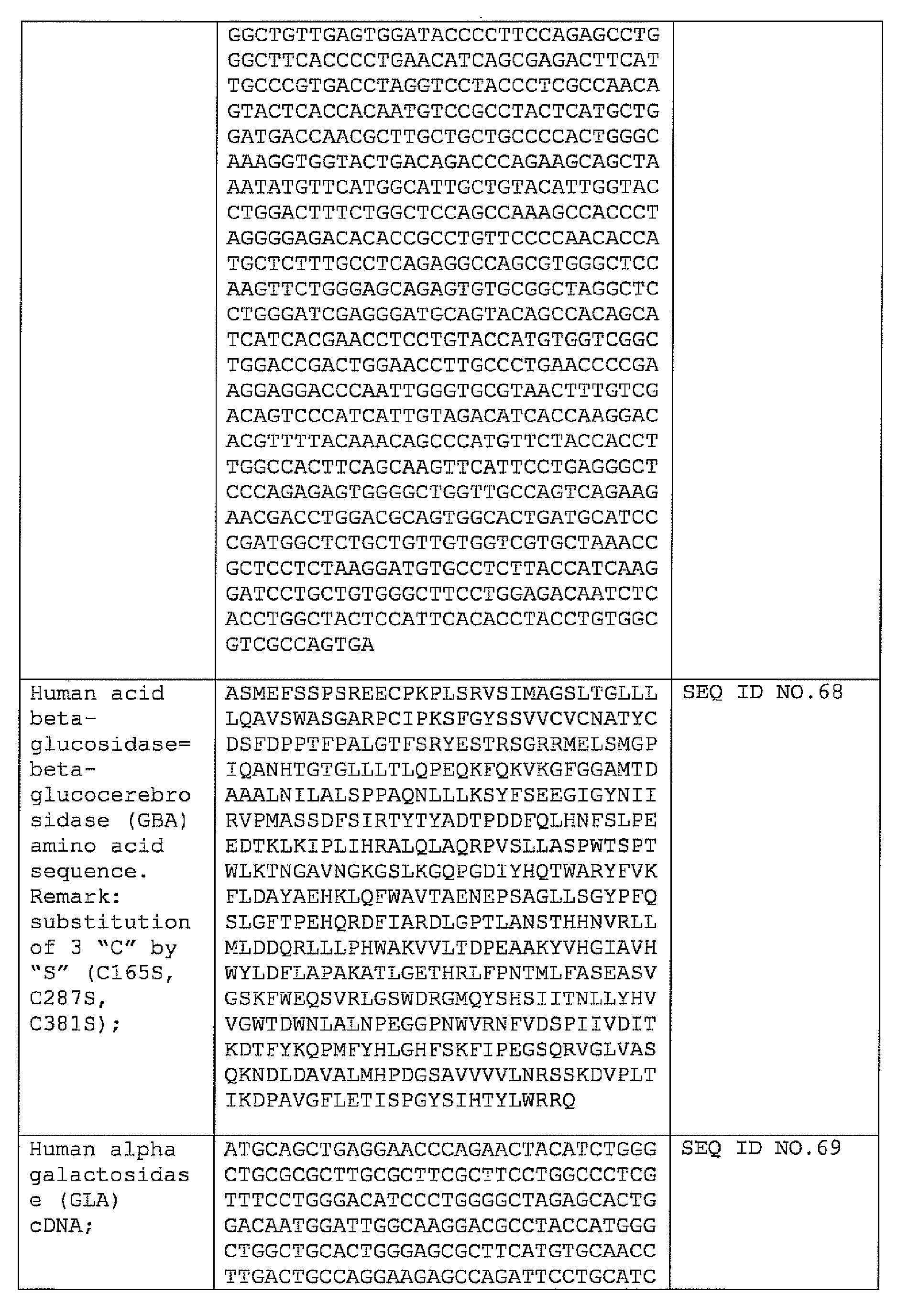

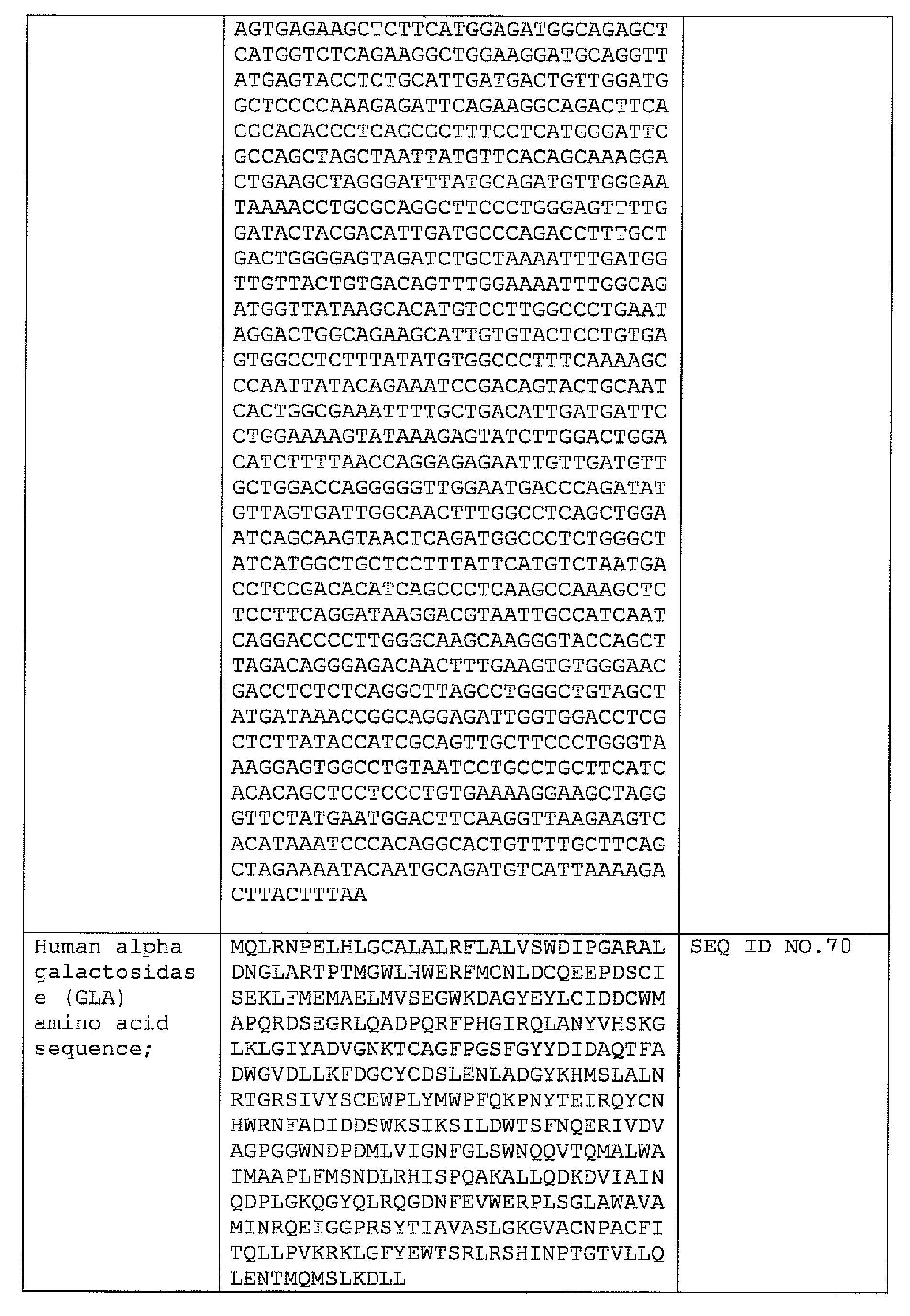

In a preferred embodiment the targeting moiety is selected from the group of Antp, CLOCK, FGF2, HB1 and HB2 including the variants as outlined above.

Preferably the targeting moiety and the enzyme moiety are linked via a peptide linker as encoded by one of the following sequences SEQ ID NO. 15 or 16. SEQ ID NO. 21 or 22 are preferably N-terminal to FGF2 variants. SEQ ID NO. 43 or 44 are preferably N-terminal to the lysosomal proteins.

In one embodiment the peptide linker comprises a protease cleavage site. Such a site may be a site recognized by factor Xa, a caspase, thrombin, trypsin, papain and plasmin. For FGF2 variant constructs this is preferred.

In a preferred embodiment the lysosomal enzyme is selected from the group consisting of β-galactocerebrosidase, arylsulfatase A (sulfatidase), α-iduronidase, sulfaminidase, α-N-acetylglucosaminidase, acetyl-CoA:a-glucosaminide-N-Ac-transferase, N-acetylglucosamine-6-sulfatase, tripeptidyl-peptidase 1, palmitoyl-protein thioesterase, β-galactosidase, sphingomyelinase, β-hexosaminidase A, β-hexosaminidase B, ceramidase, α-mannosidase, β-mannosidase, α-fucosidase, sialidase, α-N-acetylgalactosaminidase, α-L-iduronidase, iduronate-2-sulfatase, sulfamidase (heparan N-sulftase), α-N-acetylglucosaminidase, N-acetylgalactosamin-6-sulfatase, arylsulfatase B, β-glucuronidase, α-L-fucosidase, aspartylglucosaminidase, α-neuraminidase (sialidase), cathepsin A, β-hexosaminidase A + B, arylsulfatase A, cerebroside-β-galactosidase, β-glucocerebrosidase, α-galactosidase A (ceramide trihexosidase), acid α-glucosidase (acid maltase), CLN5-protein, acid lipase, steroid sulfatase (arylsulfatase C) and cathepsin D.

In a very preferred embodiment the lysosomal enzyme is selected from the group consisting of tripeptidyl-peptidase I (TPP1), Human cathepsin D (CTSD), Human palmitoyl protein thioesterase 1 (PPT1), Human sulfamidase (SGSH), Human alpha-L-iduronidase (IDUA), Human iduronate-2-sulfatase (IDS), Human arylsulfatase A (ARSA), Human acid beta-glucosidase=beta-glucocerebrosidase (GBA) and Human alpha galactosidase (GLA).

Preferably the targeting moiety is a polypeptide having a sequence according to any one of SEQ ID NO. 18, 20, 24, 26, 27, 30, 71, 72 or as encoded by 43, and 44 or the other nucleic acid sequences encoding the respective peptides.

Polypeptides with reduced nuclear translocation are preferred, such as SEQ ID NO. 28.

Polypeptides with reduced FGF receptor binding are preferred, such as SEQ ID NO. 26.

Also polypeptides with both above mentioned activity reductions are preferred such as SEQ ID NO. 30.

Preferably the enzyme moiety is a polypeptide having a sequence according to any one of SEQ ID NO. 52, 54, 56, 58, 60, 62, 64, 66, 68 and 70.

Preferably chimeric molecule polypeptide has a sequence according to any one of the SEQ ID NO. 32, 34, 36, 38 40, 42, 46, 48 and 50.

The present invention also relates to sequence variants, allelic variants or mutants of the chimeric molecule described herein, and nucleic acid sequences that encode them. Sequence variants of the invention preferably share at least 90%, 91%, 92%, 93% or 94% identity with a polypeptide of the invention or with a nucleic acid sequence that encodes it. More preferably, a sequence variant shares at least 95%, 96%, 97% or 98% identity at the amino acid or nucleic acid level. Most preferably, a sequence variant shares at least 99%, 99.5%, 99.9% or more identity with a polypeptide of the invention or a nucleic acid sequence that encodes it.

Accordingly, the present invention provides an isolated chimeric protein comprising a sequence that is at least 80%, 85%, 90%, 92%, 94%, 95%, 96%, 97%, 98%, 99%, 99.5%, 99.9% or 100% identical to the sequences outlined above.

The chimeric molecules may be pegylated. The term "pegylation," "polyethylene glycol" or "PEG" includes a polyalkylene glycol compound or a derivative thereof, with or without coupling agents or derivatization with coupling or activating moieties (e.g., with thiol, triflate, tresylate, azirdine, oxirane, or preferably with a maleimide moiety, e.g., PEG-maleimide). Other appropriate polyalkylene glycol compounds include, but are not limited to, maleimido monomethoxy PEG, activated PEG polypropylene glycol, but also charged or neutral polymers of the following types: dextran, colominic acids, or other carbohydrate based polymers, polymers of amino acids, and biotin and other affinity reagent derivatives.

The invention also relates to a polynucleotide encoding the chimeric molecule according to the invention.

Preferred polynucleotides according to the invention are selected from the group of the SEQ ID NO. 31, 33, 35, 37, 39, 41, 45, 47 and 49.

The nucleic acid may differ from the sequence outlined above, in particular due to the degeneracy of the genetic code.

The invention also relates to a pharmaceutical composition comprising a chimeric molecule according to the invention.

The invention relates to the chimeric molecule according to the invention for the use in the treatment of a disease.

The disease is preferably a lysosomal storage disease.

Preferably the lysosomal storage disease is selected from the group consisting of the late infantile form of neuronal ceroid lipofuscinosis (NCL),mucopolysaccharidosis type I, mucopolysaccharidosis type II, mucopolysaccharidosis type IIIA, mucopolysaccharidosis type IIIB, mucopolysaccharidosis type IIIC, mucopolysaccharidosis type IIID, mucopolysaccharidosis type IVA, mucopolysaccharidosis type IVB, mucopolysaccharidosis type VI, mucopolysaccharidosis type VII, fucosidosis, α-mannosidosis, β-mannosidosis, aspartylglucosaminuria, Schindler's disease, Sialidosis (Mucolipidosis I), galaktosialidosis, GM₁-gangliosidosis /MPS IVB, GM₂-gangliosidosis, Sandhoff disease, Tay-Sachs disease, metachromatic leukodystrophy, Krabbe disease, Gaucher disease, Fabry disease, Niemann-Pick disease type I (=A+B), glycogen storage disease type II ( Pompe disease), infantile NCL (CLN1-defect), late infantile NCL (CLN2-defect), late infantile NCL (CLN5-defect), NCL caused by cathepsin D deficiency (CLN10-defect), Faber's syndrome, Wolman disease, X-linked ichthyosis.

In one embodiment the lysosomal storage disease is the late infantile form of neuronal ceroid lipofuscinosis and the enzyme moiety comprises lysosomal tripeptidyl peptidase 1 (TPP1).

Combinations of disease names and enzyme defects are given in table 8 below.

**Table 8**

| **DISEASE** | **ENZYME/PROTEIN DEFECT** |
|---|---|
| Mucopolysaccharidosis Type I | α-L-Iduronidase |
| Mucopolysaccharidosis Type II | Iduronat-2-Sulfatase |
| Mucopolysaccharidosis Type IIIA | Sulfamidase (Heparan N-Sulfatase) |
| Mucopolysaccharidosis Type IIIB | α-N-Acetylglucosaminidase |
| Mucopolysaccharidosis Type IIIC | Glucosamin-N-Acetyltransferase |
| Mucopolysaccharidosis Type IIID | N-Acetylglucosamin-6-Sulfatase |
| Mucopolysaccharidosis Type IVA | N-Acetylgalactosamin-6-Sulfatase |
| Mucopolysaccharidosis Type IVB | β-Galactosidase |
| Mucopolysaccharidosis Type VI | Arylsulfatase B |
| Mucopolysaccharidosis Type VII | β-Glucuronidase |
| Fucosidosis | α-L-Fucosidase |
| α-Mannosidosis | α-Mannosidase |
| β-Mannosidosis | β-Mannosidase |
| Aspartylglucosaminuria | Aspartylglucosaminidase |
| M. Schindler | α-N-Acetylgalactosaminidase |
| Sialidosis (Mucolipidosis Type I) | α-Neuraminidase (Sialidase) |
| Galaktosialidosis | Cathepsin A |
| GM₁-Gangliosidosis / MPS IVB | β-Galactosidase |
| GM₂-Gangliosidosis | |
| M. Sandhoff | β-Hexosaminidase A + B |
| M. Tay-Sachs | β-Hexosaminidase A |
| Metachromatic Leukodystrophy | Arylsulfatase A |
| M. Krabbe | Cerebrosid-β-Galactosidase |
| M. Gaucher | β-Glucocerebrosidase |
| M. Fabry | α-Galactosidase A (Ceramidtrihexosidase) |
| M. Niemann-Pick Type I (=A+B) | Sphingomyelinase |
| Glykogenose Typ II (M. Pompe) | Acid α-Glucosidase (Acid Maltase) |
| Infantile NCL (CLN1-defect) | Palmitoyl-Protein Thioesterase 1 (PPT1) |
| Late Infantile NCL (CLN2-defect) | Tripeptidyl-Peptidase 1 (TPP1) |
| Late Infantile NCL (CLN5-defect) | CLN5-Protein |
| Cathepsin D deficient NCL (CLN10-defect) | Cathepsin D |
| M. Faber | Ceramidase |
| M. Wolman | acid Lipase |
| X-chromosomal Ichthyosis | Steroidsulfatase (Arylsulfatase C) |

Ideally the chimeric molecule is administered intraventricularly, by use of an Ommaya reservoir or a Rickham capsule.

In one embodiment the invention relates to the use of the chimeric molecule according to the invention for the manufacture of a medicament.

The invention also relates to a method of treating a lysosomal storage disease comprising the administration of a therapeutically effective amount of a chimeric molecule according to the invention.

### EXAMPLES

### Example 1

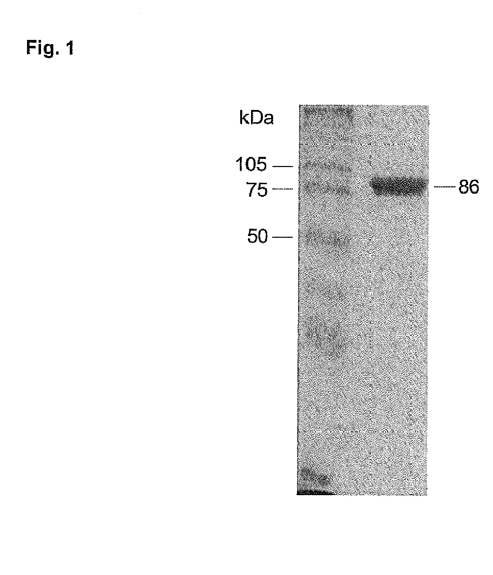

The medium to be purified is adjusted to a pH-value of 6.0 using a phosphate buffer (final concentration 20mM; stock solution: KH₂PO₄, 1 M, pH 4.5 and K₂HPO₄ 1 M pH 9). After centrifugation for 10 min at 40.000 g and 4 °C, the medium is filtrated through a 0.2 µm filter and then degassed. The supernatant, having a maximum NaCl concentration of 100 mM, is applied to a cation exchange column (for example Resource S). The flow-through is collected.
The column is then washed with 10 column volumes of a 20 mM phosphate buffer (pH 6, 100 mM NaCl). A further washing step using an intermediate gradient of 100 to 150 mM NaCl over 5 column volumes is applied. Elution is achieved by applying a linear gradient of 150 to 500 mM NaCl over 20 column volumes (1 ml fractions are collected). A final step of 1 M NaCl over 10 column volumes is applied. UV and salt gradient are monitored during the entire elution process.
Fractions containing the fusion protein are pooled and adjusted to pH 7.5 using phosphate buffer. Fig. 1 shows a purified sample of the TPP1-FGF2 fusion protein.

### Example 2

The medium is adjusted to a pH of 7.5 using a 20 mM phosphate buffer, centrifuged for 10 min at 40.000 g and 4 °C, filtrated through a 0.2 µm filter and then degassed. The supernatant is diluted with 1 volume of 20 mM phosphate buffer (pH 7.5) so that the diluted supernatant has a maximum NaCl concentration of 80 mM. The diluted supernatant is then applied to an anion exchange column (for example Resource Q). The column is subsequently washed with 10 column volumes of phosphate buffer (pH 7.5; 80 mM NaCl) followed by an intermediate NaCl gradient of 80 to 150 mM NaCl over 10 column volumes. For elution, the a linear gradient of 150-500 mM NaCl over 20 column volumes is applied (1 ml fractions are collected, peak between 200-300 mM NaCl) with a subsequent adjustment to 1 M NaCl over 10 column volumes. UV and salt gradient are monitored during the entire elution process.

### Example 3

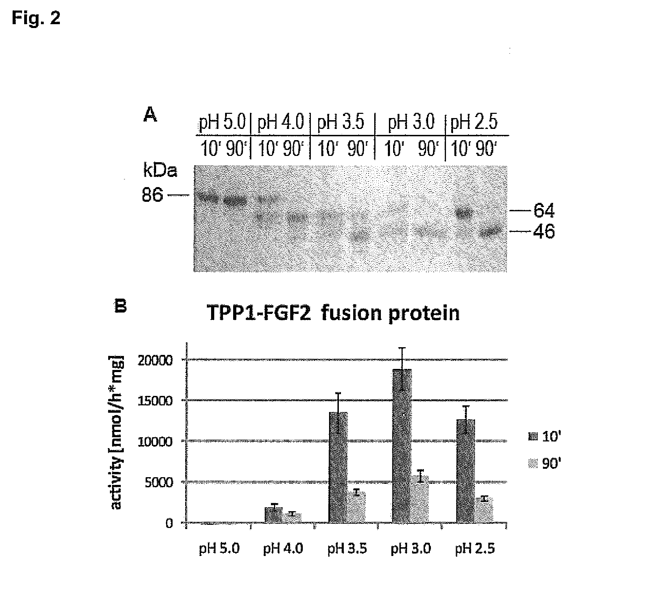

The medium is adjusted to a pH of 7.5 using 20 mM phosphate buffer. The final NaCl concentration is adjusted to 800 mM NaCl. The medium is then centrifuged for 10 min at 40.000 g and 4 °C, followed by filtration through a 0.2 µm filter and subsequent degassing. The filtered supernatant is then applied to a Heparin-Sepharose-column (flow rate 1 ml/min), the flow-through is collected.
Purification is continued by applying 10 column volumes of 20 mM phosphate buffer (pH 7.5, 800 mM NaCl). For elution a linear gradient of 0.8 - 2 M NaCl over 20 column volumes is applied (1 ml fractions are collected, peak between 1.5 and 1.8 M NaCl), followed by a 2 M NaCl step over 10 column volumes. UV and salt gradient are monitored during the entire elution process.
After subsequent desalting and buffer exchange to PBS (pH 7.5) using gel filtration or ultrafiltration, the TPP1-FGF2 fusion proteins are aliquoted and stored at -70 °C.
For further characterisation of the fusion proteins, the enzymatic activities are examined by a standardized enzyme assay. The pH dependent auto-activation of the TPP1-FGF2 proproteins was comparable to that of the TPP1 wild-type. Fig. 2 illustrates the auto-processing of a TPP1-FGF2 fusion protein.

### Example 4

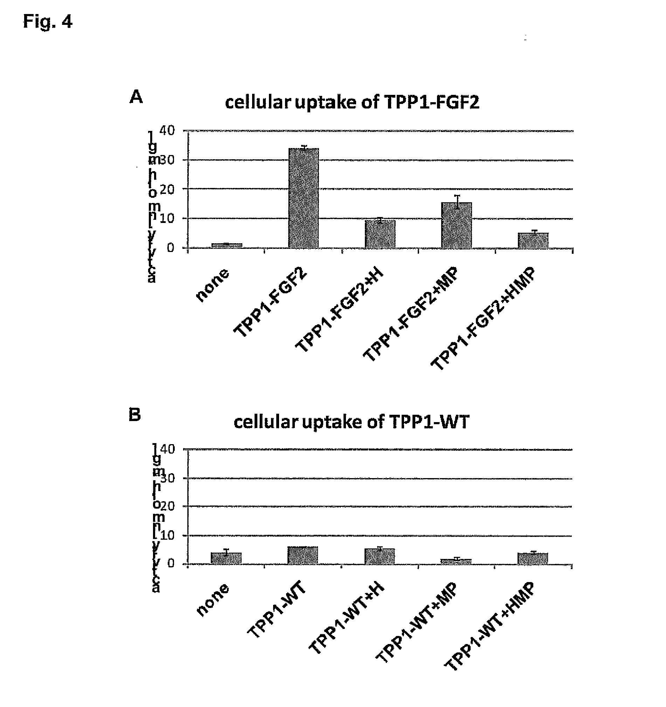

Furthermore, endocytosis into human neuronal progenitor cells (NT2 cells) was compared for TPP1-FGF2 fusion proteins and the TPP1 wild-type. At a final concentration of 0.4-0.5 µM TPP1-FGF2 fusion protein or TPP1 wild-type protein, respectively, was added to the medium. After 48 hours of incubation the intracellular TPP1-activity was measured (see Fig. 4). TPP1-activity was six times higher in cell lysates of the NT2-cells which were treated with TPP1-FGF2 fusion proteins than for the TPP1 wild-type protein. It was possible to inhibit the intracellular TPP1-activity by heparin either alone or in combination with mannose-6-phosphate. The results show that the cellular uptake of the TPP1-FGF2 fusion protein is mainly mediated by cell surface HSPG.

### Example 5

Finally, the effect of the TPP1-FGF1 fusion proteins was also examined in an animal model, namely *tpp1-*/*-* mice. In weekly intervals, the *tpp1-*/*-* mice were injected intraventricularly with 10 µg of TPP1-FGF2 fusion protein or TPP1 wild-type protein, respectively. Mice treated with TPP1-FGF2 showed a significantly higher life expectancy as compared to mice treated with the TPP1 wild-type protein (Fig. 5).

Moreover, *tpp1-*/*-* mice treated with TPP1-FGF2-fusion proteins showed a delayed course of illness in comparison to *tpp1-*/*-* mice treated with the TPP1 wild-type. This result was tested by checking the motor coordination with a so called Rotor Rod (a rotating pole) (Fig. 6). As of the 17^{th} week, *tpp1-*/*-* mice treated with the TPP1-FGF2 fusion protein were able to stay longer on the Rotor Rod than the *tpp1*-/- mice treated with the TPP1 wild-type.

## Claims

1. A chimeric molecule comprising
(i) a targeting moiety that binds to heparin or heparan sulfate proteoglycans,
(ii) a lysosomal peptide or protein,
(iii) wherein the targeting moiety is a neurotrophic growth factor and/or, wherein the targeting moiety comprises one of the following consensus sequences BBXB, BXBB, BBXXB, BXXBB, BBXXXB or BXXXBB and wherein B represents an arginine, lysine or histidine amino acid and X represents any amino acid,
(iv) with the proviso that the targeting moiety is at least thirteen amino acids long.

2. Chimeric molecule according to claim 1, wherein the targeting moiety is selected from the group of
(v) annexin II comprising the amino acid sequence according to SEQ ID NO. 1 (KIRSEFKKKYGKSLYY),
(vi) vitronectin comprising the amino acid sequence according to SEQ ID NO. 2 (QRFRHRNRKGYRSQRG),
(vii) ApoB comprising the amino acid sequence according to SEQ ID NO. 3 (KFIIPSPKRPVKLLSG),
(viii) bFGF comprising the amino acid sequence according to SEQ ID NO. 4 (GHFKDPKRLYCKNGGF),
(ix) NCAM comprising the amino acid sequence according to SEQ ID NO. 5 (DGGSPIRHYLIKYKAK),
(x) Protein C inhibitor comprising the amino acid sequence according to SEQ ID NO. 6 (GLSEKTLRKWLKMFKK),
(xi) AT-III comprising the amino acid sequence according to SEQ ID NO. 7 (KLNCRLYRKANKSSKL),
(xii) ApoE comprising the amino acid sequence according to SEQ ID NO. 8 (SHLRKLRKRLLRDADD),
(xiii) Fibrin comprising the amino acid sequence according to SEQ ID NO. 9 (GHRPLDKKREEAPSLR),
(xiv) hGDNF comprising the amino acid sequence according to SEQ ID NO. 10 (SRGKGRRGQRGKNRG),
(xv) B-thromboglobulin comprising the amino acid sequence according to SEQ ID NO. 11 (PDAPRIKKIVQKKLAG)
(xvi) Insulin-like growth factor-binding protein-3 comprising the amino acid sequence according to SEQ ID NO. 12 (DKKGFYKKKQCRPSKG),
(xvii) Antp comprising the amino acid sequence according to SEQ ID NO. 13 (RQIKIWFQNRRMKWKK)
(xviii) human clock comprising the amino acid sequence according to SEQ ID NO. 14 (KRVSRNKSEKKRR)

3. Chimeric molecule according to claims 1 to 2, wherein the growth factor is modified and lysosomal targeting is improved.

4. Chimeric molecule according to claims 1 to 3, wherein the targeting moiety and the enzyme moiety are covalently linked to each other.

5. Chimeric molecule according to claims 1 to 4, wherein the chimeric molecule is a single polypeptide chain.

6. Chimeric molecule according to dams 1 to 5, wherein the targeting moiety and the enzyme moiety are linked via a peptide linker.

7. Chimeric molecule according to claims 1 to 6, wherein the peptide linker comprises a protease cleavage site.

8. Chimeric molecule according to claims 1 to 7, wherein the protease cleavage site is that of a protease selected from the group consisting of factor Xa, thrombin, trypsin, papain and plasmin.

9. Chimeric molecule according to claim 8, wherein the lysosomal enzyme is selected from the group consisting of, β-galactocerebrosidase, arylsulfatase A (sulfatidase), α-iduronidase, sulfaminidase, α-N-acetylglucosaminidase, acetyl-CoA:α-glucosaminide-N-Ac-transferase, N-acefiyfglucosamine-6-sulfatase, tripeptidyl-peptidase I, palmitoyl-protein thioesterase, β-galactosidase, sphingomyelinase, β-hexosaminidase A, β-hexosaminidase B, ceramidase, α-mannosidase, β-mannosidase, α-fucosidase, sialidase, α-N-acetylgalactosaminidase, α-L-iduronidase, iduronate-2-sulfatase, sulfamidase (heparan N-sulftase), α-N-acetylglucosaminidase, N-acetylgalactosamin-6-sulfatase, arylsulfatase B, β-glucuronidase, α-L-fucosidase, aspartylglucosaminidase, α-neuraminidase (sialidase), cathepsin A, β-hexosaminidase A + B, arylsulfatase A, cerebroside-β-galactosidase, β-glucocerebrosidase, α-galactosidase A (ceramide trihexosidase), acid α-glucosidase (acid maltase), CLN5-protein, acid lipase, steroid sulfatase (arylsulfatase C) and cathepsin D.

10. Chimeric molecule according to any one of the claims 2 to 9, wherein the targeting moiety is a polypeptide having a sequence according to any one of SEQ ID NO. 18, 20, 24, 26, 28 and 30.

11. Chimeric molecule according to any one of the claims 1 to 10, wherein the enzyme moiety is a polypeptide having a sequence according to any one of SEQ ID NO. 52, 54, 56, 58, 60, 62, 64, 66, 68 and 70.

12. Chimeric molecule according to any one of the claims 10 to 11, wherein the polypeptide has a sequence according to any one of the SEQ ID NO. 32, 34, 36, 38, 40, 42, 46, 48 and 50.

13. Polynucleotide encoding the chimeric molecule according to any one of the claims 1 to 12.

14. Polynucleotide according to claim 13 having the sequence according to any one of the SEQ ID NO. 31, 33, 35, 37, 39, 41, 45, 47 and 49.

15. Pharmaceutical composition comprising a chimeric molecule according to any one of the claims 1 to 14.

16. Chimeric molecule according to any one of the claims 1 to 12 for the use in the treatment of a disease.

17. Chimeric molecule according to claim 16, wherein the disease is a lysosomal storage disease.

18. Chimeric molecule according to claim 17, wherein the lysosomal storage disease is selected from the group consisting of the late infantile form of neuronal ceroid lipofuscinosis (NCL),mucopolysaccharidosis type I, mucopolysaccharidosis type II, mucopolysaccharidosis type IIIA, mucopolysaccharidosis type IIIB, mucopolysaccharidosis type IIIC, mucopolysaccharidosis type IIID, mucopolysaccharidosis type IVA, mucopolysaccharidosis type IVB, mucopolysaccharidosis type VI, mucopolysaccharidosis type VII, fucosidosis, α-mannosidosis, β-mannosidosis, aspartylglucosaminuria, Schindler's disease, Sialidosis (Mucolipidosis I), galaktosialidosis, GM₁-gangliosidosis /MPS IVB, GM₂-gangliosidosis, Sandhoff disease, Tay-Sachs disease, metachromatic leukodystrophy, Krabbe disease, Gaucher disease, Fabry disease, Niemann-Pick disease type I (=A+B), glycogen storage disease type II ( Pompe disease), infantile NCL (CLN1-defect), late infantile NCL (CLN2-defect), late infantile NCL (CLN5-defect), NCL caused by cathepsin D deficiency (CLN10-defect), Faber's syndrome, Wolman disease, X-linked ichthyosis.

19. Chimeric molecule according to claim 18, wherein the lysosomal storage disease is the late infantile form of neuronal ceroid lipofuscinosis and the enzyme moiety comprises lysosomal tripeptidyl peptidase 1 (TPP1).

20. The chimeric molecule according to any one of the claims 1 to 12, wherein the chimeric molecule is administered intraventricularly, by use of an Ommaya reservoir or a Rickham capsule.

21. The use of the chimeric molecule according to any one of the claims 1 to 12 for the manufacture of a medicament.

22. A method of treating a lysosomal storage disease comprising the administration of a therapeutically effective amount of a chimeric molecule according to any one of the claims 1 to 12 to a subject.
